# EUROPEAN PATENT SPECIFICATION

(11) **EP 1 278 768 B1**
(45) Date of publication and mention of the grant of the patent: **15.11.2006**
(21) Application number: 01931657.9
(22) Date of filing: 26.04.2001
(51) Int. Cl.: C07K 14/205, A61K 39/02

(54) **METHOD FOR IDENTIFYING HELICOBACTER ANTIGENS**
METHODE ZUR IDENTIFIZIERUNG VON HELICOBACTER ANTIGENEN
PROCEDE D'IDENTIFICATION D'ANTIGENES D'HELICOBACTER

(30) Priority: 27.04.2000 EP 00108968; 23.01.2001 EP 01101439
(43) Date of publication of application: 29.01.2003
(73) Proprietor: Max-Planck-Gesellschaft zur Förderung der Wissenschaften e.V., 80539 München (DE)
(72) Inventor: MEYER, Thomas, F., 10117 Berlin (DE); JUNGBLUT, Peter, 14195 Berlin (DE); BUMANN, Dirk, 12159 Berlin (DE); AEBISCHER, Anton, 12105 Berlin (DE); HAAS, Gaby, 12683 Berlin (DE); ZIMNY-ARNDT, Ursula, 12277 Berlin (DE); LAMER, Stephanie, 13507 Berlin (DE); KARAALI, Galip, 13407 Berlin (DE); SABARTH, Nicolas, 10119 Berlin (DE); WENDLAND, Meike, 12247 Berlin (DE)
(74) Representative: Weiss, Wolfgang
(86) International application number: PCT/EP2001/004728
(87) International publication number: WO 2001/083531

(56) References cited:
- EP-A- 0 835 928
- WO-A-98/49314
- WO-A1-98/43478
- US-A- 5 942 409
- JUNGBLUT P.R. ET AL.: "Comparative proteome analysis of Helicobacter pylori" MOLECULAR MICROBIOLOGY, vol. 36, no. 3, 1 May 2000 (2000-05-01), pages 710-725, XP002173839
- TOMB J.F. ET AL: 'THE COMPLETE GENOME SEQUENCE OF THE GASTRIC PATHOGEN HELICOBACTER PYLORI' NATURE vol. 388, 1997, pages 539 - 547, XP002062106

## Description

The present invention relates to Helicobacter antigens which are suitable as targets for the treatment of Helicobacter infections.

The presence of bacteria in the stomach mucosa was described by Bizzozero as early as 1893 (Bizzozero, 1893). Only ninety years later Warren and Marshall (Warren, 1983; Marshall and Warren, 1984) succeeded in cultivating bacteria, later named *Helicobacter pylori,* which had been isolated from the gastric epithelia of active chronic gastritis patients. With the detection of *H. pylori* in the stomach, a paradigm shift in medical microbiology occurred. Epidemiological studies revealed a statistically significant correlation between the presence of *H. pylori* and stomach carcinomas (Forman et al., 1991; Nomura et al., 1991; Parsonnet et al., 1991). It is now recognized that *H. pylori* is a major cause of inflammation leading to dyspepsia, duodenal or gastric cancer or gastric mucosa-associated lymphoid tissue lymphoma (MALT). In 1994 the WHO declared *H. pylori* to be a definitive carcinogen (IARC Working Group on the Evaluation of Carcinogenic Risks to Humans, 1994).

Diagnosis of *H. pylori* is performed by invasive and noninvasive methods. Invasive methods include biopsies, urease test, histology, direct microscopy, culture, and PCR from biopsy material. Noninvasive tests are ¹³C-urea breath test, serological tests like ELISA and immunoblots. PCR, ELISA and immunoblotting require the identification of gene or protein targets characterizing *H. pylori* presence (Megraud, 1997). The genes *cagA* and *ureC* can be detected directly in biopsies by PCR (Lage *et al.,* 1995). Blots of one-dimensional SDS-PAGE gels revealed several diagnostically relevant antigens, including CagA, VacA, urease a subunit, heat shock protein B, and 35 kDa antigen. Others were only characterized by their apparent molecular mass (Aucher et al., 1998; Lamarque et al., 1999; Nilsson et al., 1997).

*H. pylori* infection can be successfully treated by "triple therapy" combining a proton pump inhibitor with two antibiotics (Moayyedi et al., 1995; Goddard and Logan, 1995; Labenz and Borsch, 1995). The high cost of antibiotic treatment, the likelihood for development of antibiotic resistance and the potential reinfection, have provided impetus for the development of a therapeutic and/or prophylactic vaccine against *H. pylori.* In small animal models such as the mouse, the *H. pylori* urease was the first protein shown to provide protective immunity to a *Helicobacter* infection (Michetti et al., 1994). Since then, VacA (Marchetti et al., 1995; Marchetti et al., 1998; Crabtree, 1998), CagA (Marchetti et al., 1998; Crabtree, 1998), catalase (Radcliff et al., 1997), a nickel-binding heat shock protein (Gilbert et al., 1995), and a citrate synthase homologue (Dunkley et al., 1999) were also used successfully as vaccines in mouse models. In addition, the Lewis antigen-binding adhesin BabA (Ilver et al., 1998) and HP0175, an open reading frame with some homology to *Campylobacter jejuni* cell binding protein 2, were proposed as vaccine candidates (McAtee et al., 1998c). Given the enormous heterogeneity of *H. pylori* strains there is a great need for.additional vaccine candidates conserved between strains and antigens of diagnostic value.

In animal models, several strategies have been applied to gain protective immunization. In 1992, Chen et al. reported induction of protective immunity in mice after oral vaccination with whole cell sonicates, this was later confirmed by Czinn et al. (Czinn et al., 1993; Chen et al., 1992).

WO 98/49314 refers to polypeptides of *H. pylori* identified by proteomics methodology. Antibodies against a number of these polypeptides were found in patient sera.

Workers in our laboratory reported 100% protection after single oral immunization with an attenuated *Salmonella typhimurium* live vaccine expressing UreA and UreB (Gomez-Duarte et al., 1998). The high efficacy in the mouse model, combined with remarkable immunogenicity, safety and low-cost production, makes attenuated live recombinant Salmonella a promising vaccine strategy for the control of H. *pylori*-related diseases in humans (Gomez-Duarte et al., 1999).

To date, the complete genome of 26 microorganisms has been sequenced, including two strains of *H. pylori* 26695 (Tomb et al., 1997) and J99 (Alm et al., 1999). The number of genes predicted for the two *H. pylori* strains sequenced is 1590 predicted genes for strain 26695 (Tomb et al., 1997); and 1495 predicted genes for strain J99 (Alm et al., 1999), respectively.

Knowing the nucleic acid sequence of the Helicobacter genome, however, is just a first step in understanding the processes taking place in the case of a Helicobacter infection and in selecting specific Helicobacter proteins as targets for the treatment of Helicobacter infections.

Thus, the object underlying the present invention was to provide proteins expressed by cultivated Helicobacter cells suitable for the manufacture of a medicament, particularly a vaccine against Helicobacter.

In order to solve this problem the present invention provides a systematic analysis of about 1,800 Helicobacter proteins, the identification of 152 proteins by peptide mass fingerprinting MALDI mass spectrometry (Example 1). This comprehensive analysis is the basis of comparative proteome analysis as exemplified by comparison of the protein composition of different *H. pylori* strains, the comparison of different biological situations, and the identification of antigens.

In the context of the present application, characterization of protein is the analysis of the chemical composition of the protein. Identification of a protein is the assignment of a spot on the 2DE-gel to its biological functions or at least the assignment to a gene including the regulatory and coding sequences. In the context of the present application, the proteome comprises the protein composition of an organism or a part of it at a defined biological situation.

The method used in the context of present invention allows characterization and identification of Helicobacter proteins of given Helicobacter strains under given cultivation conditions and, thus, analysis of the interaction between genetic information and the environment by comparison of different biological situations. By means of two-dimensional gel electrophoresis and characterization of separated individual proteins, e.g. by peptide fingerprinting a comparative proteome analysis is provided which allows the detection of functionally interesting proteins as a prerequisite for the elucidation of antigens, virulence and pathogenicity factors.

The actual technique of 2-DE used in this approach has a potential to resolve up to 5000 protein species, which may be easily increased twofold by doubling the gel size. Due to the size of *H. pylori* genome, the resolution power allows three posttranslational modifications per gene locus, if all of the proteins are really expressed. For this organism there is a good chance to resolve more than 90% of the proteome and in addition posttranslational modifications, whereas in eukaryotic organisms by 2-DE only the top of the iceberg may be visualized. In contrast to earlier investigations, where it was claimed that MALDI-MS is not sufficient for, a sure identification, we could show that for organisms with complete genome sequences MALDI-MS alone is sufficient for identifications.

The comparative analysis between different Helicobacter strains is a suitable tool for identifying pathogenicity and virulence factors as well as strain-independent immunization targets. The comparative analysis of proteins from Helicobacter cells grown under different conditions, e.g. from Helicobacter cells which have been cultivated in vitro or in vivo or from Helicobacter cells which have been cultivated at different pH values, e.g. in the range from about 5 to 8, is suitable for identifying proteins which are preferably expressed under conditions which resemble the conditions in the host and, thus, also allow the identification of relevant target molecules which are expressed in vivo. Urease, an enzyme on the surface of H.pylori leads to cleavage of the urea that is present and thus leads to local neutralization of the acidic pH value in the stomach.

The term "protein species" describes a chemically clearly defined molecule and corresponds to one spot on a high-performance 2-DE pattern (Jungblut, P., Thiede B., Zimny-Arndt, U., Müller, E.-C., Scheler, C., Wittmann-Liebold, B., Otto, A., Electrophoresis 1996, 17, 839-847).

Overall, three hundred and seventy nine antigenic protein species were detected representing about 18% of all spots separated. Twenty-one of the 30 proteins most frequently recognized by *H. pylori* positive sera, were confirmed from other studies and 9 were newly identified. Among the 156 identified most abundant protein species of *H. pylori* (Example 2) we have found one specific antigen: the predicted coding region HP0231.

The function of the protein HP 0231 is not yet known. HP 0231 is a surface exposed protein (Table 17) and a secreted protein (Table 18). HP 0231 is an *H. pylori* specific antigen in human sera (Table 8, 14) and recognized by *H. pylori* positive patients (Table 7) with significant difference to *H. pylori* negative individuals (Table 11). Vaccination with recombinant HP0231 (adjuvant: cholera toxin) protected mice against *H. pylori.*

The present invention is directed to the use of protein HP0231 for the manufacture of a vaccine for the treatment of Helicobacter infections and/or a specific Helicobacter-mediated disease.

In a further embodiment, the use of a nucleic acid encoding the protein HP0231 for the manufacture of a vaccine for the treatment of Helicobacter infections and/or a specific Helicobacter-mediated disease is claimed.

In a preferred embodiment, the vaccine is selected from recombinant subunit vaccines, recombinant live vaccines and nucleic acid vaccines.

In a preferred embodiment, specific Helicobacter-mediated diseases are selected from gastritis, cancer and ulcer.

The manufacture of a vaccine may comprise the administration of substantially purified polypeptide or peptide antigens derived from the protein species as described above. Further, the manufacture of the vaccine may comprise the administration of nucleic acid vaccines encoding a suitable polypeptide or peptide antigen. Furthermore, the manufacture of the vaccine may comprise the administration of recombinant live vaccines such as described by Gomez-Duarte et al. (1998). The vaccine may be administered in any suitable way, e.g. by oral, parenteral or mucosal routes. The vaccine is formulated as a pharmaceutical composition comprising the active agent and a suitable pharmaceutical carrier and optionally an adjuvant. The composition may be in the form of an aqueous or non-aqueous solution, suspension, tablet, cream, ointment etc. depending on the route of administration. Preferably the vaccine is administered by injection or by the oral route. The vaccine may be administered in one or several doses as required by the specific type of the vaccine and/or the disease to be treated or prevented. The amount of the antigen to be administered will also depend on the specific type of antigen used and the type or severity of the disease to be treated or prevented. Corresponding dosages can be easily determined by a skilled physician.

The present invention is to be further illustrated by the following Figures and Examples.

### Figure legends

- Figure 1:: 2-DE gel of total cell protein of (a) *H. pylori* 26695, (b) *H. pylori **J99*** and (c) *H. pylori* SS1. The original gel size is 23 x 30 x 0.075 cm. The proteins were detected by silver staining.
- Figure 2:: Sectors A-F of the 2-DE pattern of *H*. *pylori* 26695 cell proteins. Identified proteins are marked with corresponding accession numbers in Table 1.
- Figure 3:: Part of sector B with protein species differing in spot intensity depending on pH during cultivation. Six spots are marked with database numbers, which showed clearly different intensities. Five of them were identified (Table 5). B184 had not previously been identified. A, *H. pylori* 26695 cultivated at pH 8; B, *H. pylori* 26695 cultivated at pH 5.
- Figure 4:: Antigens of *H. pylori* 26695 detected by immunostaining on 2-DE blots. Spots marked with numbers were identified and the numbers correspond to the 2-DE database numbers. The protein name may be found in Table 1 or in Table 6. A, patient serum Mpi54, peptic ulcer; B, patient serum Mpi44, adenocarcinoma.
- Figure 5:: Two DE-gel of cellular proteins from *H. pylori* 26695 detected by silver staining. Six sectors (A-F) are marked by dashed lines. Spots that have been identified as immunogenic are marked with numbers and consist of the letter for the sector A-F in the gel and a number for identification.
- Figure 6:: *H. pylori* 26695 antigens detected by immunostaining on 2-DE blots with sera from patients with gastric disorders: A. *H. pylori* unrelated gastritis, B. *H. pylori* gastritis, C. *H. pylori* gastric ulcer, D. gastric cancer. Spots that have been identified are marked (see legend Figure 5).
- Figure 7:: Two-DE blot of biotinylated *H. pylori* lysates stained with NeutrAvidin-coupled peroxidase.
- Figure 8:: Two-DE blot of biotinylated intact *H. pylori* cells stained with NeutrAvidin-coupled peroxidase. Marked spots were identified. Their numbers correspond to the numbers in Tab. 17.
- Figure 9:: Two-DE blot of biotinylated membrane proteins purified from labeled intact *H. pylori* cells. A) Silverstaining, B) NeutrAvidin-staining.
- Figure 10:: Two-dimensional electrophoresis of extracellular proteins of an *H. pylori* strain 26695 liquid culture. Spot numbers correspond to Table 18.

### Example 1 (for illustrative purposes only)

### 1. Experimental Procedures

### 1.1 Helicobacter pylori strains and growth conditions

In this study, the proteomes of three different *H. pylori* strains were compared. The strains Hp26695 and J99 were used. The genome of these strains has been entirely sequenced (Tomb et al., 1997; Alm et al., 1999). A mouse-adapted *H. pylori* strain, the "Sydney strain" SS1 (Lee et al., 1997), that has been used for pre-clinical vaccine testing (Corthesy-Theulaz et al., 1997; Gomez-Duarte et al., 1998; Radcliff et al., 1997) was also analyzed.
All *H. pylori* strains were grown on serum plates (Odenbreit et al., 1996) at 37°C in a microaerobic atmosphere (5% O₂, 85% N₂, and 10% CO₂) for two days or five days for the pH variations investigated. The bacteria were harvested, washed twice in ice-cold PBS containing proteinase inhibitors (1 mM PMSF, 0.1 µM pepstatin, 2.1 µM leupeptin, 2.9 mM benzamidin), and lysed by resuspension in half a volume of distilled water. The resulting volume in µl was multiplied by i) 1.08 to obtain the amount of urea in mg to be added, ii) 0.1 to obtain the volume in µl of 1.4 M DTT and 40% Servalyte (Serva, Heidelberg, Germany) pl 2-4 to be added. CHAPS was added to obtain an end concentration of 1%. The end concentrations of DTT and urea were 70 mM and 9 M, respectively. Solubilization of the proteins occurred within 30 min at room temperature. A protein concentration of 15 µg/µl +/- 25% was obtained.

### 1.2 Two-dimensional electrophoresis

For the resolution of the *H, pylori* proteome, we used a 23 cm x 30 cm 2-DE gel system (Jungblut et al., 1994; Klose and Kobalz, 1995) with a resolution power of about 5 000 protein species. For subtractive analyses (Aebersold and Leavitt, 1990) and database construction, we applied 50-100 µg of protein to the anodic side of the IEF gel. In the second dimension we used 0.75 mm thick gels. The proteins were detected by silver staining optimized for these gels (Jungblut and Seifert, 1990). For identification of proteins, 200 - 300 µg of protein were applied and in the second dimension 1.5 mm thick gels were used. The proteins were stained by Coomassie Brilliant Blue R250 (Eckerskorn et al., 1988) or G250 (Doherty et al., 1998), or negative staining (Fernandez-Patron et al., 1995).

### 1.3 Peptide mass fingerprinting

The proteins were identified by tryptic digestion. The proteins were digested on-blot or in-gel in 10 µl or 20 µl, respectively, 50 mM ammonium bicarbonate buffer pH 7.8, 10% (v/v) acetonitrile. The digestion mix contained for on-blot or in-gel digestion 0.05 µg or 0.1 µg trypsin (Promega, Madison, WI), respectively. The proteins were digested overnight at 37°C under shaking. Only one spot was used per digestion.

Before digestion the spot was washed and equilibrated (Otto et al., 1996). The digestion buffer was used as equilibration buffer. The digestion was performed in 20 µl digestion buffer with 0.1 µg trypsin as described above. After digestion the sample was centrifuged and sonicated for 2 min. Ten µg POROS R2 beads in 100 µl 0.5% methanol, 0.1 % TFA were added. After incubation for 15 min under shaking the POROS beads were centrifuged and transferred onto the sample plate. On-target elution was performed with 1 µl matrix solution (saturated α-cyano-4-hydroxy cinnamic acid solution in 50% acetonitrile, 0.3% TFA). Alternatively, two µl of the sample were taken off directly after sonication of the digest, mixed with 2 µl matrix solution and 2 µl were applied onto the sample plate.

The peptide/matrix solution was applied to the sample template of a matrix-assisted laser desorption/ionization mass spectrometer (Voyager Elite, Perseptive, Framingham, MS, USA). Data were obtained using the following parameters: 20 kV accelerating voltage, 70% grid voltage, 0.050% guide wire voltage, 100 ns delay, and a low mass gate of 500.

Peptide mass fingerprints were searched using the program MS-FIT (http://prospector.ucsf.edu/ucsfhtml/msfit.htm)reducingtheproteins of the NCBI database to the Helicobacter proteins and to a molecular mass range estimated from 2-DE +/- 20%, allowing a mass accuracy of 0.1 Da for the peptide mass. In the absence of matches the molecular mass window was extended. Partial enzymatic cleavages leaving two cleavage sites, acetylation of the N-terminus, removal of methionine from the N-terminus and concurrent acetylation, oxidation of methionine, pyro-glutamic acid formation of N-terminal glutamine and modification of cysteine by acrylamide were considered in these searches.

### 1.4 Dependency of pH

*H. pylori* was cultivated on serum plates as described above. The pH of the medium was adjusted to 5, 6,7, and 8. For each pH value three independent cultivations were performed and from each one the proteins were separated by a small gel 2-DE method (Jungblut and Seifert, 1990). The spot intensities were determined by scanning and spot detection (Topspot, Algorithmus, Berlin, Germany). To confirm four of the detected variants large 2-DE gels of pH 5 and pH 8 samples were analyzed.

### 1.5 Immunoblotting

For immunostaining the proteins were transferred from the 2-DE gels onto PVDF membranes (ImmobilonP, Millipore, Eschborn, Germany) by semidry-blotting (Jungblut et al., 1990) using a blotting buffer containing 100 mM borate, 20% methanol, pH 9.0. The blotting time was 2 h with a current of 1 mA/cm². The gels were divided in two equal-sized parts (13 x 19 cm) to avoid too high temperatures during blotting. Antigens were detected by incubation of the membranes with human sera in a dilution of 1:200, a secondary antibody (anti-human polyvalent immunoglobulins, G, A, M, peroxidase conjugated, Sigma A-8400, Deisenhofen, Germany) at a dilution of 1:10000. Before the addition of serum, the membrane was blocked with 5% skim milk, 0.05% Tween-20 in PBS for at least 1 h at room temperature. All washing steps were performed with PBS, 0.05% Tween 20. After blocking the membrane was washed 3 times for 5 min. The sera were incubated with the membrane for 1 h at room temperature. Before and after addition of the secondary antibody the membranes were washed 4 times for 15 min in PBS, 0.05% Tween 20. The washed membrane was incubated with 30 ml/membrane of a 1 : 1 mixture of Enhanced Luminol Reagent and Oxidizing Reagent for 1 min (Renaissance Western Blot Chemiluminescence Reagent for ECL Immunostaining (NEN, Köln, Germany). The detection reagent was drained off and the membrane wrapped in a foil. The foil was overlaid with Kodak BioMax MR1 film for an exposure time of 5 min. For localization the proteins were stained on the membranes by Coomassie Brilliant Blue R-250.

### 1.6 Database construction

Gels were digitised after scanning with a UMAX Mirage Ilse scanner using the Topspot software (Algorithmus, Berlin, Germany). Before spot detection the gels were divided into six sectors, which were automatically spot-detected and afterwards interactively corrected. The resulting map files were introduced together with gif files and identification data collected within an access database into the 2-DE database (Mollenkopf et al., 1999).

### 2. Results:

### 2.1 Protein separation and identification

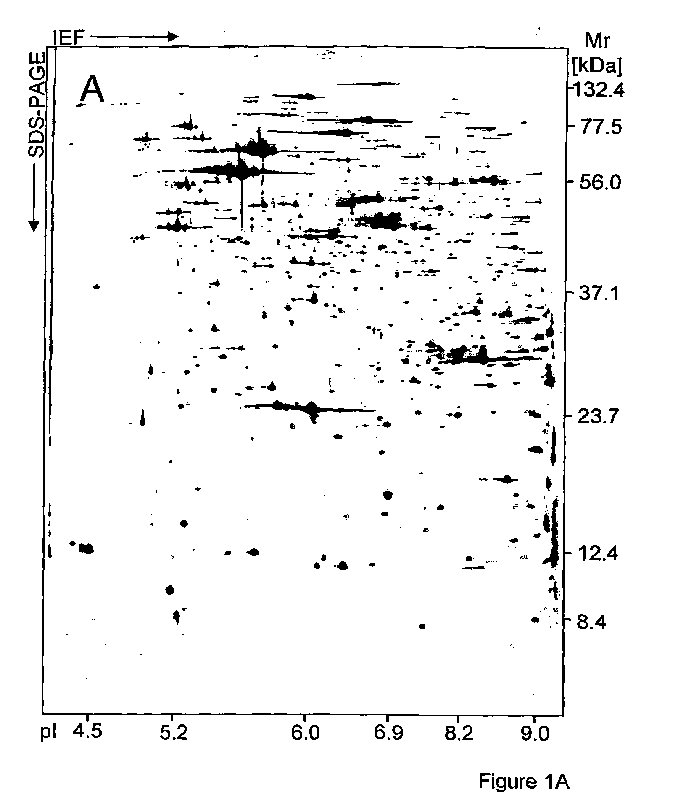

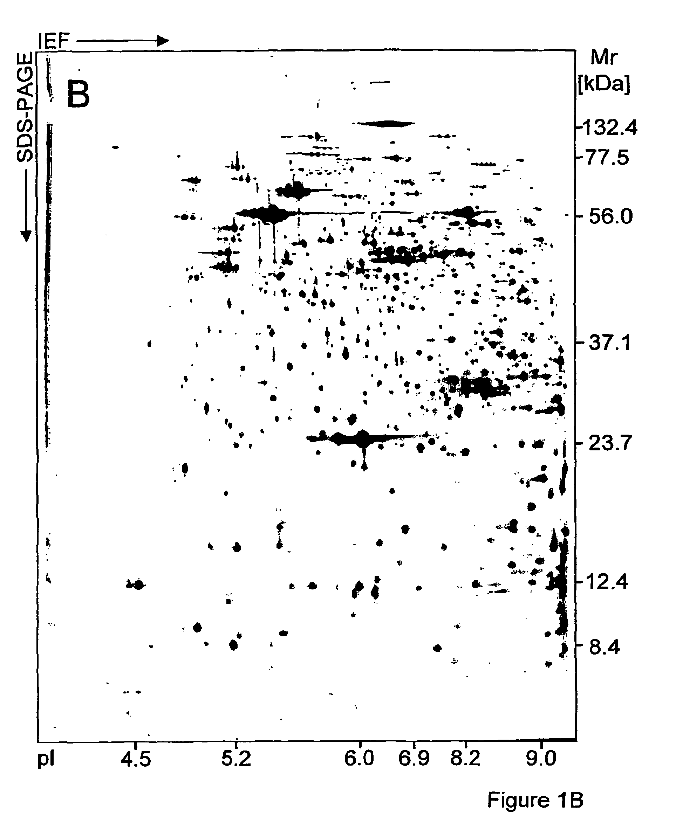

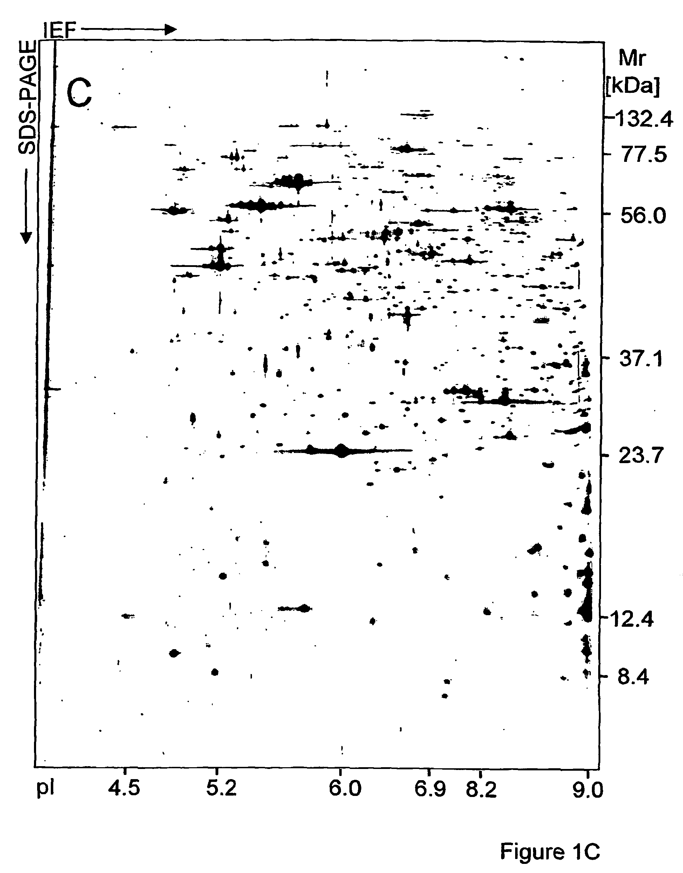

The protein composition of *H. pylori* 26695, SS1, and J99 was resolved on large 2-DE gels (Figure 1). In all 3 strains the protein spots are spread over the whole pl range of 4-10 and the whole Mr range 5 -150 kDa. There is a tendency for an increased number of protein species in the basic range of the gel and several of them are accumulated at the basic end of the gel. In total 1863, 1448, and 1622 spots were detected on the patterns of *H. pylori* 26695, SS1, and J99, respectively. The comparison of the three patterns reveals a high genetic variability. Whereas several main spots are found at the same position, many positional shifts and differentially present or absent spots are observed. Peptide mass fingerprinting using MALDI mass spectrometry allowed us to identify ten spots, which were assigned easily between the two strains 26695 and J99 (Table 2). Three protein species were identical as predicted from the genome sequence and indeed they were at the same position within the 2-DE patterns. Flavodoxin, thioredoxin, and FusA have 4, 3, and 2 amino acid exchanges, respectively, without a net charge change and therefore appear at the same position in the 2-DE pattern. Four protein species with amino acid exchanges resulting in a net charge change of at least 1 show the predicted shift. As expected the shift obtained from 1 net charge results in a larger shift for low Mr proteins as compared to high Mr proteins. In the Mr range up to 60 kDa a net charge shift of 1 discriminates two protein species on large, high-resolution 2-DE gels, as shown for GroEL and TsaA.

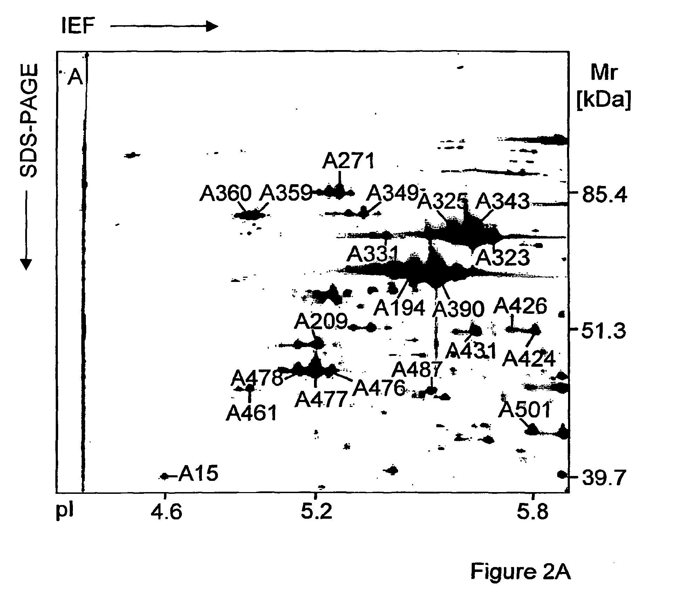

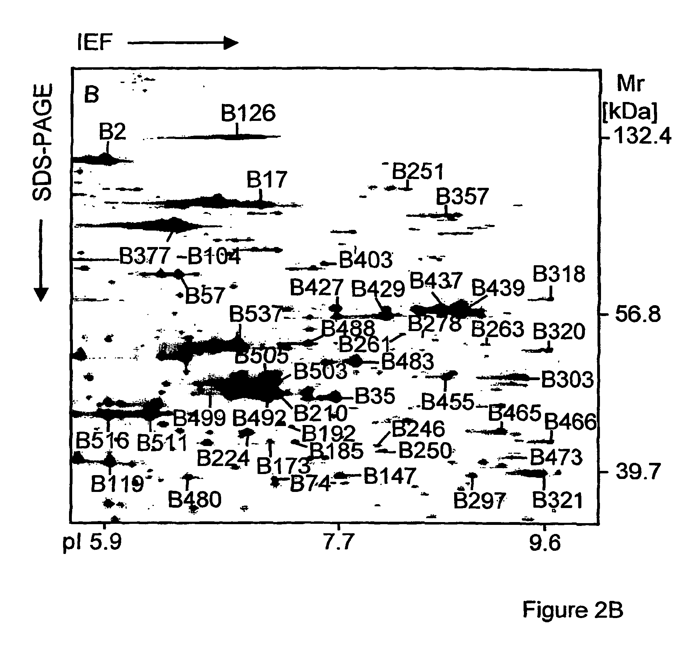

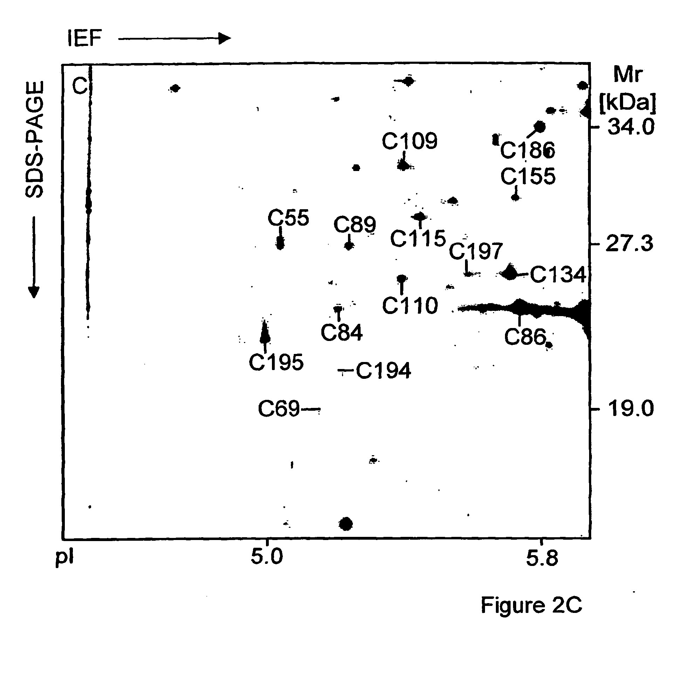

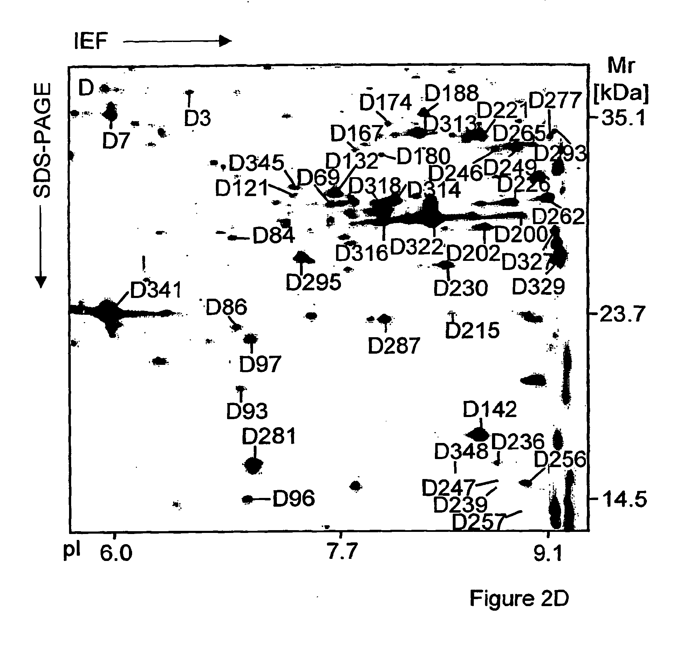

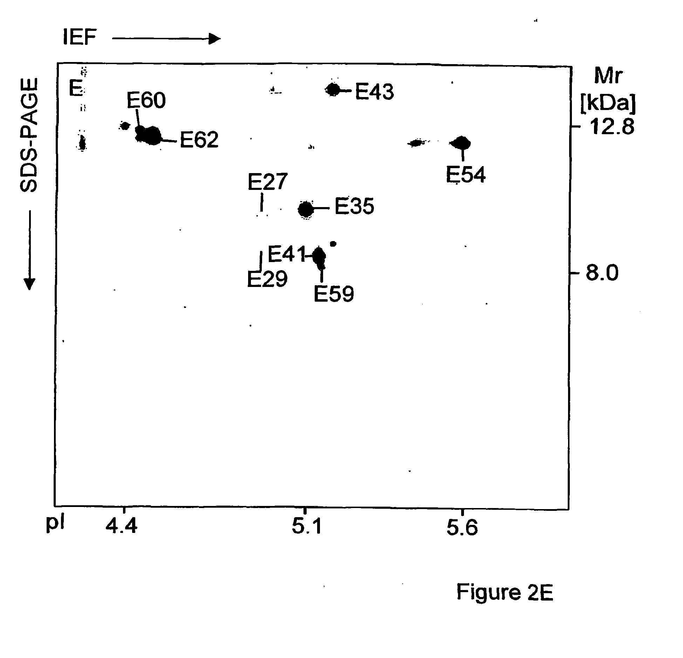

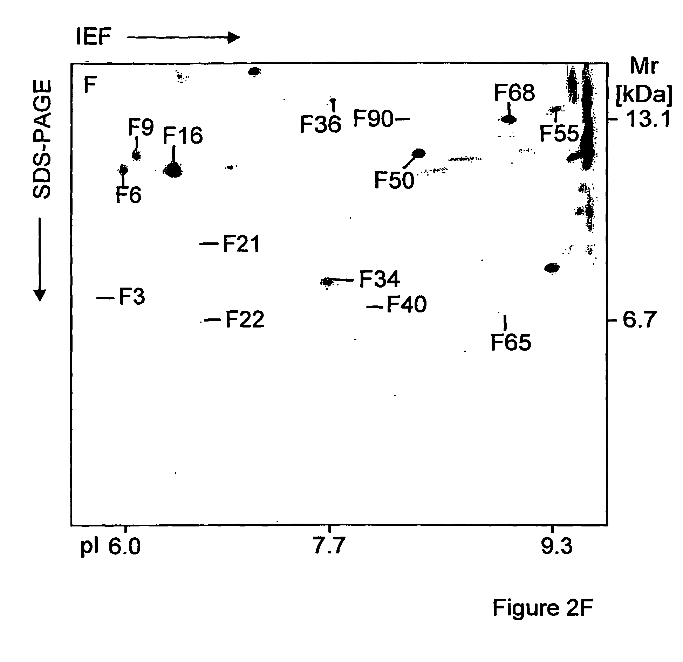

Peptide mass fingerprinting using MALDI- mass spectrometry was used to identify 152 spots of strain 26695. The complete pattern was digitized and subdivided in 6 sectors. Sectors A-F of strain 26695 are shown in Figure 2 A-F. All of the spots marked with numbers were identified. Table 1 contains a systematic protein list, in which the numbers of the spots lead to the protein name and if known to the function of the protein. For Table 1 the classification of *H. pylori* proteins of the TIGR database (http://www.tigr.org/tdb/mdb/mdb.html), which was derived from the classification of *E. coli* (Riley, 1993), was used.

The 152 identified protein spots represented 126 genes. Several proteins appeared in horizontal spot series resulting from protein species of one protein with differently charged side groups caused by posttranslational modifications. One hundred of the identified protein species (67% of all identified proteins) were within the 10% most intense silver-stained spots of the 26695 strain. Except for two, all of the twenty most intense spots were identified (Table 3). The two not identified spots were not stained by Coomassie Brilliant Blue. The first five most intense spots clearly dominated the pattern and were, in order of decreasing intensity: GroEL, UreB, TsaA, GroEL, and CagA. GroEL and UreB contributed 4 and 3 spots, respectively, which correspond to different protein species, these were all included in the list of the 20 most intense spots.

The 126 identified proteins represent about 8% of the total number of 1590 genes predicted from the genome (Tomb et al., 1997). The identified proteins are dispersed over nearly all protein classes. One pair of paralogous proteins was identified: CeuE HP1561 and CeuE HP1562. The following protein classes are underrepresented by the identified 126 proteins, with a percentage below 8% of the predicted number of ORFs: Biosynthesis of cofactors, prosthetic groups, and carriers, transport and binding proteins, DNA metabolism, cell envelope, cellular processes, and other categories. More than 20% of the predicted proteins of a certain protein class were found in the following protein classes: Central intermediary metabolism, energy metabolism, transcription, protein fate, and unknowns. More than 40% of the predicted members of a protein family were found in the following protein families: Pyridoxine; glutathione; anaerobic proteins; TCA cycle; chromosome-associated proteins; DNA-dependant RNA polymerase; transcription factors, translation factors; protein folding and stabilization; degradation of proteins, peptides and glycopeptides; surface structures; detoxification. Several well-known virulence factors were identified (Table 4) and contribute to the most intense spots of the 2-DE pattern of *H. pylori* 26695 under the chosen growth conditions.

### 2.2 PH dependent protein composition

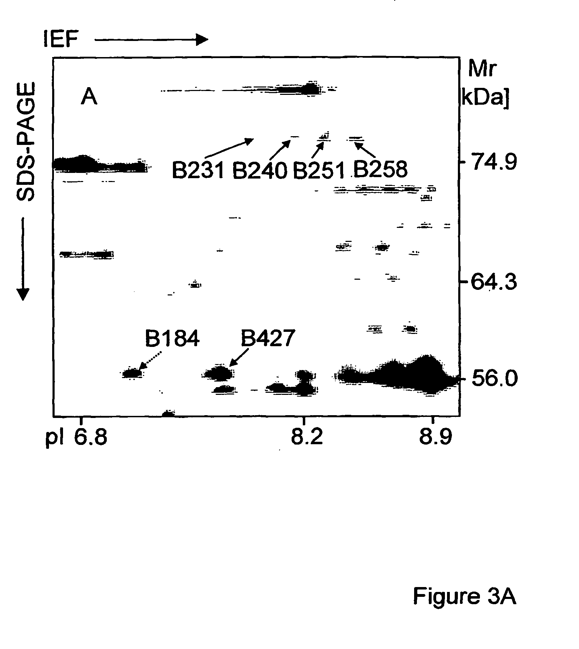

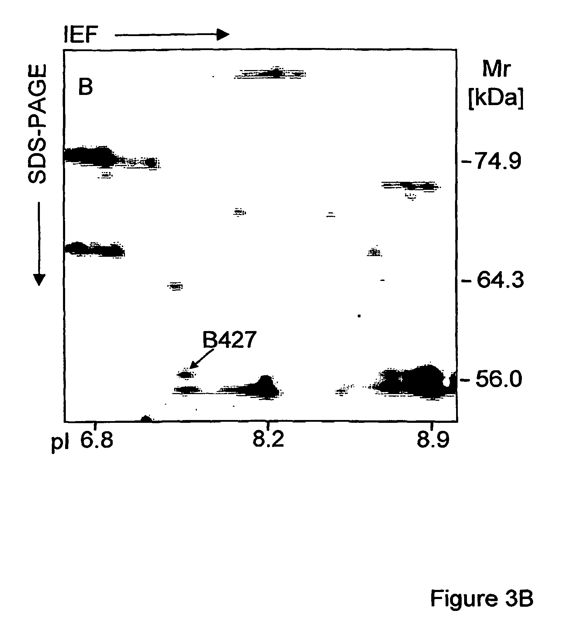

*H. pylori* is a microorganism capable of growing under extreme acidic conditions in the presence of urea (Segal et al., 1992; Solnick et al., 1995). We studied the effect of pH on protein composition by growing *H. pylori* on agar plates with pH values between 5 and 8. Several differences in the protein composition of bacteria grown at these conditions were observed. Five of the differences detected are shown in Figure 3. The spot intensities were measured after scanning the images, performing spot detection with the evaluation program Topspot and adding the pixel intensities within one spot (Table 5). Patterns were normalized on 10 spots predicted to be constant in intensity. The mean intensity value and variation coefficient were calculated from three experiments each, starting with three independent *H. pylori* cultivations per pH value. Spot 1 decreased in intensity with decreasing the pH value from 8 to 5. It was identified as serine protease HtrA (HP1019). Decreasing the pH from 8 to 5 decreased spot 5 in intensity and spots 2-4 were completely absent at pH 5. These proteins were identified as different protein species of the vacuolating cytotoxin (HP0887).

### 2.3 Antigens detected by human sera

SDS-PAGE is a common method for the detection of antigens. Unfortunately its resolution power is optimal for protein mixtures of up to only 100 protein species. Therefore a clear assignment to a certain protein species is often not possible if the expected complexity is above 100. *H. pylori* extracts contain at least 1800 protein species (Figure 1), therefore, high-resolution 2-DE is required to detect and identify antigens on the protein species level.

Sera from 3 patients were used to detect antigens on blots of 2-DE separated *H. pylori* 26695 proteins. The first patient (MP54) suffered from ulcus ventriculi and gastritis and was *H. pylori* positive as determined by histology, urease test, culture, and a high ELISA titer. The second patient (MP44) had a clinical diagnosis of adenocarcinoma of the stomach and stomach cardia, the *Helicobacter* status was unclear because of a positive urea test, negative histology and culture, a non-specific, directed against IgG, high ELISA titer and a specific, directed against *H. pylori* IgG and IgA negative ELISA titer. The third serum was from a patient, who was clearly *H. pylori* negative by all of the above criteria and had a clinical diagnosis of chronic antrum gastritis.

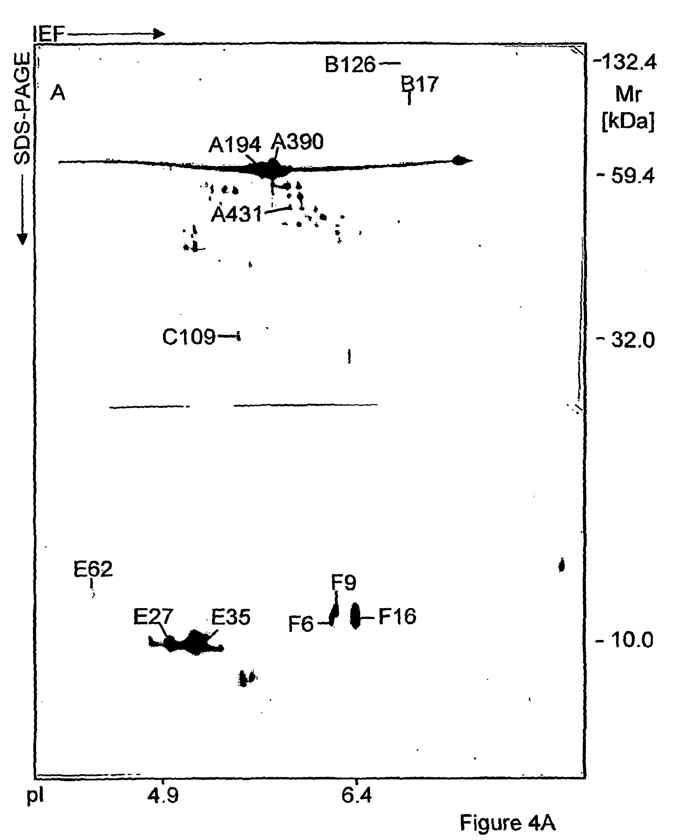

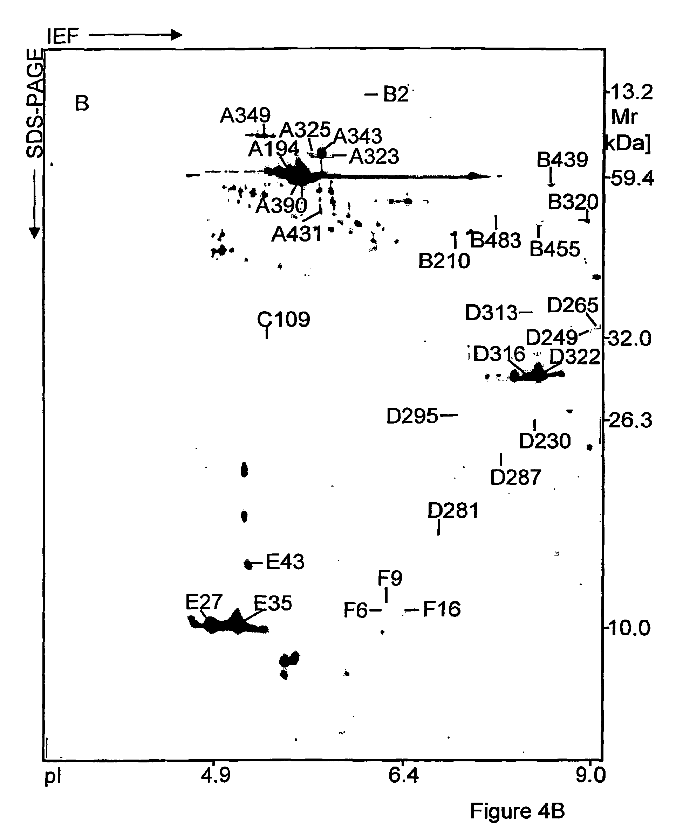

Antibodies bound to antigens were detected with a secondary antibody against human lg and visualized with an ECL system. The serum of the *H. pylori* negative patient reacted only with some of the most abundant *H. pylori* proteins on the 2-DE pattern including GroEL, urease α subunit, and catalase (Table 6). The intensity of these spots on the ECL blots was very low. The other two immunoblots had several additional spots in common (Figure 4a and 4b). The identified antigens are shown in Table 6. The main antigens GroEL and the ribosomal protein L7/L12, both present with several spots in horizontal series, occurred as high intensity spots in both immunoblots. Three GroES spots were detected by both sera (MP44 and MP54), but with a clearly higher intensity on the blot using serum from the ulcer patient. A series of about 60 spots directly below GroEL with high to low intensity common to both immunoblots could only partly be assigned to spots on the silver stained pattern. Only one of them has been identified: spot A431, glutamine synthase. A spot group below the ribososomal protein L7/L12 has the same constellation as in the silver stained pattern but is shifted relatively to the ribosomal protein to the basic side of the pattern. This protein (E41 and E59) is a common antigen of the two sera tested and was identified as thioredoxin. The third component (E45) was not identified. The serum of the patient with adenocarcinoma reacted uniquely with strong signals with GTP binding protein TypA/Bipa, urease α and β subunit, catalase, isocitrate dehydrogenase and the hypothetical protein HP0697. Only flavodoxin (FldA) with middle intensity was unique for the serum of the ulcer patient.

### 3. Discussion

### 3.1 Protein separation and identification

In contrast to other microorganisms, e.g. *Mycobacterium* tuberculosis (Jungblut et al., 1999b) and *Borrelia garinii* (Jungblut et al., 1999a), basic proteins are dominant in all of the 2-DE patterns of *H. pylori* strains investigated. This is in agreement with the high pl-values calculated from the protein sequences deduced from the genes of *H. pylori.* More than 70% of the predicted proteins in *H. pylori* have a calculated pl greater than 7.0 compared to about 40% in *Haemophilus influenzae* and *Escherichia coli* (Tomb et al., 1997). Despite the fact that only 98 genes of strain 26695 are absent in J99, there are only 41 with perfect identity and only 310 with more than 98% amino acid conservation between these two strains (Alm et al., 1999).

Alm et al. predicted 1552 and 1495 open reading frames for strain 26695 and J99, respectively. The protein patterns revealed 1863 and 1622 protein species, respectively, for these two strains. Within 152 identified spots, 126 (83%) open reading frames were represented. If the same percentage is assumed for the total detected spot number a gene number represented on the 2-DE patterns of 1546 and 1346 for 26695 and J99, respectively, may be predicted. This is only slightly below the total predicted gene numbers. However, one should be aware of the fact that not all of the proteins will be present in the biological situation studied here and that a high dynamic range of protein amount is to be expected for the different protein species, which may not be covered by silver staining. For a middle-sized protein, about 1000 molecules per cell are necessary to be detected by silver staining on a 2-DE gel, if 10⁷ cells are applied to the gel.

The significance of the proteome approach for confirmation of predicted protein species has been emphasized (Humphery-Smith et al., 1997). To date our study revealed expression of 27 conserved hypothetical proteins and 6 unknowns, not described previously at the protein level.

### 3.2 Genetic variability at the proteome level

A comparison of 10 spots with the same or nearly the same position on the 2-DE pattern has shown that one amino acid exchange causing a change of pl of 0.05 units results in a clearly detectable shift in the 2-DE pattern. The influence of the proteins on the pH- gradient in ampholyte isoelectric focusing under non-equilibrium conditions is irrelevant. Proteins with identical sequence occur at the same position within the two compared patterns, as was the case for the three proteins thioredoxin, GroES, and urease β subunit. Thus, these patterns may serve as references for the assessment of post-translational modifications and virtual patterns of ORF's determined by DNA sequencing from other strains may be established.

The extreme genome plasticity predicted by pulsed field gel electrophoresis (Jiang et al., 1996) was relativated by the overall conservation in genomic organization and gene order (Alm et al., 1999). The proteome analyzed by 2-DE shows now again a high variability. But considering the high sensitivity of isoelectric focusing against exchanges of single amino acids this variability may reflect the exact chemical structure of the protein species and not the differential presence of the protein as defined by its function. The 2-DE approach analyses the genetic variability at the protein species level. Further identifications of protein species of different *H. pylori* strains will reveal the variability at the protein level.

### 3.3 PH dependent protein composition

*H. pylori* has the capability to survive under extremely acidic conditions. This survival is mediated by the production of urease (Evans et al., 1991; Clyne et al., 1995). However, both urease negative mutants survived a 60 min exposure at pH 3.5 (Clyne et al., 1995) and urease positive, acid sensitive mutants exist (Bijlsma et al., 1998) showing the existence of additional mechanisms for acid resistance. Proteome analysis will help to reveal factors at the protein level, which contribute to the survival of *H. pylori* in the stomach. These factors are *per definitionem* virulence factors. Decreasing the pH of the growth medium from pH 8 to pH 5 decreased the amount of vacuolating cytotoxin VacA (Hp0887) and serine protease HtrA (Hp1019). Whereas VacA is a well-known virulence factor (Cover and Blaser, 1992), HtrA has not been described before to have this role/activity. An increased secretion of these proteins may explain the decrease of protein amount within the cell during acidification. VacA is activated by decreasing the pH to 5.5 (de Bernard et al., 1995). This activation may also be accompanied by an increased secretion and therefore decrease of VacA concentration within the cell. Both vacuolization of the surface epithelium and the destruction of the protective mucus layer by proteases are important activities during the pathogenesis of *H*. *pylori.* These two proteins represent only two obvious differences in the patterns obtained from different pH during cultivation. The detection and identification of further variants will give more detailed information on the molecular mechanisms of the survival of *H. pylori* within an acidic surrounding.

### 3.4 Antigens detected by human sera

The aim to correlate antibody recognition of certain *H. pylori* antigens with clinical manifestations of disease and to identify vaccine candidates has prompted several groups to undertake immunoblot analyses using panels of sera from infected and noninfected patients (Faulde et al., 1992; Mattsson et al., 1998; Faulde et al., 1993; Nilsson et al., 1997; Klaamas et al., 1996) for original publications and (Zevering et al., 1999) for a comprehensive overview). Given the complexity of the *H. pylori* genome, conventional SDS-PAGE and immunoblot analyses yielded mostly information on the molecular weight of immunoreactive bacterial proteins and not on sequence information. Methodological differences and the use of antigen preparations of different *H. pylori* strains hampered a comparison between the different studies. Nonetheless, several proteins with the respective Mr recognized by serum antibodies were identified including CagA (110-120 kDa), VacA (87 kDa), urease α and β subunit (67 kDa and 31 kDa respectively), GroEL (60 kDa), flagellins (50 kDa), p35 (35 kDa) and a 26 kDa antigen. Antibodies against CagA, VacA and the 35 kDa antigen suggested infection with a type I strain (Xiang et al., .1995) and were likely correlated with development of ulcers (Telford et al., 1994; Weel et al., 1996; Atherton et al., 1997; Aucher et al., 1998; Lamarque et al., 1999).

We detected numerous antigenic proteins in Hp26695 with individual sera from patients with a history of *H. pylori* and were able to determine the identity of a subgroup using a proteomics approach (Table 6). Recently, similar 2-DE analyses of *H. pylori* ATCC 43504 (McAtee et al., 1998b) and *H. pylori* G27 (Kimmel et al., 2000) was employed to identify antigens by immunoscreening using pooled or individual sera from infected patients. Three known antigens, urease β subunit, chaperonin GroEL, and isocitrate dehydrogenase I*cd* were detected in all three studies and 15 of the antigens in at least two of the studies. The fact that some of these proteins were used with success in vaccination studies in animal models of *H. pylori* infection (Kleanthous et al., 1998) strongly supports 2-DE as an approach for identification of vaccine candidates. This reasoning led to the identification of a protein with homology to *Campylobacter jejeuni* cell binding protein 2 (McAtee et al., 1998c) an ORF that was also present in *H. pylori* 26695, HP0175. The antigenicity of this gene product was confirmed here. However, only one of the sera recognized the spot corresponding to this protein, indicating either that not all patients react to this protein or that *H. pylori* strains exist that lack the respective gene or have an orthologous gene with modified sequence. The enormous genetic variation in *H. pylori* suggests that the latter two explanations are more likely. The sera used in the present study, reacted with a characteristic pattern of proteins with apparent molecular weights in the range of 40-60 kDa and pls of 4,9-6,4. Proteins with similar relative coordinates were also identified by McAtee et al. (McAtee et al., 1998b) and Kimmel et al. (Kimmel et al., 2000). Similarly, a set of three antigens is detected displaying a Mr below 10 and a pl of 5.1. We have identified these proteins as candidate vaccine antigens for further study. These results prompted us to perform an extended analysis of antigenic proteins recognized by a large number of individual sera that will lead to the identification of proteins of diagnostic value and also potential vaccine candidates (Haas et al., in preparation). It is of note that all proteins with vaccine efficacy tested in animals to date are abundant proteins that are also detected by immunoblot analyses (compare with Table 3). This suggests that the frequency of a protein is a key criterion to identify it as a vaccine candidate and that despite the fact that antibodies may not be protective they discriminate between bacterial proteins accessible to the immune system and those that are not.

### 3.5 Virulence factors

Virulence factors are defined as gene products that are indispensable for colonization and host to host transmission competence of a pathogen and may include gene products that are important pathogenic factors (for review see (McGee and Mobley, 1999)). The growing list of those identified of *H. pylori* includes (i) proteins involved in adhesion such as BabA that mediates binding to Lewis^{b} antigen and might be a key factor in the pathogenesis of duodenal ulcer and adenocarcinoma (Ilver et al., 1998; Gerhard et al., 1999), AlpA and AlpB (Odenbreit et al., 1999) that are members of a large family of related outer membrane proteins (Tomb et al., 1997), the sialic acid lectin HpaA (HP0410), a lipoprotein (Evans et al., 1993) which may contribute to adherence factors detected by hemagglutination or adherence assays, (ii) proteins required for motility (Bijlsma et al., 1999) such as flagellins, (iii) factors involved in acid neutralization such as urease or detoxification of aggressive oxygen metabolites such as catalase and super oxide dismutases, (vi) proteins involved in iron uptake and storage such as a lactoferrin-binding protein, siderophores and potential periplasmic iron binding proteins such as CeuE or ferritin orthologs such as NapA or Pfr (Frazier et al., 1993; Doig et al., 1993; Evans et al., 1995) (v) proteins involved in pathogenicity such as the cag pathogenicity island encoded proteins or the vacuolating toxin VacA.

The reference strain 26695 is deficient in several of the above mentioned virulence factors: it does not produce functional BabA (Ilver et al., 1998), lacks immunoreactive flagellins (McAtee et al., 1998b) and we have observed some subclones with very variable levels of catalase activity. Many proteins belonging to the aforementioned classes of virulence factors were easily identified in our 2-DE analysis (Table 4) and the urease subunits, Cag26, catalase, and GroES were also recognized by at least one of the sera. Though there is no paucity in the detection of cell envelope proteins, the class of outer membrane proteins both on silver stained gels and on immunoblots is only represented by outer membrane protein HP1564 within the identified proteins. The list of the 20 most abundant proteins (Table 3) contains additional to known virulence factors the protein TagD, which is described in the NCBI sequence database as an adhesin and NapA, which was mentioned as immunogenic *H. pylori* protein by Kimmel et al (Kimmel et al., 2000).

### Example 2 (for illustrative purposes only)

### 1. Methods

### 1.1. Patients

In this study, one hundred and thirty-eight patients with gastric disorders who underwent gastroduodenal endoscopy in the Virchow-Charite Hospital in Berlin were screened for serum antibodies against *H. pylori.* Clinical parameters leading to diagnosis, history of previous *H, pylori* infection, treatment of *H. pylori* infection, clinical disorders, further medication and diseases were recorded. The study has been approved by the ethical committee of the Virchow Hospital. Four main parameters were then evaluated for the diagnosis of *H. pylori:* histology, culture, CLO test in the gastric biopsies and serum antibody titers. Patients were classified as Helicobacter positive (*H. pylori*), when three of the criteria tested turned out to be positive. Only 24 patients were clearly *H. pylori* positive at the time of the blood sample (**Table 10**). Twelve patients who had gastric disorders unrelated to *H. pylori* and did not show any sign of prior infection were considered negative. From the six cancer patients, 5 patients had gastric cancer and one had MALT (mucosa associated lymphoid tissue) lymphoma of the upper gastric tract. One of these patients was *H. pylori* positive, one had previous infection, one showed a high antibody titer in ELISA and four including the already mentioned patients had positive CLO tests, which could, however, also be related to other bacterial infections. For 4 of these cancer patients, previous *H. pylori* infection was not documented because of incomplete records. In total, 5 of these 42 patients had previous *H. pylori* infection and 6 had already once successful eradication therapy. For 6 additional patients with unclear diagnosis of *H. pylori* because of missing records, samples were investigated because they had either precancerosis or previous *H. pylori* infection. Data from these patients were, however, not evaluated as a group.

### 1.2 Helicobacter pylori growth conditions

*H. pylori* Hp26695 was used because its genome has been entirely seuquenced (Tomb, White, et al., 1997). *H. pylori* strains were grown on serum plates (Odenbreit, Wieland et al., 1996) at 37°C in a microaerobic atmosphere (5% O₂, 85% N₂ and 10% CO₂) for two days. The bacteria were harvested, washed twice in ice-cold PBS containing proteinase inhibitors (1 mM PMSF, 0.1 µM pepstatin, 2.1 µM leupeptin, 2.9 mM benzamidin), and lysed by resuspension in half a volume of distilled water. The resulting volume in µl was multiplied by i) 1.08 to obtain the amount of urea in mg to be added, ii) 0.1 to obtain the volume of 1.4 M DTT and 40% Servalyte (Serva, Heidelberg, Germany) pl 2-4, CHAPS was added to obtain an end concentration of 1 %. The end concentrations of DTT and urea were 70 mM and 9 M, respectively. Solubilization of the proteins occured within 30 min at room temperature. A protein concentration of 15 µg/µl +/- 25% was obtained.

### 1.3 Two-dimensional electrophoresis

*H. pylori* proteins were resolved by a 7 cm x 8.5 cm 2-DE gel system (Jungblut and Seifert, 1990) with a resolution power of about 1,000 protein species. Twenty µg of protein were applied to the anodic side of the IEF gel. In the second dimension 1.5 mm thick gels were used. The proteins were detected by silver staining optimized for these gels (Jungblut and Seifert, 1990). For the identification of proteins and immunoblotting, 20 µg of protein were applied. The proteins were stained by Coomassie Brilliant Blue R250 (Eckerskorn, Jungblut et al., 1988) or G250 (Doherty, Littman et al., 1998).

### 1.4 Peptide mass fingerprinting

Peptide mass fingerprinting was performed as previously described, Lamer and Jungblut, subm.). Optimised conditions including volatile buffer, decreased trypsin concentrations, and using volumes below 20 µl allowed for the identification of weakly stained Coomassie Blue G-250 protein spots starting with only one excised spot. The peptide solution was mixed with an equal volume of a saturated α-cyano-4-hydroxy cinnamic acid solution in 50% acetonitrile, 0.3% TFA and 2 µl were applied to the sample template of a matrix-assisted laser desorption/ionization mass spectrometer (Voyager Elite, Perseptive Biosystems, Framingham, MS, USA). Data were obtained using the following parameters: 20 kV accelerating voltage, 70% grid voltage, 0.050% guide wire voltage, 100 ns delay, and a low mass gate of 500.

Peptide mass fingerprints were searched using the program MS-FIT (http://prospector.ucsf.edu/ucsfhtml/msfit.htm) by reducing the proteins of the NCBI database to the *Helicobacter* proteins and to a molecular mass range estimated from 2-DE +/- 20%, allowing a mass accuracy of 0.1 Da for the peptide mass. In the absence of matches, the molecular mass window was extended. Partial enzymatic cleavages leaving two cleavage sites, oxidation of methionine, pyro-glutamic acid formation at N-terminal glutamine and modification of cysteine by acrylamide were considered in these searches.

### 1.5 Immunoblotting

For immunostaining the proteins were transferred from the 2-DE gels onto PVDF membranes (ImmobilonP, Millipore, Eschborn, Germany) by semidry-blotting (Jungblut, Eckerskorn et al., 1990) using a blotting buffer containing 100 mM borate, 20% methanol, pH 9.0. The blotting time was 2 h with a current of 1 mA/cm². Next, the membrane was blocked with 5% skim milk, 0.05% Tween-20 in PBS for at least 1 h at room temperature. After blocking the membrane was washed 3 times for 5 min. All washing steps were performed with PBS, 0.05% Tween 20. Antigens were detected by incubation of the membranes with human sera for one hour in a dilution of 1:200, followed by a secondary antibody (anti-human polyvalent immunoglobulins, G, A, M, peroxidase conjugated, Sigma A-8400, Deisenhofen, Germany) at a dilution of 1:10000. Before and after addition of the secondary antibody the membranes were washed 4 times for 15 min in PBS, 0.05% Tween 20. To visualize the results, the washed membrane was incubated with 30 ml/membrane of a 1 : 1 mixture of Enhanced Luminol Reagent and Oxidizing Reagent for 1 min (Renaissance Western Blot Chemiluminescence Reagent for ECL Immunostaining (NEN, Köln, Germany). The detection reagent was drained off and the membrane wrapped in a plastic foil was then exposed to Kodak BioMax MR1 film for 5 min.

### 1.6 Evaluation of blots and gels

Initial analysis was performed as previously described (Jungblut, Grabher, et al., 1999). Briefly, the silver stained pattern of a small gel was used as a master pattern, with each of the spots numbered. The protein pattern corresponded to the silver stained pattern of larger gels described previously (Example 1). Spot detection was performed by the TopSpot evaluation program (Algorithmus, Berlin, Germany). After automatic segmentation and spot detection, all additional visually detected protein spots were introduced into the master pattern interactively. Each spot that reacted with the different sera was numbered and compared to the silver stained spots for further identification. The optical density of the immuno-reactive spot was classified into five categories: not detectable -, very low staining 0.5, low staining 1, staining 2, intensive staining 3, very intensive staining 4. For each group of sera a signal frequency in % was calculated as the mean intensity grade of each serum (0.5, 1, 2, 3, 4) divided by the optimal reachable value (n x 4), where n = number of sera.

### 1.7 Statistical evaluation:

Statistical analysis was performed using the software Systat 9 for Windows. For the analysis of discrete data and rates, *X*²-square analysis was used. Mean intensities of spots in the different groups of immunoblots were compared using a non-parametric test (Kruskal-Wallis test) and significance was as indicated (p < 0,05).

### 2. Results

### 2.1 Heterogeneous immunoreactivity pattern revealed by 2-DE immunoblots.

In order to correlate *H. pylori* antibody recognition with the disease and to identify highly immunogenic vaccine candidates, we evaluated patients with *Helicobacter* related disease and compared them to a control group of *H. pylori* negative patients with unrelated gastric disorders and a group of cancer patients, who all underwent gastric endoscopy (Table 10). Sera from individual patients were used for western blot analysis to detect antigens from the *H, pylori* strain 26695 separated on 2-DE gels and blotted onto PVDF membranes. The pattern of protein spots recognized on these immunoblots were compared with a 2-DE pattern of spots on a standard silver stained gel and spots are marked with a code consisting of a letter that represents the corresponding sector on the standard gel (A-F) and an attributed number. Identification of detected protein spots was performed by in gel digestion and MALDI-MS analysis. The identified protein species were named according to the classification of *H. pylori* proteins in the TIGR database (Tomb, White, et al., 1997). Protein species can be accessed by name or by spot number according to Table 1.

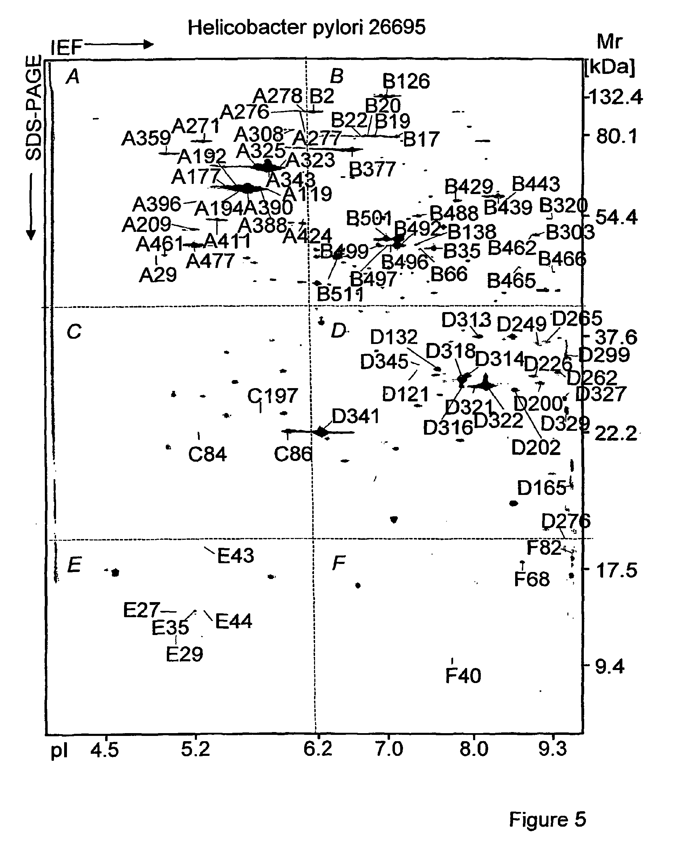

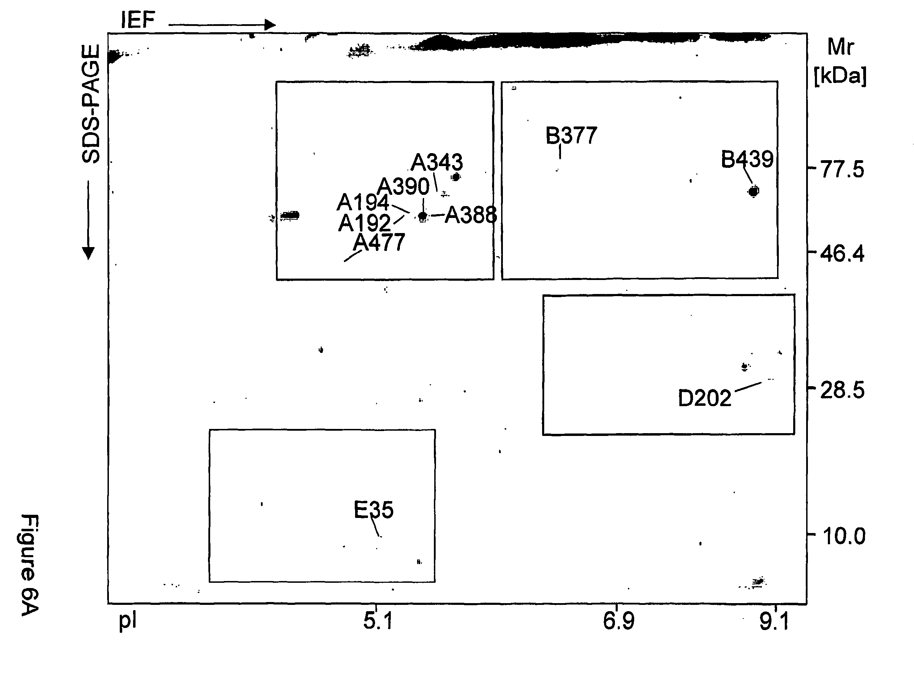

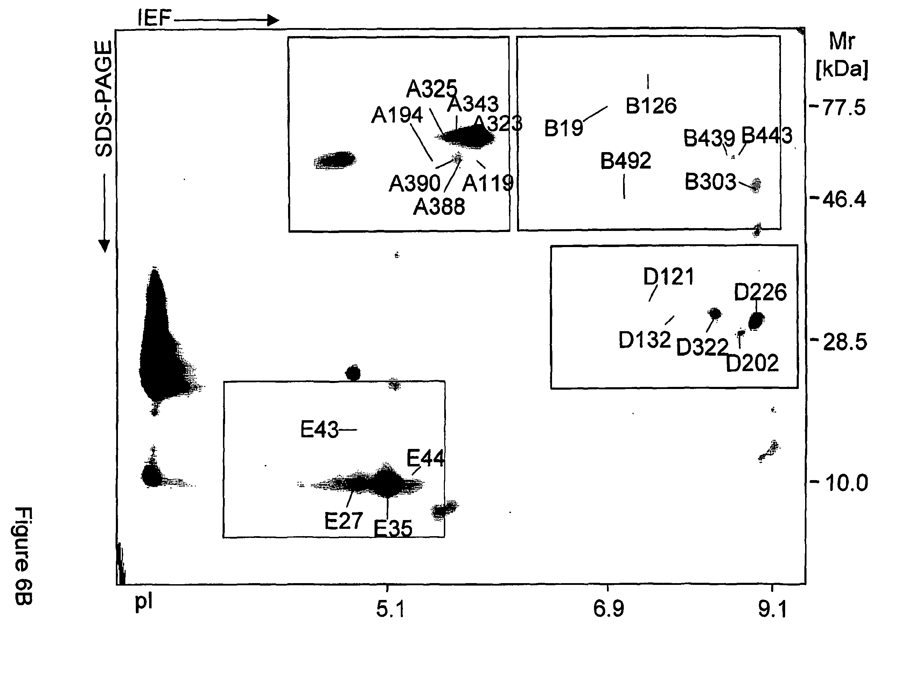

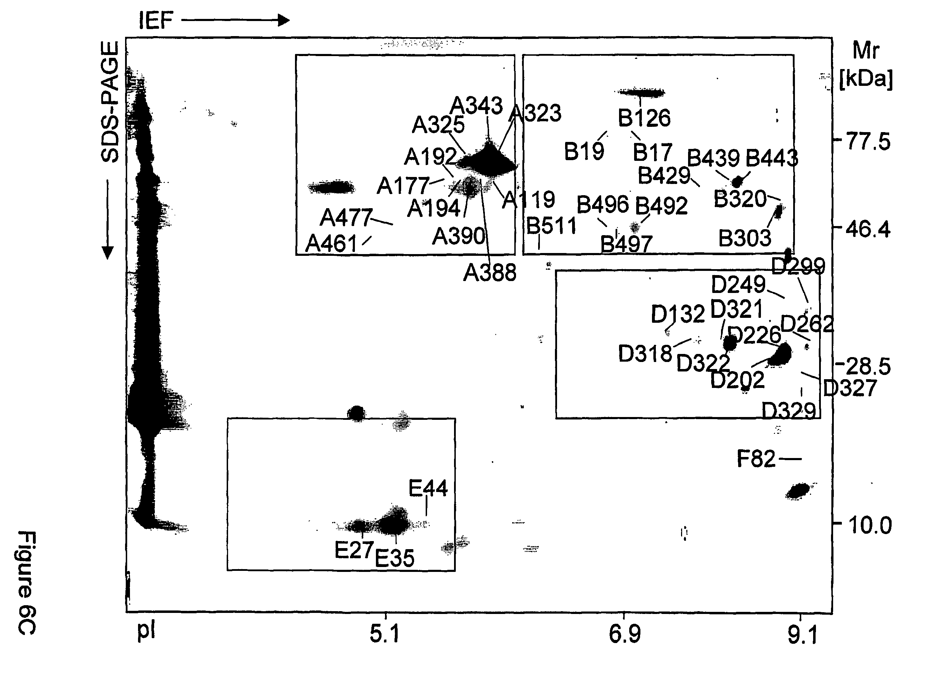

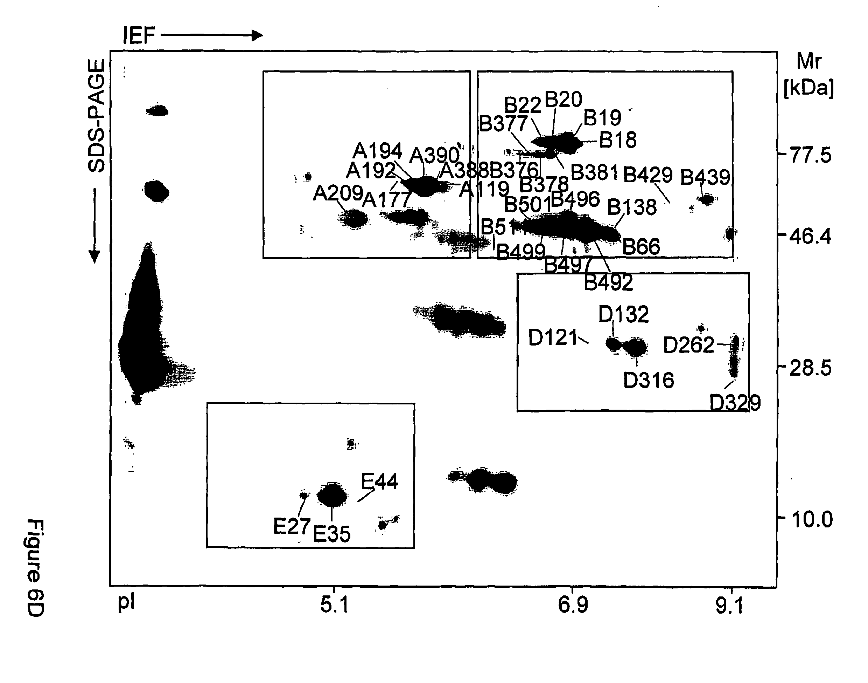

Threehundred seventy nine antigen spots from *H. pylori* 26695 could be detected by immunoblots on silver stained 2-DE gels and 156 have been identified so far. The intensity of a particular spot was assigned on an arbitrary scale between 0,5 and 4. Similar to the silver stained gel pattern, individual spots on the immunoblots were distributed over the whole pl range of 4-10 and the whole Mr range of 5-150kDa. However, more spots seemed to be immunoreactive in the basic range (Figure 5 and Figure 6). We have also observed that several protein species accumulated in the low pl range and have not migrated into the IEF gel. The significance of this observation is not yet clear and needs further investigation. Examples for individual immunoreactive pattern are shown in Figure 6 and include sera from an *H. pylori* negative patient (Figure 6A), patients with *H. pylori* related gastritis or ulcer (Figure 6B and C) or a cancer patient (Figure 6D). Between 3 and 153 spots were stained on the individual immunoblots. Although the spot patterns were quite heterogeneous, two features were observed: most protein spots that reacted on the blots with the gastritis, ulcer or cancer sera were clearly more intense than spots reacting with *H. pylori* negative sera and a higher number of spots was detected, indicative of higher antibody titers directed against a greater number of antigens. Spots that could be identified and are mentioned in the tables and in the text are marked. As an example, spot E35 corresponding to 50-S ribosomal protein L7/L12 is found in the lowest left segment of the silver gel and on all four immunoblots shown (Figure 5 and 6).

We detected seven series of spots that were prominent in the silver stain and corresponded to major antigens of *H. pylori.* The GroEL protein (HP0010, main spot A390), 50-S ribosomal protein L7/L12 (HP1199, main spot E35) and catalase (HP0875 main spot B439) reacted with sera from all four patients shown (Figure 6 A-D). Urease B subunit (HP0072, main spot A343) reacted with the negative, gastritis and ulcer sera, whereas the spot series from Cag 26 (HP0547, spot B126) was mainly stained with the gastritis or ulcer sera (Figure 6 B and C). Isocitrate dehydrogenase (HP0027, main spot B492) and the putative neuraminyl-lactose-binding protein HpaA (HP0410, main spot D132) reacted with the gastritis, ulcer and cancer sera, respectively (Figure 6 B-D), but not with *H. pylori* negative sera. All these series of spots were found useful for orientation in the 2-DE silver stain and in the immunoblots.

### 2.2 Comparison of antibody recognition in sera from H. pylori positive and negative patients.

In order to detect highly immunogenic antigens of *H. pylori,* we first concentrated on the most abundant and intense spots detected in the silver stains that were also strongly recognized by sera from the 24 *H. pylori* positive and 12 negative patients. A signal frequency was calculated by dividing the arithmetric mean of intensities through the (maximal reachable value of intensities x 100)(Jungblut, Grabher, et al., 1999). Table 11 lists spots and corresponding proteins that were recognized above a threshold of 10% by sera of *H. pylori* positive patients or spots with a significantly higher occurrence or intensity of recognition in sera from the positive group compared to negative controls (p<0,05). In total, 310 of the 379 protein spots were recognized by *Helicobacter* positive sera while 156 spots were recognized by negative sera and 272 spots showed a higher frequency in the positive group of sera. The number of spots stained by individual positive sera was widely spread (range 7 to 1 53) with a mean of 62, while negative sera stained with a range from 3 to 66 and a mean of 24 spots. Thirty-two antigens (Table 11) were recognized by positive sera with a signal frequency over 10%. The major antigens recognized by sera from *Helicobacter* positive patients were the 50 S ribosomal protein L7/L12 (HP1199, spot E35), catalase (HP0875, spot B439), GroEL(HP0010, spot A390), Cag 16 (HP0537, spot B466), Cag26 (HP0547, spot B126), UreaseA (HP0073, spot D322) and Urease B (HP0072, spot A343). Except Cag16, all these spots were stained in blots B and C (Windows of sector A, B, D and E in Figure 6). In sera from *H. pylori* negative patients, best recognition was achieved for the 50 S ribosomal protein L7/L12, UreaseA, catalase and the conserved hypothetical secreted protein HP1098 (spot D262). Staining of the 50 S ribosomal protein L7/L12 (spot E35) and catalase (spot B439) are shown on blot A of Figure 6 as well.

Fourteen protein species reacted at most with one negative serum with an intensity of 0,5 (Table 11). These proteins preferentially recognized by *H. pylori* positive sera. Five proteins were only recognized by positive sera: a predicted coding region, the serine protease HtrA, Cag 3, CLPB and the trigger factor HP0795. From the fourteen preferentially recognized protein species, the following reacted most frequently in the immunoblot pattern (Table 11): a predicted coding region (HP0231, spot D226) with 11/24 sera, the serine protease HtrA (HP1019, spot B429) with 9/24 sera. Fumarate reductase (HP0192, spot B17) and the Cag 26 protein as a typical marker for the subgroup of type I *Helicobacter* strains were recognized by 8/24 sera. Cag 3 (spot B443), CLPB (HP0264 spot A308) were recognized by 7/24 sera, the trigger factor HPO795 (spot A411) by 6/24 positive sera. Two spots of isocitrate dehydrogenase: B496 (not shown) and B497 (Table 11) were only recognized by positive sera, whereas the main spots B492 and B499 were recognized by both positive and negative sera (not shown). Three other protein species with a frequency below 10 that seemed restricted to serum recognition by *H. pylori* positive patients, correspond to the proteins aconitate hydratase (HP0779, spot B2), hydantoin utilisation protein A (HP0695, spot B377), or DnaK (HP0109, spot A359) which were all recognized at most by one serum from the control group with the lowest intensity. The so far unidentified protein spot E53 was also only recognized in one negative serum. Different combinations of these proteins are recognized on individual blots: fumarate reductase (spot B17), Cag 26 (spot B126), Cag 3 (spot B443) are found in the window of sector B from the blot stained with the gastritis and the ulcer serum, whereas the predicted coding region (HP0231, spot D226), CLPB (HP0264 spot A308) and the trigger factor HP0795 (spot A411) were only stained on gels obtained from other gastritis and ulcer patients.

In conclusion, antibodies from *H. pylori* positive patients recognized more proteins species with higher intensity compared to the control group, but only few antigens were specific.

### 2.3 Differences in protein recognition associated with disease manifestation

The *H. pylori* infected patients were diagnosed either with gastritis or ulcer. Antibodies from infected ulcer patients recognized more protein species than did those from gastritis patients. In most cases, the intensity of recognition in the immunoblots was also much higher. Therefore, a statistical analysis on the recognition pattern of sera from the 15 patients with *H. pylori* associated gastritis and the 9 patients with either gastric or duodenal ulcer was performed. Protein species which were detected with higher occurrence or intensity in one compared to the other two disease manifestations (p < 0,05), are shown in Table 12. As only 3 patients had duodenal ulcer, no further analysis was possible for the two types of ulcer.

The following proteins with dominant recognition by ulcer sera compared to gastritis sera were detected: a conserved hypothetical protein (HP1285, spot D327), a hypothetical protein precursor (HP0175, spot D249), a signal recognition particle protein (HP1152, spot B320), fumarate reductase (HP0192, spot B17), one spot from the 50s ribosomal protein L9 (HP1302, spot F68) and one from the 30s ribosomal protein S5 (spot F82), elongation factor TufB (HP1205, spot A477),cag 16 (HP1133, spot B466) and the not yet identified protein species B465. From the proteins isocitrate-dehydrogenase and 50S ribosomal protein L7/L12 several protein species were preferentially recognized, while other spots from these proteins did not show significant differences in recognition. There are few antigens as the putative neuraminyl-lactose-binding protein HpaA (HP0410, spot D121 in Figure 6) or the 26kD antigen (HP 1563, spot D341) which is known as a strong antigen in *Helicobacter* infection which are less recognized in ulcer compared to gastritis sera (Table 12). The recognition of 11 protein species was significantly higher by sera from patients with ulcer compared to the gastritis group (p<0,05). Eight protein species showed a significantly higher mean intensity (p<0,05) and 7 fulfilled both criteria.

Taken together, antigen recognition pattern varied strongly among sera from patients with *H. pylori* positive gastritis, but recognition seems to increase with severity of disease, to be strongest and most intense in sera from *Helicobacter* positive ulcer patients.

### 2.4 Antigen recognition pattern in Cancer patients

To evaluate whether the recognition pattern of *H. pylori* antigens differ between the patients with active gastritis or ulcer and cancer patients, antigen recognition by sera from the 15 patients with *H. pylori* related gastritis, and the 9 patients with *H. pylori* related ulcer was compared with recognition by 6 cancer sera. In most cases, the recognition pattern of cancer sera and ulcer sera were similar. The metabolic enzymes isocitrate-dehydrogenase (HP0027, main spot B492) and fumarate reductase (HP0192, spot B17), a conserved hypothetical protein (HP1285, spot D327), a hypothetical protein precursor (HP0175, spot D249), and elongation factor TufB (HP1205, spot A477), were recognized by more than half of the sera in both groups (Table 12). In several cases, the intensity of antibody recognition in sera from cancer patients was higher compared to ulcer sera, as shown for isocitrate deydrogenase spot **B499** in **Figure 6 C and D** and for the hypothetical protein (HP0305, spot D276) in Table 12. In addition, recognition of several proteins was dominant in sera from cancer patients compared to ulcer and gastritis sera: aliphatic amidase (HP0294, spot B511), cinnamyl-alcohol dehydrogenase (HP1104, spot B35) and the putative neuraminyl-lactose-binding protein HpaA (HP0410, spot D121), DNA- directed RNA polymerase *α* chain (HP1293, spot A29), ATP dependent Clp protease proteolytic subunit (HP0794) and for hydantoin utilization protein A (HP0695, spot B377) compared to gastritis sera (Table 12). As only six patients were tested, statistical significance could not be achieved for the pyridoxalphosphate biosynthetic protein J (HP1582, spot D314), a conserved hypothetical secreted protein HP 1098 (spot D262) and the outer membrane protein HP1564 (spot D202) (shown in Table 13), but frequencies of antibody recognition were above a threshold of 10 that was used in the comparison of positive and negative sera.

Of the 234 recognized protein species, 52 showed a higher antibody recognition frequency in the cancer group than in the *H. pylori* infected ulcer and gastritis group. The corresponding proteins with a frequency > 10 are shown in **Table 13** as well as carbonic anhydrase (HP1186, spot D200), a hypothetical hit-like protein (HP0404, spot F40), thioredoxin (HP0824, spot E29), elongation factor G (HP1195), spot A271) and superoxidase dismutase (HP0389, spot C197) which were recognized only by one or two cancer sera but with high intensity. Twenty one protein species were only recognized in cancer sera, although two thirds of them showed a heterogeneous recognition pattern and corresponded to series of proteins, and 11 immunoblot spots could not yet be identified (not shown). In conclusion, most antigens that are recognized in relation to *H. pylori* gastritis and ulcer are also recognized in sera from cancer patients although recognition of some metabolic enzymes was more often related to cancer sera.

### 3. Discussion:

### 3.1 Helicobacter specific antigens

In order to characterize candidate antigens of *H. pylori* for diagnosis and therapy, we have systematically analyzed differences in the *Helicobacter* 2-DE protein recognition profiles of the sequenced strain 26695 by the use of sera from patients with different gastric disorders. Of 1800 protein spots from this strain detected by silver staining, 379 spots reacted with the patients' sera tested and more than 150 of these could be identified. Among the proteins most frequently recognized by H. pylori positive sera, 23 were confirmed from a previous study and other 2DE analyses (Jungblut, Bumann et al., 2000, McAtee, Lim et al., 1998, Kimmel, Bosserhoff et al., 2000) and 10 were newly identified (Tables 14 and 15). Furthermore, antigen recognition that seemed associated with ulcer and cancer was evaluated.

To date, characterization of immunogenic proteins from *H. pylori* was mainly determined based on their molecular mass in immunoblots (Faulde, Schroder et al., 1992, Faulde, Cremer et al., 1993, Klaamas, Held et al., 1996, Nilsson, Ljungh et al., 1997 and reviewed in Zevering, Jacob et al.), while only few studies used 2-DE or 2-DE immunoblots combined with mass spectroscopy to detect antigens from several strains of *H. pylori* (McAtee, Lim et al., 1998, Nilsson, Utt et al., 2000, Kimmel, Bosserhoff et al., 2000, Enroth, Akerlund et al., 2000, Jungblut, Bumann et al., 2000). The technique used here allows standardized high resolution of individual protein pattern and exact identification of antigens reacting with the different sera tested. Another advantage is the separation of protein spots with high pl, thus several antigens in the basic range of the gels could be analyzed. For example, the hypothetical protein HP0305, the outer membrane protein HP 1564, Cag 16 (HP0537) or the protease HP1350 were recognized in more than half of the sera from positive patients. However, some membrane-bound antigens could be missed. In addition, genetic variation could lead to lack of detection of some antigens. To take these limitations into account, proteins which reacted only with sera from a few patients, but with high intensity were also evaluated.

More than two thirds of the spots were more frequently recognized by the sera from the *Helicobacter* positive group of patients compared to negative controls with unrelated gastric disorders. On the other hand, several very abundant proteins from the *Helicobacter* proteome seem to be crossreactive with other bacterial infections: 50 S ribosomal protein L7/L12, catalase, and GroEL could be identified. The abundance of these proteins is shown in other studies using various *Helicobacter* strains and data from other bacterial proteome analyses (McAtee, Lim et al., 1998, Kimmel, Bosserhoff et al, 2000, O'Connor, Farris et al., 1997, Tonella, Walsh et al., 1998). In addition, UreaseA and B, Protease HP1350 and the conserved hypothetical secreted protein HP1098 were recognized by antibodies from *H. pylori* negative patients and correlate with observations of low specificity of *Helicobacter* detection in diagnostic assays that contain recombinant Urease (Widmer, de Korwin et al., 1999).

At this time, the main problem of both commercial and scientific assays used for the detection of *H. pylori* is the relatively low correlation with disease and low specificity (Johansen, Norgard et al., 1995, Widmer, de Korwin et al., 1999). Furthermore, monitoring of eradication therapy by titrating the antibody response is not yet very successful (Nishizono, Gotoh et al., 1998).

Some rather specific antigens as aconitate hydratase (HP0779), a trigger factor HP0795, the stress protein ClpB (HP0364) or the heatshock protein DnaK (HP0109) were also detected in former studies (Kimmel, Bosserhoff et al., 2000, McAtee, Lim et al., 1998). The following five proteins: Cag3, the predicted coding region HP0231, the serine protease HtrA (HP1019), hydantoin utilisation protein A (HP0695) and fumarate reductase (HP0192) seem to be new specific antigens for *H. pylori* and are among the 150 most abundant protein species of *H. pylori* (Jungblut, Bumann et al. 2000) and in preparation), whereas the protein species E53 could not yet be identified.

All these proteins may represent very immunogenic candidates for rapid diagnosis and monitoring of therapeutic success. For the design of a diagnostic assay that may react with a variety of *H. pylori* strains, we suggest to include a combination of at least two of the above mentioned highly specific and highly immunogenic antigens with low crossreactivity.

The proteins related to gastritis, ulcer and cancer may represent very immunogenic candidates for rapid diagnosis and monitoring of therapeutic success. For the design of a diagnostic assay for a variety of *H. pylori* strains, we suggest to include a combination of the above mentioned highly specific and highly immunogenic antigens with low crossreactivity (Table 14).

A combination of these antigens in such a diagnostic assay should include several, e.g. 20 or better 10 antigens, including at least 2 of the antigens from Table 14 selected according to multiple criteria (high occurence or high signal frequency, low crossreactivity). A pattern of at least 5, preferably at least 7 antigens would confer a positive result. In order to recognize false positive results, but also to take strain variation into account, at least three antigens in this assay may also be conserved antigens from Table 11.

For the diagnostic distinction between gastritis, ulcer or cancer, a combination of antigens from Table 15 and 16 (ulcer and cancer antigens) would be used also selected according to multiple criteria (occurence in patients with the related disease, high frequency, low crossreactivity). Several, e.g. at least 5 antigens from the tables could be used as a combination and the pattern of recognition would allow differential diagnosis of these diseases.

### 3.2 H. pylori antigens related to disease

The determination of bacterial factors that may influence the outcome of disease is still an important task (van Zanten and Lee, 1999). In our study, both appearance and intensity of antigen recognition was dramatically higher in association with *H. pylori* related ulcer and also with cancer compared to *H. pylori* related gastritis, although each serum revealed an individual immunoblot pattern. An additional statistical analysis that compared the serum antibody recognition from *H. pylori* negative gastritis patients to the aforementioned groups, showed that recognition seemed increase in association with *H. pylori* unrelated gastritis and *H. pylori* specific gastritis towards specific ulcer or cancer (data not shown). The major antigens associated with ulcer consisted of proteins also recognized with gastritis or negative sera: the 50 S ribosomal protein L7/L12 (HP1199), catalase (HP0875), GroEL (HP001 0) and Urease A (HP 0073). This may be due to persistence of strong inflammation and tissue damage leading to increased antigen presentation during the development of ulcer. In concordance, recognition of two hypothetical proteins HP 0305 and HP1285 that have not yet been described so far, was much stronger in association with ulcer. There are four other antigens that seem more specific for ulcer: Cag 16 (HP0537) the hypothetical protein HP 0305, a hemolysin secreting protein precursor and a signal recognition particle protein HP 1152, which may prove useful as serum markers for differentiation of *H. pylori* related ulcer from gastritis. It would be interesting to investigate whether recognition of these antigens may decrease after therapy. As only three patients with successful eradication therapy were tested, no significant data for a lower occurrence of antibodies against the above mentioned antigens could be obtained for these patients as an individual group (data not shown).

Antigens which are better recognized by gastritis sera than by ulcer sera, could be protective against the development of ulcer. Spot 121 from the putative neuraminyl-lactose-binding hemagglutinin homologue HP041 0 was not recognized in ulcer sera at all, but in Hp related gastritis sera and in negative sera. The hydantoin utilization protein A (HP0695), the NapA protein, which was also a protective antigen in animal studies (Satin, Del Giudice, et al. 2000), the conserved hypothetical protein HP0318, also described in another study, and a 26kDa antigen (HP1563) are three additional antigens that were more often recognized in *H. pylori* related gastritis sera than in ulcer sera.

As gastritis and ulcer only occasionally evolve toward cancer, a serum marker for cancer would be helpful to monitor endangered patients. In sera from cancer patients several metabolic enzymes from *H. pylori* such as aconitate hydratase (HP0779), aliphatic amidase (HP0294) and cinnamyl alcohol dehydrogenase (HP1104) seem to be preferentially recognized, compared to gastritis or ulcer. Other antigens related to cancer in this study as a conserved hypothetical secreted protein HP 1098, the outer membrane protein HP1564 or pyridoxal phosphat protein J (HP1582) that are also recognized by negative sera, may either indicate crossreactions with other bacterial strains and cellular antigens or reveal cancer specific antigens (Table 16). These need to be further investigated with a larger number of cancer patients.

### 3.3 Variation of antigen profiles

Taken together, the pattern of serum antibody recognition of *H. pylori* revealed by immunoproteomics is very diverse even among sera from the same patient group. This could be due to the individual immune response including HLA differences, but could also be related to genetic variability of *H. pylori* strains. The impact of antigenic variation on antibody recognition is difficult to address in this type of study which aims to detect differences in recognition pattern related to both infection and disease and thus keeps the tested strain constant. Therefore differences between the host strain and the tested strain may leave some proteins unrecognized. The protein pattern of 26695 and other *H. pylori* strains represented by J99 (Alm, Ling et al., 1999) and the mouse adapted strain SS1 show only few common protein spots (not shown). The enzyme thioredoxin for example, shows a shift in the position on the 2-DE pattern resulting from a different amino acid sequence of strain 26695 compared to J99 (Jungblut, Bumann et al., 2000). The strong reaction of two minor protein species B496 and B497 from the enzyme isocitrate dehydrogenase (HP0027) with the sera from *H. pylori* positive patients compared to negative sera, may be related to differences in the genomic sequence between *H. pylori* and crossreactive bacteria that are recognized by the negative sera or to posttranslational modifications of the proteins. In addition, recognition of a spot in close association to the spot series of isocitrate dehydrogenase from strain 26695 revealed two protein species B66 and B138. Further separation of the series of spots related to the isocitrate dehydrogenase is under investigation. Similar observations were made for recognition of protein species E44 of 50S ribosomal protein L7/L12 (HP1199) which reacts strongly with sera from ulcer patients, although it represents only a minor spot on silver stained patterns of *H. pylori* 26695 and on the immunoblots of sera from the other groups of patients. Such differences may also indicate specific strains or modified proteins present in the ulcer patients' autologous strains, In another study (McAtee, Fry et al., 1998), two species of this protein with different amino terminal sequences were detected by pooled patients' sera and did occur in parallel in the same *H. pylori* strain. A comparison of the complete sequences from these protein species with spot sequences in our database would be interesting. Interestingly, the NapA protein, which was a major antigen detected in another 2-DE analysis and also in a one dimensional study (Kimmel, Bosserhoff et al., 2000, Satin, Del Giudice et al., 2000) using a different *H. pylori* strain, was only poorly recognized by the sera tested here, indicating that the strain variation may indeed count for antigenic variation. However, the evolution of quasispecies in individual patients has only recently been investigated (Kuipers, Israel et al., 2000, Yamaoka and Graham, 1999). Several antigens identified in this study were also immunogenic in mice using a mouse adapted strain of *H. pylori.*

So far, mainly conserved or very abundant antigens of *H. pylor* including Urease A and B subunits, catalase, CagA, VacA, the GroES homologue HspA and NapA have been analyzed for their protective or therapeutic potential in animal studies, but none have been shown to be highly immunogenic so far in humans (Lee, Weltzin et al., 1995, Radcliff, Hazell et al., 1997, Gomez-Duarte, Lucas et al., 1998, Corthesy-Theulaz, Hopkins et al., 1998, Dubois, Lee et al., 1998, DiPetrillo, Tibbetts et al., 1999). As a combination of Urease and GroES augmented the protective capacity of either antigen in the mouse (Ferrero, Thiberge et al., 1995), a combination of the strong human B cell antigens detected in this study seems promising for testing in preclinical animal models.

In conclusion, immunoproteomics by 2-DE and MALDI MS revealed a variety of antigen patterns associated with *H. pylori* infection and different manifestations of disease. Nevertheless, the immunogenicity of known antigens was confirmed and new antigens, both non-specific and disease-related, could be identified. We have developed a database which compiles data from proteome analysis and genomic sequences providing a basis for classification and comparison of complex protein profiles. Data from other studies can be unequivocally compared by using the HP orf accession number or the sequence, thus facilitating the identification of more proteins. Further analyses are in progress to compare antigen recognition of different strains. Our database offers an unique opportunity to compile a large number of protein profiles in order to evaluate candidates for diagnostic assays and vaccine design. With such tools, immunoproteomics open a new gate to elucidate multiple immunorelevant proteins and may also be helpful in the context of other pathogens.

### Example 3 (for illustrative purposes only)

### 1. Materials and Methods

*H. pylori strain* 26695 (Eaton, Morgan and Krakowa, 1989) was cultured at 37°C in a micoraerobic atmosphere (5% O₂, 85% N₂, and 10% CO₂) on serum-agar plates (Odenbreit, Wieland and Haas, 1996) for 3 days and then grown for one additional day on fresh plates. The bacteria were harvested and suspended in ice-cold 40 mM MOPS, pH 7,4, 8 g/l NaCl, 10% Glycerol, 1 mM CaCl₂, 0,5 mM MgCl₂ at an optical density at 600 nm of 2,5-3,5 (equivalent to 1-2 x 10⁹ cfu/ml). The bacteria were surface-labeled by incubation with 200 µM (final concentration) sulfosuccinimidyl-6-(biotinamido)hexanoate (Pierce) for 30 minutes on ice. The reaction was stopped by adding two volumes of TSGCM (50 mM Tris; pH 7,4; 8 g/l NaCl; 10% Glycerol; 1 mM CaCl₂; 0,5 mM MgCl₂). After 10 minutes incubation at room temperature, the bacteria were sedimented by centrifugation at 3500 x g for 10 min and' washed three times with TSGCM.

The viability of the bacteria before and after labeling was determined by plating on serum-agar and by flow cytometry using a membrane-permeable (Syto 9) and a membrane-impermeable (Propidium iodide) fluorophores according to the instructions of the manufacturer (Live-Dead Kit, Molecular Probes) except that an altered ratio of 3 mM/27 mM of the dyes Syto9/Propidiumiodid was used.

To isolate biotinylated membrane proteins, labeled bacteria were resuspended in 50 mM Tris-HCl; pH 7,4; 1mM MgCl₂ with protease inhibitors (0,5 mM PMSF; 1 µM pepstatin; 1 µM leupeptin; 2,9 mM benzamidin) and disrupted by 4 passages through a French press at 15.000 Ib/in². After removal of intact bacteria by two centrifugations at 4.000 x g and 4°C for 10 minutes, membranes were pelleted at 40.000 x g and 4°C for 30 minutes, washed and resuspended in TKE (50 mM Tris-HCl, 150 mM KCI, 10 mM EDTA, protease inhibitors, pH 7,4). Membranes were adjusted to a protein concentration of 5 mg/ml, solubilized with 2% zwittergent 3-14 (Fluka) and incubated for 1 h at 4°C with head-over-head mixing. Insoluble membrane were removed by ultracentrifugation for 1 h at 100.000 x g and 4 °C. The soluble fraction was purified by affinity chromatography on reversibly binding avidin-agarose according to the instructions of the manufacturer (Boehringer) with slight modifications. In brief, 20-24 mg membrane proteins were diluted tenfold in 100 mM Na₂HPO₄, 150 mM NaCl, pH 7,2 and mixed with 1 ml avidin-agarose matrix equilibrated in washing buffer (100 mM Na₂HPO₄; 150 mM NaCl; pH 7,2; 0.2% zwittergent). After 30 minutes incubation at room temperature, the matrix was washed five times with 2 ml washing buffer. The biotinylated proteins were eluted by rising the avidin-agarose five times for 15 min at 37°C with washing buffer containing 20 mM D-biotin. Protein containing fractions were pooled and concentrated by acetone precipitation.

One dimensional SDS-PAGE and blotting on PVDF membranes were performed according to standard protocols. Biotinylated proteins were detected on the blots using NeutrAvidin-peroxidase staining (Pierce) and chemiluminescent visualization (ECL, Amersham) according to the manufacturers instructions. Two-dimensional gel electrophoresis, characterization by MALDI-MS and identification (assignment to a gene including the regulatory and coding sequences) were performed as described previously (Jungblut et al., 2000).

### 2. Results

To selectively label amino groups of *Helicobacter pylori* surface proteins that may play a role in host-pathogen interactions, we incubated intact *H. pylori* with the biotinylation reagent sulfosuccinimidyl-6-biotinamido-hexanoate that is highly hydrophilic due to its negatively charged sulfonate-group. The amount of biotinylation reagent was optimized to obtain selective labeling of many surface proteins at a minimum number of spot series (see below). As a control, we also biotinylated *H. pylori* lysates which should label all cell proteins with accessible amine groups.

Cell lysis could lead to a release of cytosolic components and thereby decrease selectivity. To test if such unwanted lysis occurred during labeling, the viability of the bacteria was determined before and after labeling by plating and flow cytometric analysis using a combination of'membrane-permeable and non-permeable fluorescent dyes ("live-dead-staining"). Plating indicated that less than 10% of the bacteria were killed during incubation and washing. Moreover, only 1-3% of the bacteria had a compromised membrane integrity after labeling as determined by flow cytometry. The small portion of killed bacteria might have released some cytosolic material but most of this would be removed during subsequent washing steps. In conclusion, cytosolic components are not likely to contaminate the labeled proteins.

To analyze the extent of biotinylation, unlabelled or labeled *H. pylori* samples were separated on two-dimensional polyacrylamid gels, blotted on PVDF membranes, incubated with an avidin-peroxidase conjugate and developed using a chemoluminescence assay.

Unlabelled samples contained only one weakly avidin-binding spot (apparent molecular weight about 20 kDa, pl > 9.0; data not shown).

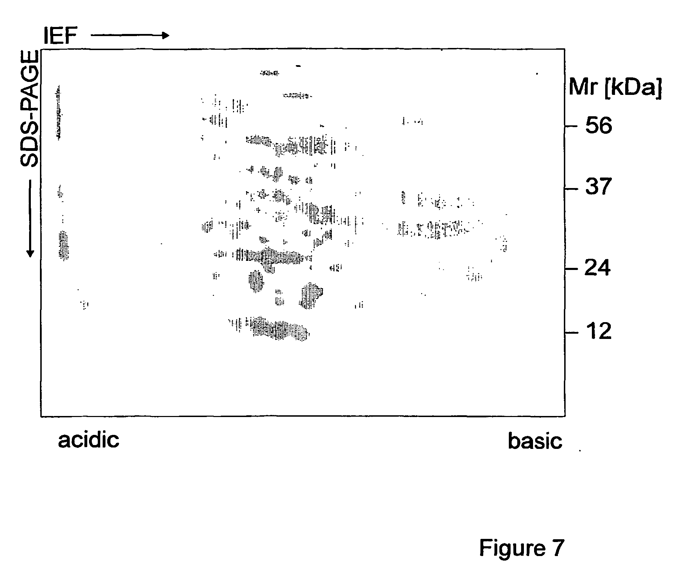

When *H. pylori* lysates were labeled, almost all proteins that could be detected by staining with Coomassie Blue were also biotinylated indicating that almost all proteins possess accessible amino groups (Fig. 7). Among the very few proteins that did not bind avidin were the previously identified citrate synthase and NapA (Jungblut, Bumann, Haas, Zimny-Arndt, Holland, Lamer, Siejak, Aebischer and Meyer, 2000). In addition, the highly abundant proteins Hsp60 and urease β subunit were only weakly labeled. This is surprising as Hsp60, NapA, and urease β subunit contain many lysine residues. Possibly, these lysines are not accessible for the biotinylation reagent.

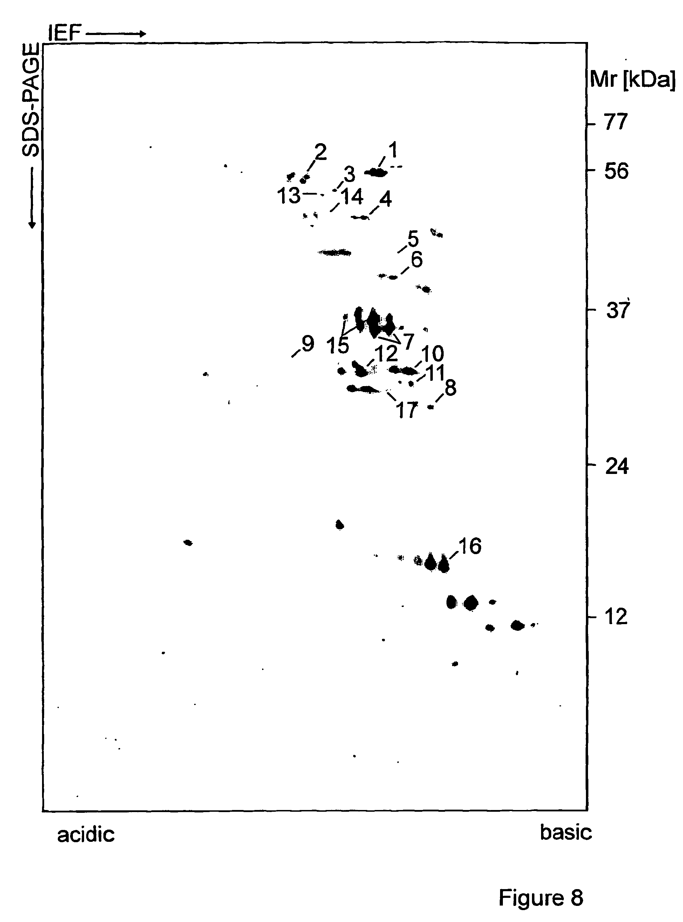

In contrast to the almost complete labeling of lysate proteins, labeled intact *H. pylori* contained only 44 species that were reproducibly biotinylated in three independent experiments (Fig. 7). Most species had isoelectric points in the alkaline range. Several species appeared as horizontal spot series instead of single spots. A comparison of labeled intact cells *vs*. labeled lysates (Fig. 7, 8) revealed a high selectivity of biotinylation as expected for a hydrophilic reagent and intact membranes. In contrast, the uncharged sulfhydryl-reactive reagent PEO-iodoacetyl biotin, labeled several known cytoplasmatic proteins indicating permeation of the bacterial membranes.

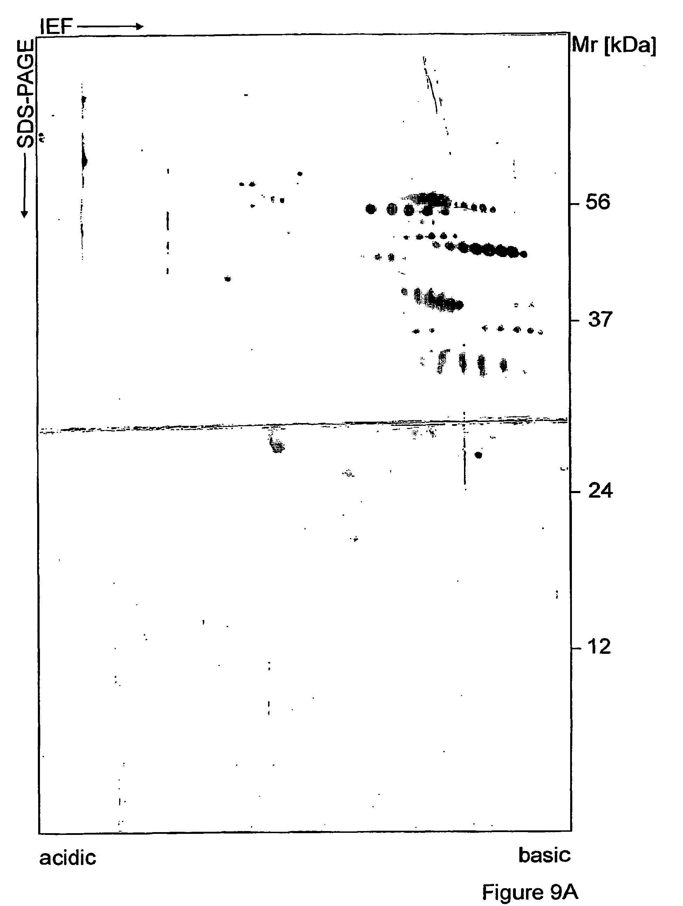

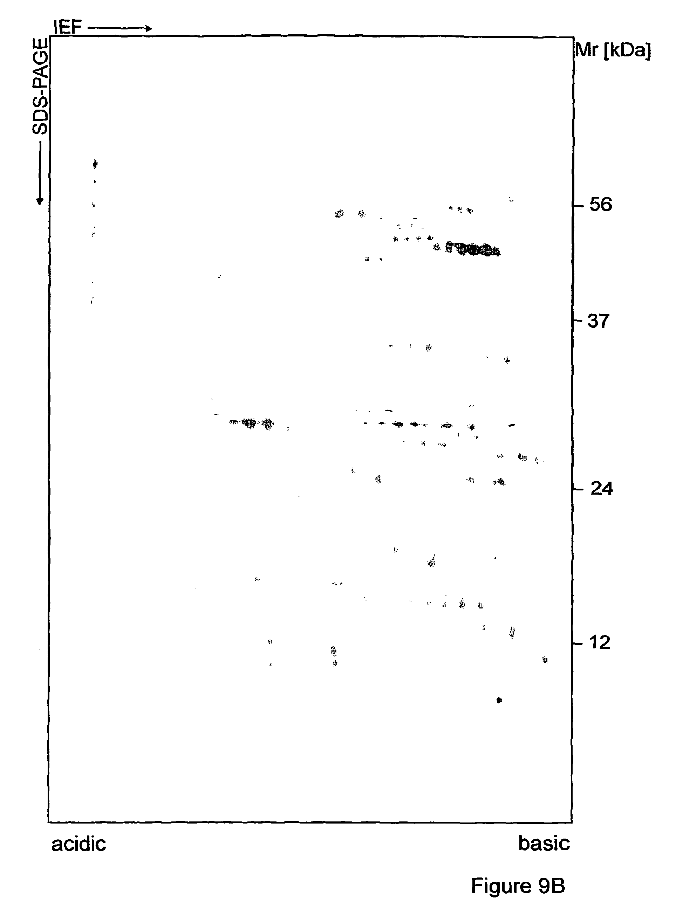

To enrich the biotinylated putative surface proteins, labeled bacteria were lysed and total membranes were isolated. After solubilization with zwittergent 3-14, membrane proteins were loaded on an reversibly biotin-binding avidin D-column, washed, and eluted with biotin. Two-dimensional electrophoresis of these purified labeled proteins revealed 58 species as detected by silverstaining (Fig. 9a) most of which (52 out of 58, 90%) were biotinylated (Fig. 9b). The remaining 6 species were minor contaminants as indicated by their weak silverstaining. Moreover, 29 additional signals appeared in the NeutrAvidin-peroxidase staining which could not be visualized by silver staining probably due to their low amount. Compared with whole biotinylated *H. pylori,* the overall avidin-binding patterns of purified proteins were quite similar but more complex due to longer spot series and more total detectable species (36 spots in addition). Possibly, these additional species escaped detection in non-enriched whole cell samples because of their low abundance. On the other hand, all biotinylated proteins except three of whole *H. pylori* could be also detected in the purified membrane fraction indicating that almost no soluble proteins had been labeled. The relative positions of 18 biotinylated species matched with previously identified *Helicobacter* proteins (Table 17). To confirm these tentative assignments, the 13 most abundant biotinylated species were cut out from the blots, digested, and identified by peptide mass fingerprinting. All of the direct identifications were consistent with the indirect assignments which confirmed the validity of the indirect approach.

Most unlabelled proteins of *H. pylori* appear as single spots in the two-dimensional pattern. In contrast, most labeled protein species appeared as horizontal spot series which could represent the same protein with slight modifications that alter the pl. This was confirmed for three members of a biotinylated spot series corresponding to catalase (data not shown). Probably, a variable number of amino groups reacted with the biotinylation reagent resulting in a differential loss of protonable residues and slightly decreasing pl's. The pl difference between consecutive members of spot series were rather constant and in the range of one protonable group per molecule (Δpl ≈ 0.05) which is consistent with this hypothesis. Using a 25 fold higher amount of biotinylation reagent, much more extensive spot series were obtained (data not shown). Moreover, acidic members of spot series were enriched during the avidin-affinity chromatography which further supports that they are highly biotinylated species.

### 3. Discussion

Surface proteins of *H. pylori* mediate important pathogen-host interactions that are essential for colonization, adherence, survival, and virulence of this pathogen. To identify *H. pylori* surface proteins, several approaches have been used (see introduction). In a global proteome approach, we combined a selective surface biotinylation of free amino groups with affinity purification, two-dimensional gel electrophoresis, and peptide mass finger printing.

A prerequisite for this method is the presence of free amino groups that are exposed to the external medium. The genome sequences indicate that all except 3 predicted proteins contain one or several lysine residues. Many of these residues seem to be on the outside of the corresponding protein structures since almost all detectable protein species in *H. pylori* lysates can be labeled using the highly hydrophilic biotinylation reagent sulfosuccinimidyl-6-(biotinamido)hexanoate that reacts with free amino groups. In contrast, in intact bacteria only a few protein species were labeled indicating that the hydrophobic membranes limited the accessibility of most proteins for the highly hydrophilic biotinylation reagent. This is confirmed by the fact that no putative cytoplasmic proteins were found among the selectively biotinylated proteins (see below).

While the inner membrane is impermeable for hydrophilic molecules unless they bind to specific carriers, the outer membrane contains porins that permit the diffusion of hydrophilic molecules. Usually rather small molecules permeate the outer membrane but exclusion limits up to 800 Dalton have been reported for some porins (Benz and Bauer, 1988). *H. pylori* might also posses porins with such a large exclusion limit and in this case, the biotinylation reagent (molecular weight: 560 Da) might gain access to the periplasmic space where it would label soluble and membrane associated periplasmic proteins as well as integral membrane proteins of the inner membrane all of which are not true surface proteins. In the case of *E. coli,* the same biotinylation reagent has been shown to weakly label periplasmatic proteins while labeling of true surface proteins was much more prominent. We attempted to further enhance selectivity by using five times lower concentrations of the labeling reagent. Under these conditions, almost all labeled protein species of intact *H. pylori* were recovered in total membrane preparations indicating that soluble periplasmic proteins had been scarcely labeled. Moreover, among the identified biotinylated proteins there was no putative inner membrane protein (see below) although many such proteins were labeled in lysates. Therefore, most proteins that were labeled in intact *H. pylori* are probably true surface proteins.

In total, 81 *H. pylori* protein species were found to be surface-exposed using selective labeling. It is likely, that *H. pylori* possesses some additional surface proteins that escaped labeling. Three prominent proteins (UreB, NapA, Hsp60) could not be labeled either in lysates or in intact bacteria indicating that their localization could not be assessed using this approach while their surface localization has previously been demonstrated (Dunn and Phadnis, 1998; Namavar et al., 1998). Additional surface proteins might contain only lysine residues that are exposed to the periplasmic space but not to the extracellular medium. Such proteins would be labeled in lysates but not in intact bacteria despite their surface localization. However, proteins with such an asymmetric lysine distribution are probably rather rare since almost all selectively labeled proteins contained many different accessible lysine residues on the external surface as indicated by their appearance as multiple horizontal spot series on two-dimensional gels after non-saturating labeling.

Among the group of labeled proteins that could be detected by avidin-staining of two-dimensional blots, 18 species matched with previously identified proteins (Tab. 17). However, the complex pattern of biotinylated proteins due to horizontal spot series and additional species that are below the detection limits of protein staining might impair such an indirect identification based on two-dimensional pattern comparisons. To directly confirm the tentative assignments, we separated the biotinylated proteins from unlabelled proteins using avidin-affinity chromatography of solubilized membrane proteins. After two-dimensional gel electrophoresis of the purified fraction, we could identify 12 labeled proteins by tryptic digestion and peptide mass fingerprinting using MALDI-MS and all 12 assignments were consistent with the previous indirect identifications (Tab. 17). We currently further improve the yield of the purification procedure to obtain enough material for the identification of additional biotinylated surface proteins.

Among the 18 identified surface-exposed proteins, urease A (Dunn and Phadnis, 1998), catalase (Phadnis et al., 1996) and flagellar sheath protein (HP0410) (Jones et al., 1997) have been previously reported to be on the surface of *H. pylori* while MsrA has been found in the extracellular medium (Cao et al., 1998). HefA (Bina et al., 2000) is a homolog of *E. coli* ToIC outer membrane protein which is known to be involved in multiple drug efflux. HP1564 is a predicted outer membrane protein and has a homolog in *Pasteurella haemolytica* that is a known outer membrane protein (Murphy and Whitworth, 1993). The homolog of cell binding factor 2 from *C. jejuni* has been purified by acid extraction suggesting a surface-association although it was not detected by an antiserum on intact bacteria (Kervella et al., 1993; Pei, Ellison, III, and Blaser, 1991). In summary, 7 out of 18 identified proteins or their homologs have been independently localized on the surface of *H. pylori* or other bacteria which confirms the validity of our approach.

For the other proteins, the localization is less clear from previous findings. The *E. coli* homologs of the protease HtrA and the iron ABC transporter CeuE are localized in the periplasma (Skorko-Glonek et al., 1997; Staudenmaier et al., 1989) and a homolog of carbonic anhydrase has been found in the periplasma of *Neisseria gonoroae* (Huang et al., 1998). The gamma-glutamyltranspeptidase of *E. coli* is a periplasmatic protein (Suzuki, Kumagai and Tochikura, 1986) but homologs in *Proteus mirabilis* (Nakayama, Kumagai and Tochikura, 1986) and *Actinobacillus actinomycetemcomitans* (Mineyama, Mikami and Saito, 1995) are localized on the surface. A homolog of the protease HP1350 is found in the periplasma of E. coli (Hara et al., 1991) and homologs in *C. jejuni* and *Bartonella bacilliformis* are secreted (Mitchell and Minnick, 1997; Parkhill et al., 2000). It is surprising that several of our biotinylated proteins from *H. pylori* have homologs in the periplasma of other organisms. It is unlikely that *H. pylori* proteins that copurify with membranes are soluble periplasmatic proteins but some of them could be periplasmatic membrane-associated proteins that stick to the membranes during purification. However, labeling of proteins in the periplasma is unlikely to occur under our conditions (see above). Moreover, the localization of the homologs from various organisms are controversial and several *H. pylori* proteins (including catalase, urease, Hsp60, Hsp70) are known to be localized on the surface despite different localization of their homologs in other organisms which casts doubts on predictions based on homologs of other organisms. In conclusion, it is likely that the identified proteins are truly surface-exposed in *H. pylori* although an independent localization method might be helpful to confirm this.

Interestingly, only two of the 18 identified proteins (HP0605, HP1564) have been theoretically predicted to be surface proteins and none of the "hypothetical outer membrane proteins" (HOP's) have yet been found. The strain HP 26695 is known not to express BabA and several members of the HOP family (Ilver, Arnqvist, Ogren, Frick, Kersulyte, Incecik, Berg, Covacci, Engstrand and Boren, 1998; Peck et al., 1998). HopC has been reported to be expressed in this strain (McAtee et al., 1998) and might be among the yet unidentified labeled protein species.

Several surface proteins of *H. pylori* mediate important host-pathogen interactions. This is also the case for some of the 18 proteins that were identified in this study. Two of them have been previously described as essential virulence factors (urease, *y-*glutamyltranspeptidase). Moreover, the flagellar sheath protein is part of functional flagella that are also essential for virulence. Cag 16 is a member of the CAG (cytotoxin-associated genes) pathogenicity island that is known to enhance inflammatory responses to *H. pylori* but no specific information on Cag16 is available. For other human pathogens, homologs of catalase and the protease HtrA are important for virulence and all fresh human isolates of *H. pylori* express catalase although this enzyme is not necessary for colonization in a mouse infection model. Information about a potential role of HtrA for *H. pylori* virulence is lacking. It would be interesting to functionally characterize HtrA and, particularly, the additional surface proteins with no known homologs in other organisms.

Surface proteins of *H. pylori* are especially exposed to the host immune system and therefore might represent major antigens. Indeed, 10 out of the 18 identified proteins are among the previously described 30 antigens that are recognized by the majority of sera from infected humans (McAtee, Lim, Fung, Velligan, Fry, Chow and Berg, 1998; Kimmel et al., 2000) (Haas et al., in prep.). This indicates that our identification procedure strongly enriched for highly immunogenic antigens (10 antigens out of 18 surface proteins *vs.* 30 antigens out of 1560 total proteins). This set of proteins therefore represents a rational basis to select antigen candidates for vaccine development. Indeed, three of the 18 identified proteins (catalase, γ-glutamyl peptidase, urease) have previously been shown to induce protective immunity against an *H. pylori* challenge infection in the mouse model. Additional candidate antigens are currently being tested.

In conclusion, a rapid proteome approach has been developed to identify surface proteins of *H. pylori* that are promising targets for the control of this important human pathogen. Moreover, this approach should be generally applicable to characterize the surface of several human pathogens to identify new potential target proteins for drug therapy and vaccine development.

### Example 4: Identification of secreted proteins obtained from H. pylori strain 26695

### 1. Bacterial culture

In order to analyse protein secretion of *H. pylori,* we need a liquid culture. In addition, the culture medium itself must be free of proteins. Thus, standard culture protocols using serum could not be used. We improved a method of serum free culture of *H. pylori* developed by Vanet and Labigne (1998). Briefly, the frozen stock of strain PA4 was plated on normal H. pylori serum agar plates. After 3 days, cells were suspended in BHI, washed, and the OD was determined. A liquid culture was inoculated by *H. pylori* suspension (final concentration 0.02 OD) in a 150 ml culture flask. The culture was shaken at 150 rpm at 37°C overnight, reaching an OD of 0.5-1. Cells were harvested, washed and used to inoculate 60 ml fresh medium in a 250 ml .flask to 0.01 OD. After 20 h cultivation, the culture reached an OD of 0.3-0.5 (about 4 x 10⁸ cfu/ml). Culture medium: BHI plus Vancomycin, Nystatin, Trimethoprim, plus 1 % beta-cyclodextrin. Before using the medium for cell culture, it was equilibrated in a *H. pylori* atmosphere overnight at 37°C.

### 2. TCA precipitation

Standard protocols for protein precipitation with 10% trichloro acetic acid (TCA) yielded only low protein amounts, due to a sticky sediment which could not be resuspended. Thus, we used the protocol of Komoriya, Shibano et al. (1995). After centrifugation at 18.500xg for 15 min the supernatant was treated with prechilled 25% TCA (final concentration 6%) and incubated on ice for 15 min. Proteins were sedimented by centrifugation (10.000 x g, 10 min), resuspended in 0.5 ml acetone using sonification, and again pelleted. The acetone washing was repeated twice. Finally the pellets were dried and resuspended in sample buffer.

### 3. Analysis by 2-DE gelelectrophoresis and MALDI-MS

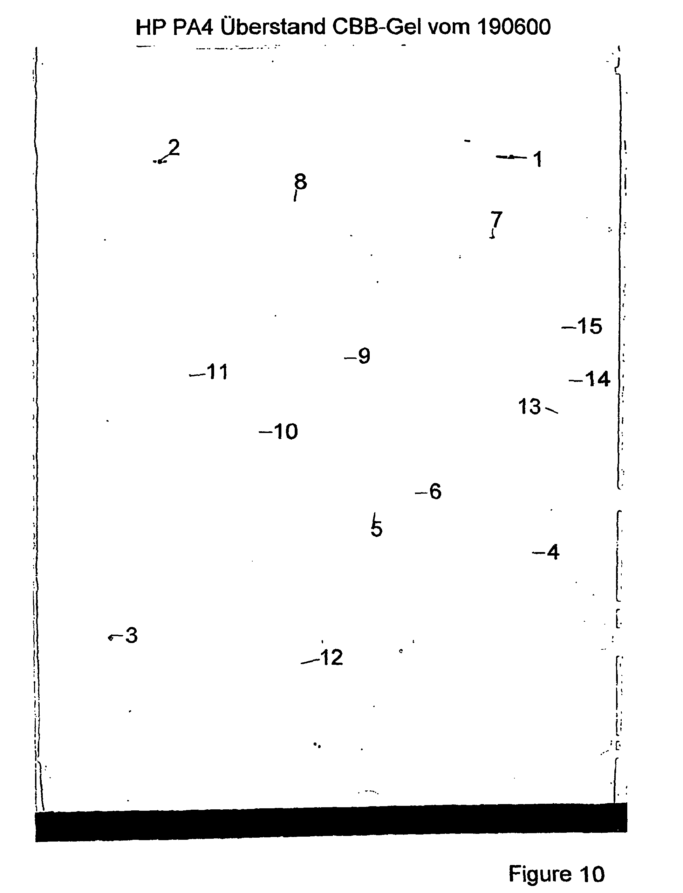

Two-dimensional gelelectrophoresis, characterization by MALDI-MS and identification (assignment to a gene including the regulatory and coding sequences) were performed as described previously (Jungblut et al., 2000). Proteome analysis of extracellular proteins reproducibly resulted in a pattern largely different from total cell lysates (see Figure 10). Thus, we conclude that spontaneous lysis of H. pylori, which might cause release of most of the *H. pylori* proteins, did occur only to a very small extent in our culture at the logarithmic growth phase (OD<0.5).

We digested 20 spots with trypsin and analysed the fragments by MALDI-MS (see Table 18). By comparing the intensities with corresponding spots obtained from total lysates, we conclude that urease B and fructose bisphosphat aldolase are most probably non-secreted proteins which might be released unspecificly to a small extent. All other spots were enriched in the extracellular medium. Thus, we conclude that they were secreted specificly.

Database search was performed with MS-FIT (NCBI database). We regarded a protein to be identified with a sequence coverage of at least 30%. We cannot exclude, however, the presence of additional proteins within the spots.

### Example 5: Vaccination with recombinant H. pylori surface proteins against H. pylori in the mouse model

The protein HP410 in cited for illustrative purposes only. In *Helicobacter pylori* strain P76, the ORFs HP 231 and HP410 were isolated from genomic DNA and amplified by PCR. A C-terminal hexa-histidin residue was fused to the ORFs by the primer sequence. The DNA fragments were cloned in the expression vector pet15b by *Nde1*/*BamH1* and the identities of the cloned PCR products were confirmed by sequencing. The proteins were expressed in *E. coli.* Purification was performed by affinity chromatography (Ni binding sepharose column) and subsequent dialysis.

Three groups of 10 mice each were orally immunized with 500 µg *H. pylori* P76 lysate, 100 µg recombinant *H. pylori* HP 231, or HP 410, respectively + 10 µg cholera toxin in each group. Immunization was performed at day 0, 18, 25 and 35. As a control, two groups of 5 mice each were immunized by pure PBS + 10 µg cholera toxin, or 100 µg p21 activated kinase 2 (PAK2), which does not occur in *H. pylori,* + 10 µg cholera toxin. At day 45, the mice were infected with 2,5 * 10⁸ *H. pylori* cells. At day 84, the stomach was removed. *H. pylori* colonization and the urease activity were determined. The IgG titer was determined by ELISA before and after immunization and after infection.

In the group immunized with *H. pylori* lysate, colonization was reduced to 9% of the PBS and the PAK2 control groups (P<0.005). Compared with the PBS and the PAK2 groups, the colonization in the HP 231 and HP 410 immunized groups was reduced to 6% (P<0.005) and 30% (P<0.05), indicating that immunization with HP 231 or HP 410 alone has a similar effect as lysate immunization. Urease activity was significantly reduced in the HP 231, HP 410 or *H. pylori* lysate groups compared with the PAK2 group. The titer of HP 231 specific IgG was increased in HP 231 immunized mice compared with naive mice and the control groups. In HP 410 immunized mice, the HP 410 specific IgG titer was not increased significantly.

Our results show that immunization of mice with the proteins HP 231 and HP 410 protect against *H. pylori* infection. Thus, HP 231 and HP 410 can be used for production of a vaccine or an immunogenic composition for the treatment of *Helicobacter* infections.

### References

1. Aebersold, R., and Leavitt, J. (1990) Sequence analysis of proteins separated by polyacrylamide gel electrophoresis: towards an integrated protein database. *Electrophoresis* **11**: 517-527.
2. Alm, R.A., Ling, L.S., Moir, D.T., King, B.L., Brown, E.D., Doig, P.C., Smith, D.R., Noonan, B., Guild, B.C., deJonge, B.L., Carmel, G., Tummino, P.J., Caruso, A., Uria-Nickelsen, M., Mills, D.M., Ives, C., Gibson, R., Merberg, D., Mills, S.D., Jiang, Q., Taylor, D.E., Vovis, G.F., and Trust, T.J. (1999) Genomic-sequence comparison of two unrelated isolates of the human gastric pathogen Helicobacter pylori. *Nature* **397:** 176-180.
3. Atherton, J.C., Peek, R.M.J., Tham, K.T., Cover, T.L., and Blaser, M.J. (1997) Clinical and pathological importance of heterogeneity in vacA, the vacuolating cytotoxin gene of Helicobacter pylori. *Gastroenterology* **112:** 92-99.
4. Aucher, P., Petit, M.L., Mannant, P.R., Pezennec, L., Babin, P., and Fauchere, J.L. (1998) Use of immunoblot assay to define serum antibody patterns associated with Helicobacter pylori infection and with H. pylori-related ulcers. *J Clin Microbiol* **36:** 931-936.
5. Bijlsma, J.J., Gerrits, M.M., Imamdi, R., Vandenbroucke-Grauls, C.M., and Kusters, J.G. (1998) Urease-positive, acid-sensitive mutants of Helicobacter pylori: urease-independent acid resistance involved in growth at low pH. *FEMS Microbiol Lett* **167**: 309-313.
6. Bijlsma, J.J., Vandenbroucke-Grauls, C.M., Phadnis, S.H., and Kusters, J.G. (1999) Identification of virulence genes of Helicobacter pylori by random insertion mutagenesis. *Infect Immun* **67:** 2433-2440.
7. Bizzozero, G. (1893) Über die schlauchförmigen Drüsen des Magendarmkanals und die Beziehung ihres Epithels zu dem Oberflächenepithel der Schleimhaut. *Arch Mikr Anat* **42:** 82-152.
8. Chen, M., Lee, A., and Hazell, S. (1992) immunisation against gastric helicobacter infection in a mouse/Helicobacter felis model. *Lancet* **339:** 1120-1121.
9. Clyne, M., Labigne, A., and Drumm, B. (1995) Helicobacter pylori requires an acidic environment to survive in the presence of urea. *Infect Immun* **63**: 1669-1673.
10. Corthesy-Theulaz, I.E., Bergonzelli, G.E., Henry, H., Bachmann, D., Schorderet, DF, Blum, A.L., and Ornston, L.N. (1997) Cloning and characterization of Helicobacter pylori succinyl CoA:acetoacetate CoA-transferase, a novel prokaryotic member of the CoA-transferase family. *J Biol Chem* **272**: 25659-25667.
11. Cover, T.L., and Blaser, M.J. (1992) Purification and characterization of the vacuolating toxin from Helicobacter pylori. *J Biol Chem* **267:** 10570-10575.
12. Crabtree, J.E. (1998) Eradication of chronic Helicobacter pylori infection by therapeutic vaccination. *Gut* **43**: 7-8.
13. Czinn, S.J., Cai, A., and Nedrud, J.G. (1993) Protection of germ-free mice from infection by Helicobacter felis after active oral or passive IgA immunization. *Vaccine* **11:** 637-642.
14. de Bernard, M., Papini, E., de Filippis, V., Gottardi, E., Telford, J., Manetti, Fontana, A., Rappuoli, R., and Montecucco, C. (1995) Low pH activates the vacuolating toxin of Helicobacter pylori, which becomes acid and pepsin resistant. *J Biol Chem* **270:** 23937-23940.
15. Doherty, N.S., Littman, B.H., Reilly, K., Swindell, A.C., Buss, J.M., and Anderson, N.L. (1998) Analysis of changes in acute-phase plasma proteins in an acute inflammatory response and in rheumatoid arthritis using two-dimensional gel electrophoresis. *Electrophoresis* **19**: 355-363.
16. Doig, P., Austin, J.W., and Trust, T.J. (1993) The Helicobacter pylori 19.6-kilodalton protein is an iron-containing protein resembling ferritin. *J Bacteriol* **175:** 557-560.
17. Dunkley, M.L., Harris, S.J., McCoy, R.J., Musicka, M.J., Eyers, F.M., Beagley, L.G., Lumley, P.J., Beagley, K.W., and Clancy, R.L. (1999) Protection against Helicobacter pylori infection by intestinal immunisation with a 50/52-kDa subunit protein. *FEMS Immunol Med Microbiol* **24:** 221-225.
18. Dunn, B.E., Perez-Perez, G.I., and Blaser, M.J. (1989) Two-dimensional gel electrophoresis and immunoblotting of Campylobacter pylori proteins. *Infect Immun* **57:** 1825-1833.
19. Eckerskorn, C., Jungblut, P., Mewes, W., Klose, J., and Lottspeich, F. (1988) Identification of mouse brain proteins after two-dimensional electrophoresis and electroblotting by microsequence analysis and amino acid composition analysis. *Electrophoresis* **9**: 830-838.
20. Evans, D.G., Karjalainen, T.K., Evans, D.J.J., Graham, D.Y., and Lee, C.H. (1993) Cloning, nucleotide sequence, and expression of a gene encoding an adhesin subunit protein of Helicobacter pylori. *J Bacteriol* **175:** 674-683.
21. Evans, D.J.J., Evans, D.G., Kirkpatrick, S.S., and Graham, D.Y. (1991) Characterization of the Helicobacter pylori urease and purification of its subunits. *Microb Pathog* **10**: 15-26.
22. Evans, D.J.J., Evans, D.G., Takemura, T., Nakano, H., Lampert, H.C., Graham, D.Y., Granger, D.N., and Kvietys, P.R. (1995) Characterization of a Helicobacter pylori neutrophil-activating protein. *Infect Immun* **63**: 2213-2220.
23. Faulde, M., Cremer, J., and Zoller, L. (1993) Humoral immune response against Helicobacter pylori as determined by immunoblot. *Electrophoresis* **14**: 945-951.
24. Faulde, M., Schroder, J.P., and Sobe, D. (1992) Serodiagnosis of Helicobacter pylori infections by detection of immunoglobulin G antibodies using an immunoblot technique and enzyme immunoassay. *Eur J Clin Microbiol Infect Dis* **11**: 589-594.
25. Fernandez-Patron, C., Calero, M., Collazo, P.R., Garcia, J.R., Madrazo, J., Musacchio, A., Soriano, F., Estrada, R., Frank, R., Castellanos-Serra, L.R., and et al (1995) Protein reverse staining: high-efficiency microanalysis of unmodified proteins detected on electrophoresis gels. *Anal Biochem* **224:** 203-211.
26. Forman, D., Newell, D.G., Fullerton, F., Yarnell, J.W., Stacey, A.R., Wald, N., and Sitas, F. (1991) Association between infection with Helicobacter pylori and risk of gastric cancer: evidence from a prospective investigation. *BMJ* **302:** 1302-1305.
27. Frazier, B.A., Pfeifer, J.D., Russell, D.G., Falk, P., Olsen, A.N., Hammar, M., Westblom, T.U., and Normark, S.J. (1993) Paracrystalline inclusions of a novel ferritin containing nonheme iron, produced by the human gastric pathogen Helicobacter pylori: evidence for a third class of ferritins. J *Bacteriol* **175:** 966-972.
28. Gerhard, M., Lehn, N., Neumayer, N., Boren, T., Rad, R., Schepp, W., Miehlke, S., Classen, M., and Prinz, C. (1999) Clinical relevance of the Helicobacter pylori gene for blood-group antigen-binding adhesin. *Proc Natl Acad Sci U S A* **96:** 12778-12783.
29. Gilbert, J.V., Ramakrishna, J., Sunderman, F.W.J., Wright, A., and Plaut, A.G. (1995) Protein Hpn: cloning and characterization of a histidine-rich metal-binding polypeptide in Helicobacter pylori and Helicobacter mustelae. *Infect Immun* 63: 2682-2688.
30. Goddard, A., and Logan, R. (1995) One-week low-dose triple therapy: new standards for Helicobacter pylori treatment. *Eur J Gastroenterol Hepatol* **7:** 1-3.
31. Gomez-Duarte, O.G., Bumann, D., and Meyer, T.F. (1999) The attenuated Salmonella vaccine approach for the control of Helicobacter pylori-related diseases. *Vaccine* **17**: 1667-1673.
32. Gomez-Duarte, O.G., Lucas, B., Yan, Z.X., Panthel, K., Haas, R., and Meyer, T.F. (1998) Protection of mice against gastric colonization by Helicobacter pylori by single oral dose immunization with attenuated Salmonella typhimurium producing urease subunits A and B. *Vaccine* **16**: 460-471.
33. Humphery-Smith, I., Cordwell, S.J., and Blackstock, W.P. (1997) Proteome research: complementarity and limitations with respect to the RNA and DNA worlds. *Electrophoresis* **18***:* 1217-1242.
34. IARC Working Group on the Evaluation of Carcinogenic Risks to Humans (1994) Schistosomes, liver flukes and Helicobacter pylori. *IARC Monogr Eval Carcinog Risks Hum* **61:** 1-241.
35. Ilver, D., Arnqvist, A., Ogren, J., Frick, I.M., Kersulyte, D., Incecik, E.T., Berg, D.E., Covacci, A., Engstrand, L., and Boren, T. (1998) Helicobacter pylori adhesin binding fucosylated histo-blood group antigens revealed by retagging. *Science* **279**: 373-377.
36. Jiang, Q., Hiratsuka, K., and Taylor, D.E. (1996) Variability of gene order in different Helicobacter pylori strains contributes to genome diversity. *Mol Microbiol* **20:** 833-842.
37. Jungblut, P., Eckerskorn, C., Lottspeich, F., and Klose, J. (1990) Blotting efficiency investigated by using two-dimensional electrophoresis, hydrophobic membranes and proteins from different sources. *Electrophoresis* **11**: 581-588.
38. Jungblut, P., Otto, A., Zeindl-Eberhart, E., Pleissner, K.P., Knecht, M., Regitz-Zagrosek, V., Fleck, E., and Wittmann-Liebold, B. (1994) Protein composition of the human heart: the construction of a myocardial two-dimensional electrophoresis database. *Electrophoresis* **15:** 685-707.
39. Jungblut, P.R., Grabher, G., and Stöffler, G. (1999a) Comprehensive detection of immunorelevant Borrelia garinii antigens by two-dimensional electrophoresis. *Electrophoresis* **20:** 3611-3622.
40. Jungblut, P.R., Schaible, U.E., Mollenkopf, H.J., Zimny-Arndt, U., Raupach, B., Mattow, J., Halada, P., Lamer, S., Hagens, K., and Kaufmann, S.H.E. (1999b) Comparative proteome analysis of Mycobacterium tuberculosis and Mycobacterium bovis BCG strains: towards functional genomics of microbial pathogens. *Mol Microbiol* **33:** 1103-1117.
41. Jungblut, P.R., and Seifert, R. (1990) Analysis by high-resolution two-dimensional electrophoresis of differentiation-dependent alterations in cytosolic protein pattern of HL-60 leukemic cells. *Journal of Biochemical and Biophysical Methods* **21**: 47-58.
42. Kimmel, B., Bosserhoff, A., Frank, R., Gross, R., Goebel, W., and Beier, D. (2000) Identification of Immunodominant Antigens from *Helicobacter pylori* and Evaluation of Their Reactivities with Sera from Patients with Different Gastroduodenal Pathologies. *Infect Immun* **68:** 915-920.
43. Klaamas, K., Held, M., Wadstrom, T., Lipping, A., and Kurtenkov, O. (1996) IgG immune response to Helicobacter pylori antigens in patients with gastric cancer as defined by ELISA and immunoblotting. *Int J Cancer* **67:** 1-5.
44. Kleanthous, H., Lee, C.K., and Monath, T.P. (1998) Vaccine development against infection with Helicobacter pylori. *Br Med Bull* **54:** 229-241.
45. Klose, J., and Kobalz, U. (1995) Two-dimensional electrophoresis of proteins: an updated protocol and implications for a functional analysis of the genome. *Electrophoresis* **16:** 1034-1059.
46. Labenz, J., and Borsch, G. (1995) Toward an optimal treatment of Helicobacter pylori-positive peptic ulcers. *Am J Gastroenterol* **90:** 692-694.
47. Lage, A.P., Godfroid, E., Fauconnier, A., Burette, A., Butzler, J.P., Bollen, A., and Glupczynski, Y. (1995) Diagnosis of Helicobacter pylori infection by PCR: comparison with other invasive techniques and detection of cagA gene in gastric biopsy specimens. *J Clin Microbiol* **33:** 2752-2756.
48. Lamarque, D., Gilbert, T., Roudot-Thoraval, F., Deforges, L., Chaumette, M.T., and Delchier, J.C. (1999) Seroprevalence of eight Helicobacter pylori antigens among 182 patients with peptic ulcer, MALT gastric lymphoma or non-ulcer dyspepsia. Higher rate of seroreactivity against CagA and 35-kDa antigens in patients with peptic ulcer originating from Europe and Africa. *Eur J Gastroenterol Hepato****l* 11:** 721-726.
49. Lee, A., O'Rourke, J., De Ungria, M.C., Robertson, B., Daskalopoulos, G., and Dixon, M.F. (1997) A standardized mouse model of Helicobacter pylori infection: introducing the Sydney strain. *Gastroenterology* **112:** 1386-1397.
50. Müller, E.-C., Thiede, B., Zimny-Arndt, U., Scheler, C., Prehm, J., Müller-Werdan, U., Wittmann-Liebold, B., Otto, A., and Jungblut, P. (1996) High-performance human myocardial two-dimensional electrophoresis database - edition 1996. *Electrophoresis* **17:** 1700-1712.
51. Marchetti, M., Arico, B., Burroni, D., Figura, N., Rappuoli, R., and Ghiara, P. (1995) Development of a mouse model of Helicobacter pylori infection that mimics human disease. *Science* **267:** 1655-1658.
52. Marchetti, M., Rossi, M., Giannelli, V., Giuliani, M.M., Pizza, M., Censini, S., Covacci, A., Massari, P., Pagliaccia, C., Manetti, R., Telford, J.L., Douce, G., Dougan, G., Rappuoli, R., and Ghiara, P. (1998) Protection against Helicobacter pylori infection in mice by intragastric vaccination with H. pylori antigens is achieved using a non-toxic mutant of E. coli heat-labile enterotoxin (LT) as adjuvant. *Vaccine* **16:** 33-37.
53. Marshall, B.J., and Warren, J.R. (1984) Unidentified curved bacilli in the stomach of patients with gastritis and peptic ulceration. *Lancet* **1**: 1311-1315.
54. Mattsson, A., Quiding-Jarbrink, M., Lonroth, H., Hamlet, A., Ahlstedt, I., and Svennerholm, A. (1998) Antibody-secreting cells in the stomachs of symptomatic and asymptomatic Helicobacter pylori-infected subjects. *Infect Immun* **66:** 2705-2712.
55. McAtee, C.P., Fry, K.E., and Berg, D.E. (1998a) Identification of potential diagnostic and vaccine candidates of Helicobacter pylori by "proteome" technologies. *Helicobacter* 3: 163-169.
56. McAtee, C.P., Lim, M.Y., Fung, K., Velligan, M., Fry, K., Chow, T., and Berg, D.E. (1998b) Identification of potential diagnostic and vaccine candidates of Helicobacter pylori by two-dimensional gel electrophoresis, sequence analysis, and serum profiling. *Clin Diagn Lab Immunol* 5: 537-542.
57. McAtee, C.P., Lim, M.Y., Fung, K., Velligan, M., Fry, K., Chow, T.P., and Berg, D.E. (1998c) Characterization of a Helicobacter pylori vaccine candidate by proteome techniques. *J Chromatogr B Biomed Appl* **714:** 325-333.
58. McGee, D.J.,and Mobley, H.L. (1999) Mechanisms of Helicobacter pylori infection: bacterial factors. *Curr Top Microbiol Immunol* **241:** 155-180.
59. Megraud, F. (1997) How should Helicobacter pylori infection be diagnosed? *Gastroenterology* **113:** S93-S98.
60. Michetti, P., Corthesy-Theulaz, I., Davin, C., Haas, R., Vaney, A.C., Heitz, M., Bille, J., Kraehenbuhl, J.P., Saraga, E., and Blum, A.L. (1994) Immunization of BALB/c mice against Helicobacter felis infection with Helicobacter pylori urease. *Gastroenterology* 107: 1002-1011.
61. Moayyedi, P., Sahay, P., Tompkins, D.S., and Axon, A.T. (1995) Efficacy and optimum dose of omeprazole in a new 1-week triple therapy regimen to eradicate Helicobacter pylori. *Eur J Gastroenterol Hepatol* **7:** 835-840.
62. Mollenkopf, H.J., Jungblut, P.R., Raupach, B., Mattow, J., Lamer, S., Zimny-Arndt, U., Schaible, U.E., and, Kaufmann, S.H. (1999) A dynamic two-dimensional polyacrylamide gel electrophoresis database: the mycobacterial proteome via Internet. *Electrophoresis* **20:** 2172-2180.
63. Nilsson, I., Ljungh, A., Aleljung, P., and Wadstrom, T. (1997) Immunoblot assay for serodiagnosis of Helicobacter pylori infections. J *Clin Microbiol* **35:** 427-432.
64. Nomura, A., Stemmermann, G.N., Chyou, P.H., Kato, I., Perez-Perez, G.I., and Blaser, M.J. (1991) Helicobacter pylori infection and gastric carcinoma among Japanese Americans in Hawaii. *N Engl J Med* **325:** 1132-1136.
65. Odenbreit, S., Till, M., Hofreuter, D., Faller, G., and Haas, R. (1999) Genetic and functional characterization of the alpAB gene locus essential for the adhesion of Helicobacter pylori to human gastric tissue. *Mol* **Microbiol 31:** 1537-1548.
66. Odenbreit, S., Wieland, B., and Haas, R. (1996) Cloning and genetic characterization of Helicobacter pylori catalase and construction of a catalase-deficient mutant strain. *J Bacteriol* **178:** 6960-6967.
67. Otto, A., Thiede, B., Müller, E.-C., Scheler, C., Wittmann-Liebold, B., and Jungblut P. (1996) Identification of human myocardial proteins separated by two-dimensional electrophoresis using an effective sample preparation for mass spectrometry. *Electrophoresis* **17:** 1643-1650.
68. Parsonnet, J., Friedman, G.D., Vandersteen, D.P., Chang, Y., Vogelman, J.H., Orentreich, N., and Sibley, R.K. (1991) Helicobacter pylori infection and the risk of gastric carcinoma. *N Engl J Med* **325:** 1127-1131.
69. Radcliff, F.J., Hazell, S.L., Kolesnikow, T., Doidge, C., and Lee, A. (1997) Catalase, a novel antigen for Helicobacter pylori vaccination. *Infect Immun* **65:** 4668-4674.
70. Riley, M. (1993) Functions of the gene products of Escherichia coli. *Microbiol Rev* **57:** 862-952.
71. Segal, E.D., Shon, J., and Tompkins, L.S. (1992) Characterization of Helicobacter pylori urease mutants. *Infect Immun* **60:** 1883-1889.
72. Shevchenko, A., Wilm, M., Vorm, O., and Mann, M. (1996) Mass spectrometric sequencing of proteins silver-stained polyacrylamide gels. *Anal Chem* **68:** 850-858.
73. Solnick, J.V., Josenhans, C., Suerbaum, S., Tompkins, L.S., and Labigne, A. (1995) Construction and characterization of an isogenic urease-negative mutant of Helicobacter mustelae. *Infect Immun* **63:** 3718-3721.
74. Telford, J.L., Ghiara, P., Dell'Orco, M., Comanducci, M., Burroni, D., Bugnoli, Tecce, M.F., Censini, S., Covacci, A., and Xiang, Z. (1994) Gene structure of the Helicobacter pylori cytotoxin and evidence of its key role in gastric disease. *J Exp Med* **179:** 1653-1658.
75. Tomb, J.F., White, O., Kerlavage, A.R., Clayton, R.A., Sutton, G.G., Fleischmann, RD, Ketchum, K.A., Klenk, H.P., Gill, S., Dougherty, B.A., Nelson, K., Quackenbush, J., Zhou, L., Kirkness, E.F., Peterson, S., Loftus, B., Richardson, Dodson, R., Khalak, H.G., Glodek, A., McKenney, K., Fitzegerald, L.M., Lee, N., Adams; M.D., and Venter, J.C. (1997) The complete genome sequence of the gastric pathogen Helicobacter pylori. *Nature* **388:** 539-547.
76. Warren, J.R. (1983) Unidentified curved bacilli on gastric epithelium in active chronic gastritis. *Lancet* **1:** 1273-1275.
77. Weel, J.F., van der Hulst, R.W., Gerrits, Y., Roorda, P., Feller, M., Dankert, J., Tytgat, G.N., and van der Ende, A. (1996) The interrelationship between cytotoxin-associated gene A, vacuolating cytotoxin, and Helicobacter pylori-related diseases. *J Infect Dis* **173:** 1171-1175.
78. Xiang, Z., Censini, S., Bayeli, P.F., Telford, J.L., Figura, N., Rappuoli, R., and Covacci, A. (1995) Analysis of expression of CagA and VacA virulence factors in 43 strains of Helicobacter pylori reveals that clinical isolates can be divided into two major types and that CagA is not necessary for expression of the vacuolating cytotoxin. *Infect Immun* **63:** 94-98.
79. Zevering, Y., Jacob, L., and Meyer, T.F. (1999) Naturally acquired human immune responses against Helicobacter pylori and implications for vaccine development. *Gut* **45:** 465-474.
80. Megraud,F. 1998. Epidemiology and mechanism of antibiotic resistance in helicobacter pylori. *Gastroenterology* **115:** 1278-1282.
81. IARC Working Group on the Evaluation of Carcinogenic Risks to Humans. 1994. Schistosomes, liver flukes and Helicobacter pylori. *IARC Monogr Eval Carcinog Risks Hum* **61:** 1-241.
82. Axon,A. and Forman,D. 1997. Helicobacter gastroduodenitis - a serious infectious disease - antibiotic treatment may prevent deaths in the decades ahead. *British Medical Journal* **314:** 1430-1431.
83. Kabir,S. 1999. The role of PCR in the diagnosis of Helicobacter pylori infections. *Reviews in Medical Microbiology* **10:** 197-212.
84. Jungblut,P.R., Bumann,D., Haas,G., Zimny-Arndt,U., Holland,P., Lamer,S., Siejak,F., Aebischer,A., and Meyer,T.F. 2000. Comparative proteome analysis of helicobacter pylori [In Process Citation]. *Mol Microbiol* **36:** 710-725.
85. Tomb,J.F., White,O., Kerlavage,A.R., Clayton,R.A., Sutton,G.G., Fleischmann, RD, Ketchum,K.A., Klenk,H.P., Gill,S. *et al.* 1997. The complete genome sequence of the gastric pathogen Helicobacter pylori. *Nature* **388:** 539-547.
86. Nilsson,T., Utt,M., Nilsson,H.O., Ljungh,A., and Wadstrom,T. 2000. Two-dimensional electrophoretic and immunoblot analysis of cell surface proteins of spiral-shaped and coccoid forms of Helicobacter pylori. *Electrophoresis* **21(13):** 2670-2677.
87. Enroth,H., Akerlund,T., Sillen,A., and Engstrand,L. 2000. Clustering of clinical strains of Helicobacter pylori analyzed by two-dimensional gel electrophoresis. Clinical & Diagnostic *Laboratory Immunology* **7(2):** 301-+.
88. O'Connor,C.D., Farris,M., Fowler,R., and Qi,S.Y. 1997. The proteome of Salmonella enterica serovar typhimurium: current progress on its determination and some applications. *Electrophoresis* **18:** 1483-1490.
89. Tonella,L., Walsh,B.J., Sanchez,J.C., Ou,K., Wilkins,M.R., Tyler,M., Frutiger,S., Gooley,A.A., Pescaru,I., Appel,R.D. *et al.* 1998. '98 Escherichia coli SWISS-2DPAGE database update. *Electrophoresis* **19:** 1960-1971.
90. Widmer,M., de Korwin,J.D., Aucher,P., Thiberge,J.M., Suerbaum,S., Labigne,A., and Fauchere,J.L. 1999. Performance of native and recombinant antigens for diagnosis of Helicobacter pylori infection. European Journal of Clinical *Microbiology & Infectious Diseases* **18(11):** 823-826.
91. Johansen,H.K., Norgaard,A., Andersen,L.P., Jensen,P., Nielsen,H., and Hoiby,N. 1995. Cross-reactive antigens shared by Pseudomonas aeruginosa, Helicobacter pylori, Campylobacter jejuni, and Haemophilus influenzae may cause false-positive titers of antibody to H. pylori. *Clin Diagn Lab Immunol* **2:** 149-155.
92. Nishizono,A., Gotoh,T., Fujioka,T., Murakami,K., Kubota,T., Nasu,M., Watanabe,M., and Mifune,K. 1998. Serological assessment of the early response to eradication therapy using an immunodominant outer membrane protein of Helicobacter pylori. *Clin Diagn Lab Immuno****l* 5:** 856-861.
93. van Zanten,S.J.O.V. and Lee,A. 1999. The role of Helicobacter pylori infection in duodenal and gastric ulcer [Review]. *GASTRODUODENAL DISEASE AND HELICOBACTER PYLORI: PATHOPHYSIOLOGY, DIAGNOSIS AND TREATMENT* **241:** 47-56.
94. Satin,B., Del Giudice,G., Della,B., V, Dusi,S., Laudanna,C., Tonello,F., Kelleher,D., Rappuoli,R., Montecucco,C., and Rossi,F. 2000. The neutrophil-activating protein (HP-NAP) of Helicobacter pylori is a protective antigen and a major virulence factor. *Journal of Experimental Medicine.* **191:** 1467-1476.
95. Kuipers,E.J., lsrael,D.A., Kusters,J.G., Gerrits,M.M., Weel,J., van Der,E., van Der,H., Wirth,H.P., Hook-Nikanne,J., Thompson,S.A. *et al.* 2000. Quasispecies development of Helicobacter pylori observed in paired isolates obtained years apart from the same host. *J Infect Dis* **181:** 273-282.
96. Yamaoka,Y. and Graham,D.Y. 1999. CagA status and gastric cancer unreliable serological tests produce unreliable data [letter; comment]. *Gastroenterology* **117:** 745.
97. Lee,C.K., Weltzin,R., Thomas,W.D.J., Kleanthous,H., Ermak,T.H., Soman,G., Hill,J.E., Ackerman,S.K., and Monath,T.P. 1995. Oral immunization with recombinant Helicobacter pylori urease induces secretory IgA antibodies' and protects mice from challenge with Helicobacter felis. J *Infect Dis* **172:** 161-172.
98. Corthesy-Theulaz,I.E., Hopkins,S., Bachmann,D., Saldinger,P.F.; Porta,N., Haas,R., Zheng-Xin,Y., Meyer,T., Bouzourene,H., Blum,A.L. *et al.* 1998. Mice are protected from Helicobacter pylori infection by nasal immunization with attenuated Salmonella typhimurium phoPc expressing urease A and B subunits. *Infect Immun* **66:** 581-586.
99. Dubois,A., Lee,C.K., Fiala,N., Kleanthous,H., Mehlman,P.T., and Monath,T. 1998. Immunization against natural Helicobacter pylori infection in nonhuman primates. *Infect Immun* **66:** 4340-4346.
100. DiPetrillo,M.D., Tibbetts,T., Kleanthous,H., Killeen,K.P., and Hohmann,E.L. 1999. Safety and immunogenicity of phoP/phoQ-deleted Salmonella typhi expressing Helicobacter pylori urease in adult volunteers. *Vaccine* **18:** 449-459.
101. Ferrero,R.L., Thiberge,J.M., Kansau,I., Wuscher,N., Huerre,M., and Labigne,A. 1995. The groes homolog of helicobacter pylori confers protective immunity against mucosal infection in mice. *Proceedings of the National Academy of Sciences of the United States of America* **92:** 6499-6503.
102. Alm, R.A., Bina,J., Andrews,B.M., Doig,P., Hancock,R.E., and Trust,T.J. (2000) Comparative genomics of Helicobacter pylori: analysis of the outer membrane protein families *Infect.Immun.* **68:** 4155-4168.
103. Benz,R., Bauer,K. (1988) Permeation of hydrophilic molecules through the outer membrane of gram-negative bacteria. Review on bacterial porins *Eur.J.Biochem.* 176: 1-19.
104. Bina,J.E., Alm,R.A., Uria-Nickelsen,M., Thomas,S.R., Trust,T.J., and Hancock,R.E. (2000) Helicobacter pylori uptake and efflux: basis for intrinsic susceptibility to antibiotics in vitro *Antimicrob.Agents Chemother.* 44: 248-254.
105. Bradburne,J.A., Godfrey,P., Choi,J.H., and Mathis,J.N. (1993) In vivo labeling of Escherichia coli cell envelope proteins with N-hydroxysuccinimide esters of biotin *Appl. Environ. Microbiol.* **59:** 663-668.
106. Cao,P., McClain,M.S., Forsyth,M.H., and Cover,T.L. (1998) Extracellular release of antigenic proteins by Helicobacter pylori *Infect.Immun.* **66:** 2984-2986.
107. Doig,P., Trust,T.J. (1994) Identification of surface-exposed outer membrane antigens of Helicobacter pylori *Infect.Immun.* 62: 4526-4533.
108. Dunn,B.E., Phadnis,S.H. (1998) Structure, function and localization of Helicobacter pylori urease *Yale J Biol.Med 71:* 63-73.
109. Eaton,K.A., Morgan,D.R., and Krakowka,S. (1989) Campylobacter pylori virulence factors in gnotobiotic piglets *Infect.Immun.* **57:** 1119-1125.
110. Exner,M.M., Doig,P., Trust,T.J., and Hancock,R.E. (1995) Isolation and characterization of a family of porin proteins from Helicobacter pylori *Infect. Immun.* **63:** 1567-1572.
111. Hara,H., Yamamoto,Y., Higashitani,A., Suzuki,H., and Nishimura,Y. (1991) Cloning, mapping, and characterization of the Escherichia coli prc gene, which is involved in C-terminal processing of penicillin-binding protein 3 *J.Bacteriol.* **173:** 4799-4813.
112. Huang,S., Xue,Y., Sauer-Eriksson,E., Chirica,L., Lindskog,S., and Jonsson,B.H. (1998) Crystal structure of carbonic anhydrase from Neisseria gonorrhoeae and its complex with the inhibitor acetazolamide *J.Mol.Biol.* **283:** 301-310.
113. Jones,A.C., Logan,R.P., Foynes,S., Cockayne,A., Wren,B.W., and Penn,C.W. (1997) A flagellar sheath protein of Helicobacter pylori is identical to HpaA, a putative N-acetylneuraminyllactose-binding hemagglutinin, but is not an adhesin for AGS cells *J. Bacteriol.* **179:** 5643-5647.
114. KerveIla,M., Pages,J.M., Pei,Z., Grollier,G., Blaser,M.J., and Fauchere,J.L. (1993) Isolation and characterization of two Campylobacter glycine-extracted proteins that bind to HeLa cell membranes *Infect.Immun.* **61:** 3440-3448.
115. Mineyama,R., Mikami,K., and Saito,K. (1995) Partial purification and some properties of gamma-glutamyl peptide-hydrolysing enzyme from Actinobacillus actinomycetemcomitans *Microbios* **82:** 7-19.
116. Mitchell,S.J., Minnick,M.F. (1997) A carboxy-terminal processing protease gene is located immediately upstream of the invasion-associated locus from Bartonella bacilliformis *Microbiology* **143:** 1221-1233.
117. Murphy,G.L., Whitworth,L.C. (1993) Analysis of tandem, multiple genes encoding 30-kDa membrane proteins in Pasteurella haemolytica A1 *Gene* **129:** 107-111.
118. Nakayama,R., Kumagai,H., and Tochikura,T. (1984) gamma-Glutamyltranspeptidase from Proteus mirabilis: localization and activation by phospholipids *J.Bacteriol.* **160:** 1031-1036.
119. Namavar,F., Sparrius,M., Veerman,E.C., Appelmelk,B.J., and Vandenbroucke-Grauls,C.M. (1998) Neutrophil-activating protein mediates adhesion of Helicobacter pylori to sulfated carbohydrates on high-molecular-weight salivary mucin *Infect. Immun.* **66:** 444-447.
120. Nilsson,C.L., Larsson,T., Gustafsson,E., Karlsson,K.A., and Davidsson,P. (2000) Identification of protein vaccine candidates from Helicobacter pylori using a preparative two-dimensional electrophoretic procedure and mass spectrometry *Anal. Chem.* **72:** 2148-2153.
**121.** Parkhill,J., Wren,B.W., Mungall,K., Ketley,J.M., Churcher,C., Basham,D., Chillingworth,T., Davies,R.M., Feltwell,T., Holroyd,S., Jagels,K., Karlyshev,A.V., Moule,S., Pallen,M.J., Penn,C.W., Quail,M.A., Rajandream,M.A., Rutherford,K.M., van Vliet,A.H., Whitehead,S., and Barrell,B.G. (2000) The genome sequence of the food-borne pathogen Campylobacter jejuni reveals hypervariable sequences *Nature* **403:** 665-668.
122. Peck,B., Ortkamp,M., Diehl,K.D., Hundt,E., and Knapp,B. (1999) Conservation, localization and expression of HopZ, a protein involved in adhesion of Helicobacter pylori *Nucleic Acids Res.* **27:** 3325-3333.
123. Pei,Z.H., Ellison,R.T., III, and Blaser,M.J. (1991) Identification, purification, and characterization of major antigenic proteins of Campylobacter jejuni *J.Biol.Chem.* **266:** 16363-16369.
124. Phadnis,S.H., Parlow,M.H., Levy,M., liver,D., Caulkins,C.M., Connors,J.B., and Dunn,B.E. (1996) Surface localization of Helicobacter pylori urease and a heat shock protein homolog requires bacterial autolysis *Infect.Immun.* **64:** 905-912.
125. Skorko-Glonek,J., Lipinska,B., Krzewski,K., Zolese,G., Bertoli,E., and Tanfani,F. (1997) HtrA heat shock protease interacts with phospholipid membranes and undergoes conformational changes *J.Biol.Chem.* **272:** 8974-8982.
126. Staudenmaier,H., Van Hove,B., Yaraghi,Z., and Braun,V. (1989) Nucleotide sequences of the fecBCDE genes and locations of the proteins suggest a periplasmic-binding-protein-dependent transport mechanism for iron (III) dicitrate in Escherichia coli *J.Bacteriol.* **171:** 2626-2633.
127. Suzuki,H., Kumagai,H., and Tochikura,T. (1986) gamma-Glutamyltranspeptidase from Escherichia coli K-12: formation and localization *J.Bacteriol.* **168:** 1332-1335.
128. Zevering,Y., Jacob,L., and Meyer,T.F. (1999) Naturally acquired human immune responses against Helicobacter pylori and implications for vaccine development *Gut* **45:** 465-474.

**Table 1: Systematic assignment of the proteins identified from H. pylori 26695**

| Proteins of *Helicobacter pylori* 26695 were separated by 2-DE. The protein spots were identified by PMF using MALDI-mass spectrometry. The proteins were grouped according to the protein classification described in Tomb *et. al.* (Nature 388:539-547, 1997), which is deduced from the *Echerichia coli* gene classification of Riley (Microbiol. Rev. 57, 862-952, 1993). The number in brackets after each category refer to the total number of genes in this category. The classification, protein names and numbers were taken from TIGR database (Nov 24^{th} 1999) (http://www.tigr.org/tdb/). | | | | |
|---|---|---|---|---|
| All proteins are listed forillustrative purposes only | | | | |
| Spot No | NCBI | Protein name TIGR | Short name | ORF |
| | AccNo | | | |
| **A Amino acid biosynthesis (44)** | | | | |
| **A 1. Aromatic amino acid family (14)** | | | | |
| **A 2. Aspartate family (14)** | | | | |
| B185 | 2313754 | Tetrahydrodipicolinate N-succinyltransferase | DapD | HP0626 |
| **A 3. Glutamate family (3)** | | | | |
| A431 | 2494748 | Glutamine synthetase | GlnA | HP0512 |
| **A 4. Pyruvate family (3)** | | | | |
| B192 | 3024012 | Branched-chain-amino-acid aminotransferase | IIvE | HP1468 |
| **A 5. Serine family (9)** | | | | |
| D3 - | 2313177 | Phosphoglycerate dehydrogenase | - | HP0096 |
| **A 6. Other (1)** | | | | |
| B377 | 2313818 | Hydantoin utilization protein A | HyuA | HP0695 |
| **B Purines, pyrimidines, nucleosides, and nucleotides (38)** | | | | |
| **B 1. 2'-Deoxyribonucleotide metabolism (5)** | | | | |
| C186 | 3024765 | Thioredoxin reductase | TrxB | HP0825 |
| **B 2. Purine ribonucleotide biosynthesis (12)** | | | | |
| F50 | 2498068 | Nucleoside diphosphate kinase | Ndk | HP0198 |
| C89 | 2497478 | Adenylate kinase | Adk | HP0618 |
| B488 | 2497358 | Inosine-5'-monophosphate dehydrogenase | GuaB | HP0829 |
| **B 3. Pyrimidine ribonucleotide biosynthesis (11)** | | | | |
| **B 4. Salvage of nucleosides and nucleotides (5)** | | | | |
| B403 | 2313187 | 2',3'-cyclic-nucleotide 2'-phosphodiesterase | CpdB | HP0104 |
| **B 5. Sugar-nucleotide biosynthesis and conversions (4)** | | | | |
| **B 6. Other (1)** | | | | |
| **C Fatty acid and phospholipid metabolism (26)** | | | | |
| **C 1. Biosynthesis (24)** | | | | |
| D69 | 2313282 | Enoyl-(acyl-carrier-protein) reductase (NADH) | FabI | HP0195 |
| D295 | 2313678 | 3-ketoacyl-acyl carrier protein reductase | FabG | HP0561 |
| B224 | 2313814 | Acetyl coenzyme A acetyltransferase (thiolase) | FadA | HP0690 |
| D96 | 2314546 | 3R)-hydroxymyristoyl-(acyl carrier protein) dehydratase | FabZ | HP1376 |
| **C 2. Degradation (2)** | | | | |
| **D Biosynthesis of cofactors, prosthetic groups, and carriers (62)** | | | | |
| **D 1. Biotin (7)** | | | | |
| **D 2. Folic acid (7)** | | | | |
| **D 3. Heme and porphyrin, and cobalamin (10)** | | | | |
| **D 4. Menaquinone and ubiquinone (3)** | | | | |
| **D 5. Molybdopterin (12)** | | | | |
| **D 6. Pantothenate (4)** | | | | |
| **D 7. Pyridoxine (2)** | | | | |
| D314 | 2314765 | Pyridoxal phosphate biosynthetic protein J | PdxJ | HP1582 |
| **D 8. Riboflavin, FMN. and FAD (6)** | | | | |
| **D 9. Glutathione (1)** | | | | |
| B321 | 2314270 | Gamma-glutamyltranspeptidase | Ggt | HP1118 |
| **D 10. Thiamine (4)** | | | | |
| **D 11. Pyridine nucleotides (3)** | | | | |
| **D 12. Other (3)** | | | | |
| **E Central intermediary metabolism (34)** | | | | |
| **E 1. Amino sugars (1)** | | | | |
| **E 2. Phosphorus compounds (3)** | | | | |
| C195 | 2500043 | Inorganic pyrophosphatase | Ppa | HP0620 |
| **E 3. Polyamine biosynthesis (3)** | | | | |
| **E 4. Other (27)** | | | | |
| C55 | 2507528 | Urease accessory protein | UreG | HP0068 |
| D84 | 2507527 | Urease accessory protein | UreF | HP0069 |
| D215 | 2507525 | Urease accessory protein | UreE | HP0070 |
| A343 | 137076 | Urease beta subunit (urea amidohydrolase) | UreB | HP0072 |
| A325 | 137076 | Urease beta subunit (urea amidohydrolase) | UreB | HP0072 |
| A323 | 137076 | Urease beta subunit (urea amidohydrolase) | UreB | HP0072 |
| D322 | 137069 | Urease, alpha subunit | UreA | HP0073 |
| D318 | 137069 | Urease, alpha subunit | UreA | HP0073 |
| D316 | 137069 | Urease, alpha subunit | UreA | HP0073 |
| D323 | 137069 | Urease, alpha subunit | UreA | HP0073 |
| D326 | 130769 | Urease, alpha subunit | UreA | HP0073 |
| C109 | 2314035 | Hydrogenase expression/formation protein | HypB | HP0900 |
| D200 | 2314346 | Carbonic anhydrase | - | HP1186 |
| **F Energy metabolism (108)** | | | | |
| **F 1. Aerobic (15)** | | | | |
| **F 2. Amino acids and amines (8)** | | | | |
| B511 | 2313392 | Aliphatic amidase | AimE | HP0294 |
| **F 3. Anaerobic (11)** | | | | |
| B17 | 2494617 | Fumarate reductase, flavoprotein subunit | FrdA | HP0192 |
| B516 | 2313707 | Ferredoxin oxidoreductase, alpha subunit | - | HP0589 |
| D287 | 2314259 | Pyruvate ferredoxin oxidoreductase, gamma subunit | - | HP1108 |
| D188 | 2314262 | Pyruvate ferredoxin oxidoreductase, beta subunit | - | HP1111 |
| | 2565251 | | | |
| **F 4. ATP-proton motive force interconversion (9)** | | | | |
| A209 | 2197129 | ATP synthase F1, subunit beta | AtpD | HP1132 |
| B465 | 2493030 | ATP synthase F1, subunit gamma | AtpG | HP1133 |
| **F 5. Electron transport (29)** | | | | |
| E41 | 3024719 | Thioredoxin | TrxA | HP0824 |
| E59 | 3024719 | Thioredoxin | TrxA | HP0824 |
| E29 | 3024719 | Thioredoxin | TrxA | HP0824 |
| D230 | 2314091 | Oxygen-insensitive NAD(P)H nitroreductase | - | HP0954 |
| E62 | 2314319 | Flavodoxin | FldA | HP1161 |
| E60 | 2314319 | Flavodoxin | FldA | HP1161 |
| B480 | 2314321 | Thioredoxin reductase | TrxB | HP1164 |
| F34 | 2314636 | Thioredoxin | - | HP1458 |
| D236 | 2314722 | Ubiquinol cytochrome c oxidoreductase, Rieske 2Fe-2S subunit | FbcF | HP1540 |
| **F 6. Entner-Doudoroff (2)** | | | | |
| **F 7. Fermentation (6)** | | | | |
| C155 | 2492992 | 3-oxoadipate coA-transferase subunit A | YxjD | HP0691 |
| C110 | 2492996 | 3-oxoadipate coA-transferase subunit B | YxjE | HP0692 |
| **F 8. Glycolysis/gluconeogenesis (14)** | | | | |
| A487 | 2506387 | Enolase | Eno | HP0154 |
| D7 | 2492813 | Fructose-bisphosphate aldolase | Tsr | HP0176 |
| **F 9. Pentose phosphate pathway (5)** | | | | |
| **F 10. Sugars (2)** | | | | |
| B173 | 2313462 | UDP-glucose 4-epimerase | - | HP0360 |
| **F 11. TCA cycle (5)** | | | | |
| B483 | 2493711 | Citrate synthase | GltA | HP0026 |
| B492 | 2497255 | Isocitrate dehydrogenase | lcd | HP0027 |
| B499 | 2497255 | Isocitrate dehydrogenase | lcd | HP0027 |
| B210 | 2497255 | Isocitrate dehydrogenase | lcd | HP0027 |
| B2 | 3023247 | Aconitase B | AcnB | HP0779 |
| B505 | 2314492 | Fumarase | FumC | HP1325 |
| **F 12. Other (2)** | | | | |
| **G Transport and binding proteins (119)** | | | | |
| **G 1. Amino acids, peptides and amines (29)** | | | | |
| D174 | 2313399 | Dipeptide ABC transporter, ATP- | DppD | HP0301 |
| | | binding protein | | |
| **G 2. Anions (5)** | | | | |
| **G 3. Carbohydrates, organic alcohols, and acids (6)** | | | | |
| **G 4. Cations (24)** | | | | |
| D221 | 2314745 | Iron(III) ABC transporter, | CeuE | HP1561 |
| | | periplasmic iron-binding protein | | |
| D313 | 2314746 | Iron(III) ABC transporter, | CeuE | HP1562 |
| | | periplasmic iron-binding protein | | |
| **G 5. Nucleosides, purines and pyrimidines (2)** | | | | |
| **G 6. Other (15)** | | | | |
| **G 7. Unknown substrate (38)** | | | | |
| **H DNA metabolism (105)** | | | | |
| **H 1. DNA replication, recombination, and repair (54)** | | | | |
| **H 2. Restriction/modification (48)** | | | | |
| **H 3. Degradation of DNA (2)** | | | | |
| **H 4. Chromosome-associated proteins (1)** | | | | |
| D180 | 2314294. | Plasmid replication-partition related protein | - | HP1138 |
| **I Transcription (10)** | | | | |
| **I 1. Degradation of RNA (1)** | | | | |
| **I 2. DNA-dependent RNA polymerase (2)** | | | | |
| A461 | 2500600 | DNA-directed RNA polymerase, alpha subunit | RpoA | HP1293 |
| **I 3. Transcription factors (4)** | | | | |
| C69 | **2494920** | Transcription elongation factor GreA | GreA | HP0866 |
| D93 | 2499340 | Transcription termination factor NusG | NusG | HP1203 |
| **I 4. RNA processing (3)** | | | | |
| **J Protein synthesis (99)** | | | | |
| **J 1. tRNA aminoacylation (26)** | | | | |
| **J 2. Nucleoproteins (1)** | | | | |
| **J 3. Ribosomal proteins: synthesis and modification (54)** | | | | |
| B57 | 2500384 | Ribosomal protein S1 | Rps1 | HP0399 |
| F68 | 2500245 | Ribosomal protein L9 | Rpl9 | HP0514 |
| E35 | 2500212 | Ribosomal protein L7/L12 | RpI7/I12 | HP1199 |
| E27 | 2500212 | Ribosomal protein L7/L12 | RpI7/I12 | HP1199 |
| D329 | 2500400 | Ribosomal protein S4 | Rps4 | HP1294 |
| F55 | 2500432 | Ribosomal protein S10 | Rps10 | HP1320 |
| B147 | 2500388 | Ribosomal protein S2 | Rps2 | HP1554 |
| **J 4. tRNA and rRNA base modification (5)** | | | | |
| **J 5. Translation factors (11)** | | | | |
| C115 | 2494272 | Translation elongation factor EF-P | Efp | HP0177 |
| A271 | 2494251 | Translation elongation factor EF-G | FusA | HP1195 |
| A477 | 2494256 | Translation elongation factor EF-Tu | TufB | HP1205 |
| A478 | 2494256 | Translation elongation factor EF-Tu | TufB | HP1205 |
| A476 | 2494256 | Translation elongation factor EF-Tu | TufB | HP1205 |
| D86 | 3024576 | Ribosome releasing factor | Frr | HP1256 |
| B119 | 2494278 | Translation elongation factor EF-Ts | Tsf | HP1555 |
| A501 | 2494278 | Translation elongation factor EF-Ts | Tsf | HP1555 |
| **J 6. Other (2)** | | | | |
| F65 | 2313961 | ss-DNA binding protein 12RNP2 precursor | - | HPO827 |
| **K Protein fate (44)** | | | | |
| **K 1. Protein modification and repair (3)** | | | | |
| **K 2. Protein folding and stabilization (9)** | | | | |
| A390 | 2506272 | Chaperone and heat shock protein | GroEL | HP0010 |
| A194 | 2506272 | Chaperone and heat shock protein | GroEL | HP0010 |
| F16 | 2506278 | Co-chaperone | GroES | HP0011 |
| F6 | 2506278 | Co-chaperone | GroES | HP0011 |
| F9 | 2506278 | Co-chaperone | GroES | HP0011 |
| A359 | 2495351 | Chaperone and heat shock protein 70 | DnaK | HP0109 |
| A360 | 2495351 | Chaperone and heat shock protein 70 | DnaK | HP0109 |
| A331 | 2495363 | Chaperone and heat shock protein C62.5 | HtpG | HP0210 |
| D167 | 2314166 | Co-chaperone-curved DNA binding protein A | CbpA | HP1024 |
| F36 | 2314613 | Peptidyl-prolyl cis-trans isomerase B, cyclosporin-type | Ppi | HP1441 |
| | | rotamase | | |
| **K 3. Protein and peptide secretion and trafficking (13)** | | | | |
| B320 | 2500881 | Signal recognition particle protein | Ffh | HP1152 |
| **K 4. Degradation of proteins, peptides, and glycopeptides (19)** | | | | |
| F40 | 2495234 | Protein kinase C inhibitor (SP:P16436) | - | HP0404 |
| B537 | 3182910 | Aminopeptidase a/i | PepA | HP0570 |
| 8455 | 2313782 | Processing protease | YmxG | HP0657 |
| C84 | 2493736 | ATP-dependent clp protease proteolytic component | ClpP | HPO794 |
| B210 | 2314155 | Protease | PqqE | HP1012 |
| 8429 | 2314163 | Serine protease | HtrA | HP1019 |
| B427 | 2314163 | Serine protease | HtrA | HP1019 |
| B303 | 2314520 | Protease | - | HP1350 |
| **L Regulatory functions (37)** | | | | |
| **L 1. Other (37)** | | | | |
| B503 | 2313314 | Peptide methionine | MsrA | HP0224 |
| D265 | 2313506 | Penicillin tolerance protein | LytB | HP0400 |
| D257 | 3913690 | Ferric uptake regulation protein | Fur | HP1027 |
| **M Cell envelope (105)** | | | | |
| **M 1. Lipoproteins (3)** | | | | |
| **M 2. Surface structures (1)** | | | | |
| D132 | 2313516 | Putative neuraminyllactose-binding hemagglutinin homolog | HpaA | HP041 0 |
| D121 | 2313516 | Putative neuraminyllactose-binding hemagglutinin homolog | HpaA | HP0410 |
| D345 | 2313516 | Putative neuraminyllactose-binding hemagglutinin homolog | HpaA | HP0410 |
| **M 3. Biosynthesis of murein sacculus and peptidoglycan (20)** | | | | |
| **M 4. Biosynthesis and degradation of surface polysaccharides and lipopolysaccharides (34)** | | | | |
| B297 | 2313283 | UDP-3-0-(3-hydroxymyristoyl) glucosamine N-acyltransferase | LpxD | HP0196 |
| 8246 | 2313467 | Spore coat polysaccharide biosynthesis protein C | - | HP0366 |
| **M 5. Other (47)** | | | | |
| D249 | 2499779 | Cell binding factor 2 | - | HP0175 |
| D202 | 2314748 | Outer membrane protein | - | HP1564 |
| D326 | 2314748 | Outer membrane protein | - | HP1564 |
| **N Cellular processes (161)** | | | | |
| **N 1. Cell division (21)** | | | | |
| **N 2. Chemotaxis and motility (73)** | | | | |
| **N 3. Detoxification (6)** | | | | |
| E54 | 2506370 | Neutrophil activating protein (bacterioferriti) | NapA | HP0243 |
| C134 | 2313490 | Superoxide dismutase | SodB | HP0389 |
| C197 | 2313490 | Superoxide dismutase | SodB | HP0389 |
| B439 | 1561776 | Catalase | - | HP0875 |
| B437 | 2493545 | Catalase | - | HP0875 |
| D341 | 2507172 | Alkyl hydroperoxide reductase | TsaA | HP1563 |
| C86 | 2507172 | Alkyl hydroperoxide reductase | TsaA | HP1563 |
| **N 4. Transformation (5)** | | | | |
| **N 5. Toxin production and resistance (8)** | | | | |
| D97 | 2313748 | Modulator of drug activity | Mda66 | HP0630 |
| B251 | 2499106 | Vacuolating cytotoxin | VacA | HPO887 |
| **N 6. Pathogenesis (36)** | | | | |
| B318 | 2313643 | Cag pathogenicity island protein | Cag8 | HPO528 |
| B466 | 2313652 | Cag pathogenicity island protein | Cag16 | HP0537 |
| B126 | 2498230 | Cag pathogenicity island protein | Cag26 | HP0547 |
| **N 7. Adaptions to atypical conditions (9)** | | | | |
| D329 | 2500311 | General stress protein | Ctc | HP1496 |
| **N 8. Other (3)** | | | | |
| A424 | 2313716 | Hemolysin secretion protein precursor | HylB | HP0599 |
| A426 | 2313716 | Hemolysin secretion protein precursor | HylB | HP0599 |
| **O Other categories (20)** | | | | |
| **O 1. Plasmid-related functions (3)** | | | | |
| **O 2. Transposon-related functions (17)** | | | | |
| **P Unknown (23)** | | | | |
| **P 1. General (23)** | | | | |
| D281 | 2313491 | Adhesin-thiol peroxidase | TagD | HP0390 |
| A349 | 3123132 | GTP-binding protein, fusA-homolog | YihK | HP0480 |
| D277 | 2313595 | Catalase-like protein | | HP0485 |
| D293 | 2313595 | Catalase-like protein | - | HP0485 |
| B35 | 2314257 | Cinnamyl-alcohol dehydrogenase EL13-2 | Cad | HP1104 |
| B74 | 2314358 | Aldo-keto reductase, putative | - | HP1193 |
| **Q Hypothetical (289)** | | | | |
| **Q 1. Conserved (289)** | | | | |
| D51 | 2313188 | Conserved hypothetical protein | - | HP0105 |
| B263 | 2501535 | Conserved hypothetical ATP-binding protein | - | HP0269 |
| D318 | 2313418 | Conserved hypothetical | - | HP0318 |
| F90 | 2313863 | Conserved hypothetical protein | - | HP0741 |
| D246 | 2499779 | Conserved hypothetical protein | - | HP1075 |
| D262 | 2314247 | Conserved hypothetical secreted protein | - | HP1098 |
| F21 | 2314405 | Conserved hypothetical protein | - | HP1242 |
| D327 | 2314453 | Conserved hypothetical protein | - | HP1285 |
| D142 | 2314454 | Conserved hypothetical secreted protein | - | HP1286 |
| B250 | 3122972 | Conserved hypothetical protein | - | HP1335 |
| B357 | 2829454 | Conserved hypothetical ATP-binding protein | - | HP1430 |
| **Q 2. Hypothetical proteins** | | | | |
| A15 | 2313260 | Hypothetical protein | - | HP0170 |
| D226 | 2313333 | Hypothetical protein | - | HP0231 |
| D323 | 2313333 | Hypothetical protein | - | HP0231 |
| F3 | 2313371 | Hypothetical protein | | HP0268 |
| B278 | 2313728 | Hypothetical protein | - | HP0605 |
| B261 | 2313789 | Hypothetical protein | - | HP0659 |
| E43 | 2313821 | Hypothetical protein | - | HP0697 |
| C194 | 2313821 | Hypothetical protein | - | HP0697 |
| C84 | 2313821 | Hypothetical protein | - | HP0697 |
| D239+ D247 | 2313852 | Hypothetical protein | | HP0721 |
| D348 | 2313852 | Hypothetical protein | - | HP0721 |
| B473 | 2313904 | Hypothetical protein | - | HP0773 |
| F22 | 2314040 | Hypothetical protein | | HP0902 |
| D256 | 2313338 | Hypothetical protein | - | HP1173 |
| D195 | 2314632 | Hypothetical protein | - | HP1454 |
| B104 | 2314715 | Hypothetical protein | - | HP1527 |

**Table 2: Comparison of 10 assigned spots in strains 26695 and J99. Spots with a comparable intensity at the same position or with a horizontal shift of up to 2 cm were assigned to the same protein. The pl given in the table was calculated from the sequence of the TIGR gene sequence database with the help of the pl calculation program of Expasy. The theoretical pl values and the resulting predicted pH shift were compared with those found on the 2-DE patterns.**

| **26695** | | **J99** | | **Identity** | **Amino acid** | **Shift** | |
|---|---|---|---|---|---|---|---|
| **ORF** | **pI** | **ORF** | **pI** | | **26695->J99** | **Predicted** | **2-DE** |
| HP0824 | 5.16 | jhp763 | 5.16 | Thioredoxin | identical | no shift | no shift |
| | | | | TrxA | | | |
| HP0011* | 6.12 | jhp9 | 6.12 | Co-chaperone | identical | no shift | no shift |
| | | | | GroES | | | |
| HP0072* | 5.64 | jhp67 | 5.64 | Urease β subunit | identical | no shift | no shift |
| | | | | UreB | | | |
| HP1161* | 4.45 | jhp1088 | 4.45 | Flavodoxin | V->I; S->G; | no shift | no shift |
| | | | | FldA | T->N; S->A | | |
| HP1458 | 7.72 | jhp1351 | 7.72 | Thioredoxin | S->L; M->V; I->T | no shift | no shift |
| HP0480* | 5.30 | jhp432 | 5.30 | GTP-binding protein, fusA-homolog | R->K; I->L; A->T; T->A | no shift | no shift |
| | | | | YihK | | | |
| HP1199* | 5.22 | jhp1122 | 5.00 | Ribosomal protein L7/L12 | K -> E | J99->left | J99->left |
| | | | | Rp17/112 | | | |
| HP0010* | 5.55 | jhp8 | 5.50 | Chaperone and heat shock protein GroEL | H->D; E->Q | J99->left | J99->left |
| HP0389 | 5.77 | jhp992 | 6.04 | Superoxide dismutase SodB | D->A; G->E; Q->K; E->G; I->V | J99->right | J99->right |
| HP1563 | 5.88 | jhp1471 | 5.98 | Alkyl hydroperoxide reductase TsaA | A→T; T→S; Q→H | J99->right | J99->right |

| | | | | | | | |
|---|---|---|---|---|---|---|---|
| *Detected in this study as antigen | | | | | | | |

**Table 3: The 20 most abundant protein species of the 2-DE pattern of H. pylori 26695. The intensity was determined by adding the optical densities of all of the pixels within each spot. Mr and pl were estimated from the 2-DE position. The spots were identified by peptide mass fingerprinting MALDI mass spectrometry. Protein names in brackets represent proteins in series where the main spot was identified by peptide mass fingerprinting.**

| **Spot No** | **Intensity** | **Mr kDa** | **pI** | **Identity** | **Short name** | **ORF** |
|---|---|---|---|---|---|---|
| A390 | 1899.53 | 59.4 | 5.5 | Chaperone and heat shock protein | GroEL | HP0010 |
| A343 | 1432.48 | 64.7 | 5.6 | Urease β subunit | UreB | HP0072 |
| D341 | 1355.94 | 23.7 | 6.0 | Alkyl hydroperoxide reductase | TsaA | HP1563 |
| A194 | 1209.85 | 60.0 | 5.4 | Chaperone and heat shock protein | GroEL | HP0010 |
| B126 | 1193.57 | 132.4 | 6.6 | Cag pathogenicity island protein | Cag26 | HP0547 |
| A192 | 834.17 | 60.0 | 5.4 | (Chaperone and heat shock protein) | (GroEL) | HP0010 |
| D322 | 791.85 | 28.9 | 8.6 | Urease, α subunit | UreA | HP0073 |
| D329 | 765.38 | 26.7 | 9.1 | Ribosomal protein S4 general stress protein | Rps4 Ctc | HP1294 HP 1496 |
| E35 | 735.28 | 10.0 | 5.1 | Ribosomal protein L7/L12 | Rpl7/l12 | HP1199 |
| D281 | 727.92 | 16.0 | 6.8 | Adhesin-thiol peroxidase | TagD | HP0390 |
| A388 | 704.27 | 59.7 | 5.6 | (Chaperone and heat shock protein) | (GroEL) | HP0010 |
| F16 | 691.56 | 11.5 | 6.4 | Co-chaperone | GroES | HP0011 |
| A323 | 682.03 | 64.8 | 5.7 | Urease β subunit | UreB | HP0072 |
| E54 | 673.21 | 12.3 | 5.6 | Neutrophil activating protein | NapA | HP0243 |
| A325 | 667.99 | 65.1 | 5.6 | Urease β subunit | UreB | HP0072 |
| F44 | 667.53 | 11.4 | 8.3 | - | - | - |
| F52 | 640.92 | 11.8 | 8.6 | - | - | - |
| D142 | 635.50 | 17.5 | 8.8 | Conserved hypothetical secreted protein | - | HP1286 |
| B537 | 576.40 | 52.2 | 6.7 | Aminopeptidase a/i | PepA | HP0570 |
| A477 | 567.52 | 46.6 | 5.2 | Translation elongation factor EF-Tu | TufB | HP1205 |

**Table 4: Known virulence factors identified on the 2-DE pattern of H. pylori 26695.**

| **Spot No** | **Short name** | **ORF** |
|---|---|---|
| A323, A325, A343 | UreB | HP0072 |
| B126 | Cag26 | HP0547 |
| B251 | VacA | HP0887 |
| B318 | Cag8 | HP0528 |
| B437, B439 | Catalase | HP0875 |
| C134, C197 | SodB | HP0389 |
| D121, D132, D345 | HpaA | HP0410 |
| D200, D316, D318, D322, D323, D326 | UreA | HP0073 |
| F6, F9, F16 | GroES | HP0011 |

**Table 5: Spots varying in intensity dependent on the pH of the medium. H. pylori 26695 was cultivated for 5 days on agarose plates adjusted to pH values between 5 and 8. Three cultures per pH value and spot No. were analysed by 2-DE and evaluated by the software program Topspot. Spot intensity was normalised on 10 spots with predicted identical intensity. Mean values for 5 spots evaluated as clearly different were calculated and are presented together with their coefficients of variability. OD, optical density.**

| **Spot No.** | **Mean value of intensity (sum of pixel OD)** | | | | **Coefficient of variability (%)** | | | | **Identity** | **Open reading frame** |
|---|---|---|---|---|---|---|---|---|---|---|
| | **pH5** | **pH6** | **pH** | **pH8** | **pH5** | **pH6** | **pH7** | **pH8** | | |
| 1 B429 | 397.5 | 631.5 | 777.3 | 832.7 | 28.1 | 6.6 | 5.8 | 21.0 | Serine protease HtrA | Hp1019 |
| 2 B231 | - | 24.5 | 123.9 | 138.9 | - | - | 49.6 | 31.8 | Vacuolati ng toxin VacA | Hp0887 |
| 3 B240 | - | 53.2 | 214.1 | 216.4 | - | 53.7 | 19.7 | 15.8 | Vacuolati ng toxin VacA | Hp0887 |
| 4 B251 | - | 73.6 | 294.1 | 275.9 | - | 8.5 | 14.6 | 18.0 | Vacuolati ng toxin VacA | Hp0887 |
| 5 B258 | 40.5 | 109.1 | 242.3 | 217.3 | 35.4 | 19.7 | 11.8 | 31.0 | Vacuolati ng toxin VacA | Hp0887 |

**Table 6: Identified antigens of H. pylori 26695 detected by immunoblotting with human sera. After protein separation by 2-DE the resulting protein pattern was blotted onto PVDF. The antigens immobilized on the membrane reacted with antibodies of sera from an adenocarcinoma patient (Mpi44) and from an ulcus ventriculi patient (Mpi54). The serum of a patient with clearly no H. pylori history (Mpi40) was analyzed to detect potential unspecific immune reactions. x, positive reaction; -, no reaction.**

| **Spot No** | **Identity** | **ORF** | **Mpi40** | **Mpi44** | **Mpi54** |
|---|---|---|---|---|---|
| A194 | Chaperone and heat shock protein GroEL | HP0010 | x | x | x |
| A323 | Urease β subunit UreB | HP0072 | - | x | - |
| A325 | Urease β subunit UreB | HP0072 | - | x | - |
| A343 | Urease β subunit UreB | HP0072 | - | x | - |
| A349 | GTP-binding protein, fusA-homolog YihK | HP0480 | - | x | - |
| A390 | Chaperone and heat shock protein GroEL | HP0010 | x | x | x |
| A431 | Glutamine synthase GlnA | HP0512 | - | - | x |
| B2 | Aconitase B AcnB | HP0779 | - | x | - |
| B 17 | Fumarate reductase flavoprotein subunit FrdA | HP0192 | - | - | x |
| B126 | Cag pathogenicity island protein Cag26 | HP0547 | - | - | x |
| B210 | Isocitrate dehydrogenase Icd | HP0027 | - | x | - |
| B320 | Signal recognition particle protein Ffh | HP1152 | - | x | - |
| B439 | Catalase | HP0875 | x | x | - |
| B455 | Processing protease YmxG | HP0657 | - | x | - |
| B483 | Citrate synthase GltA | HP0026 | - | x | - |
| C109 | Hydrogenase expression/formation protein HypB | HP0900 | - | x | x |
| D230 | Oxygen insensitive NAD(P)H nitroreductase | HP0954 | - | x | - |
| D249 | Cell binding factor 2 | HP0175 | - | x | - |
| D265 | Penicillin tolerance protein LytB | HP0400 | - | x | - |
| D281 | Adhesin-thiol peroxidase TagD | HP0390 | - | x | - |
| D287 | Pyruvate ferredoxin oxidoreductase γ unit | HP1108 | - | x | - |
| D295 | 3-ketoacyl-acyl carrier protein reductase FabG | HP0561 | - | x | - |

| | | | | | |
|---|---|---|---|---|---|
| D313 | Iron (III)ABC transporter, periplasmic iron-binding protein CeuE | HP1562 | - | x | - |
| D316 | Urease, α subunit UreA | HP0073 | x | x | - |
| D322 | Urease, α subunit UreA | HP0073 | - | x | - |
| E27 | Ribosomal protein L7/L12 Rp17/112 | HP1199 | - | x | x |
| E35 | Ribosomal protein L7/L12 Rp17/112 | HP1199 | - | x | x |
| E43 | Hypothetical protein | HP0697 | - | x | - |
| E62 | Flavodoxin FldA | HP1161 | - | - | x |
| F6 | Co-chaperone GroeS | HP0011 | - | x | x |
| F9 | Co-chaperone GroeS | HP0011 | - | x | x |
| F16 | Co-chaperone GroeS | HP0011 | - | x | |

**Table 7: Proteins preferentially recognized in H pylori positive patients. H. pylori 26695 was separated by a small gel 2-DE technique (7 x 8 cm) (Jungblut and Seifert, 1990) and blotted onto PVDF membranes (Jungblut et al., 1990, Electrophoresis). Sera of 24 patients with positive H. pylori diagnosis were compared with 12 sera of patients with negative H. pylori diagnosis concerning their immunoreactivity with antigens separated on the 2-DE blots. The spots of the small gels were assigned to spots of the large gels published in the 2-DPAGE database and by Jungblut et al, 2000, Mol.Microbiol.). (serie) describes that the protein was assigned to an identified main spot within the same spot serie. no id., the protein was not identified until now.**

| **2-DPAGE No** | **ORF** | **Identity** |
|---|---|---|
| D226 | HP 0231 | Predicted coding region |
| E44 | HP 1199 | 50 rib. ProteinL7/I12 |
| A177 | HP 0010 | GroEL (Serie) |
| A194 | HP 0010 | 60kDchaperonin GroEL |
| D276 | no id. | no id. |
| A390 | HP 0010 | 60kDchaperonin GroEL |
| E35 | HP 1199 | 50s rib. ProteinL7/L12 |
| A388 | HP 0010 | 60kDchaperonin GroEL |
| A343 | HP 0072 | Urease B-unit |
| A209 | HP 1132 | F1F0-ATPase B-subunit |
| A477 | HP 1205 | transl.elong.fact. EF-Tu |
| A308 | no id. | no id. |
| B126 | HP 0547 | cag Protein 26 |
| E27 | HP 1199 | 50s rib. Protein I7/L12 |
| A464 | no id. | no id. |
| A307 | no id. | no id. |
| A461 | HP 1293 | DNA-directed RNA polymerase A |
| A396 | no id. | no id. |
| A97 | no id. | no id. |
| A411 | no id. | no id. |
| E49 | no id. | no id. |
| A192 | HP 0010 | 60kDchaperonin GroEL (Serie) |
| E53 | no id. | no id. |
| B126 | HP 0547 | cag Protein 26 |
| 8126 | HP 0547 | cag Protein 26 |
| B126 | HP 0547 | cag Protein 26 |
| B126 | HP 0547 | cag Protein 26 |
| B19 | HP 0192 | Fumarate reductase f. subunit |
| A325 | HP 0072 | Urease B-unit |
| A119 | HP 0010 | GroEL (Serie) |
| A190 | no id. | no id. |
| A323 | HP 0072 | Urease B-unit |
| A424 | HP 0599 | hemolysin secr.protein precursor |
| B3 | no id. | no id. |
| A107 | no id. | no id. |
| D322 | HP 0073 | Urease A-subunit |
| D132 | HP 0410 | HpaA |
| B488 | HP 0829 | Inosine-5'-Monophosphatase deh. |
| D173 | no id. | no id. |
| D265 | HP 0400 | Penicillin tolerance protein LytB |
| D202 | HP 1564 | Outer membrane protein |
| D314 | HP 1582 | Pyridoxal phosphatase b.prot. J.. |
| D318 | HP 0318 | Conserved hypothetical protein |
| D321 | HP 0073 | Urease A-subunit |
| B429 | HP 1019 | Serine protease HtrA |
| B437 | HP 0875 | catalase |
| B439 | HP 0875 | catalase |
| B443 | no id. | no id. |
| B303 | HP 1350 | protease |
| 8320 | HP 1152 | Signal recognition particle protein cag 16 |
| D259 | no id. | no id. |
| D261 | no id. | no id. |
| D165 | no id. | no id. |
| F82 | no id. | no id. |
| D299 | no id. | no id. |
| A359 | HP 0109 | DNAK protein |
| E35 | HP1199 | 50s rib.Protein (Serie) |
| B17 | HP0192 | Fumarate redudase flavoprotein subunit (Serie) |
| B499 | HP0027 | Isocitrate dehydrogenase(Serie) |
| B499 | HP0027 | Isocitrate dehydrogenase(Serie) |
| D262 | HP 1098 | conserved h.secreted protein |
| B492 | HP 0027 | Isocitrate dehydrogenase |
| D327 | HP 1285 | Conserved hypothetical protein |

**Table 8: H. pylori proteins with statistically significant specifity for antigenicity against human sera. H. pylori 26695 was separated by a small gel 2-DE technique (7 x 8 cm) (Jungblut and Seifert, 1990) and blotted onto PVDF membranes (Jungblut et al., 1990, Electrophoresis). Sera of 24 patients with positive H.pylori diagnosis were compared with 12 sera of patients with negative H. pylori diagnosis concerning their immunoreactivity with antigens separated on the 2-DE blots. The spots of the small gels were assigned to spots of the large gels published in the 2-DPAGE database and by Jungblut et al, 2000, Mol.Microbiol.). (serie) describes that the protein was assigned to an identified main spot within the same spot serie. no id., the protein was not identified until now.**

| **2-DPAGE No** | **ORF** | **Identity** |
|---|---|---|
| D226 | Hp 0231 | Predicted coding region |
| E44 | HP1199 | 50Sribosomal protein L7/L12 |
| A177 | HP0010 | GroEL (Serie) |
| D276 | n.id. | n.id. |
| A390 | HP0010 | GroEL |
| E35 | HP1199 | 50Sribosomal protein L7/L12 |
| A388 | HP0010 | GroEL (Serie) |
| A29 | HP1293 | DNA-directed RNA polymerase A(Serie) |
| B2 | HP0779 | Aconitate hydratase 2(Citrate hydrolisase z) |
| B126 | HP0547 | Cag26 |
| E27 | HP1199 | 50Sribosomal protein L7/L12 |
| C84 | HP0794 | ATP-Dependent Clp Protease Proteolytic subunit(X) |
| A396 | n.id. | n.id. |
| E35 | HP1199 | 50s rib.Protein (Serie) |
| A97 | n.id. | n.id. |
| A192 | HP0010 | GroEL (Serie) |
| E53 | n.id. | n.id. |
| B126 | HP0547 | Cag26 |
| B126 | HP0547 | Cag26 |
| B126 | HP0547 | Cag26 |
| B19 | HP0192 | Fumarate reductase flavoprotein subunit |
| A119 | HP0010 | GroEL (Serie) |
| B17 | HP0192 | Fumarate reductase flavoprotein subunit(Serie) |
| A190 | n.id. | n.id. |
| A424 | HP0599 | hemolysin secretion protein precursor |
| A107 | n.id. | n.id. |
| B511 | HP0294 | Aliphatic amidase (aimE) |
| B499 | HP0027 | Isocitrate dehydrogenase(Serie) |
| B499 | HP0027 | Isocitrate dehydrogenase(Serie) |
| D121 | HP0410 | Putative neuroaminyl-lactose-bindung hemagglutinin homolog(xx) |
| B492 | HP0027 | Isocitrate dehydrogenase |
| B35 | HP1104 | Cinnamyl-alcohol dehydrogenase ELI3-2 |
| B429 | HP1019 | Serine protease HtrA |
| B437 | HP0875 | catalase |
| D249 | HP0175 | Hypothetical protein precursor(Serie) |
| B320 | HP1152 | Signal recognition particle protein Ffh |
| E3465 | HP1133 | ATPSyntase Gamma chain |
| B499 | HP0027 | Isocitrate dehydrogenase(Serie) |
| B499 | HP0027 | Isocitrate dehydrogenase(Serie) |

**Table 9: Proteins of H. pylori significantly reacting with antibodies of sera from carcinoma patients, ORF, open reading frame; (serie) describes that the protein was assigned to an identified main spot within the same spot serie.**

| **2-DPAGE No** | **ORF** | **Identity** |
|---|---|---|
| D132 | HP0410 | Putative neuraminyl-lactose-binding hemagglutinin homolog (hpaA) |
| E29 | HP0824 | Thioredoxin |
| B2 | HP0779 | Aconitate hydratase 2 (Citrate hydro-Lysase-2) |
| B2 | HP0779 | Aconitate hydratase 2 (Citrate hydro-Lysase-2) |
| A461 | HP1293 | DNA-directed RNA polymerase a-chain RNA Polymerase A-chain [Serie] |
| A177 | HP0010 | GroEL [Serie] |
| A271 | HP1195 | Elongation factor G (EF-G) |
| B2 | HP0779 | Aconitate hydratase 2 (Citrate hydro-Lysase-2) |
| C197 | HP0389 | Superoxidase dismutase 8sod B9 [Serie] |
| B2 | HP0779 | Aconitate hydratase 2 (Citrate hydro-Lysase-2) |
| B2 | HP0779 | Aconitate hydratase 2 (Citrate hydro-Lysase-2) |
| B2 | HP0779 | Aconitate hydratase 2 (Citrate hydro-Lysase-2) |
| B377 | HP0695 | Hydantoin utilization protein A (HyuA) [Serie] |
| B377 | HP0695 | Hydantoin utilization protein A (HyuA) [Serie] |
| B377 | HP0695 | Hydantoin utilization protein A (HyuA) [Serie] |
| B492 | HP0027 | Isocitrate dehydrogenase |
| B210 | HP0027 | Isocitrate dehydrogenase |
| F40 | HP0404 | Hypothetical hit-like protein |
| D132 | HP0410 | Putative neuraminyl-lactose-binding hemagglutinin homolog (hpaA) |
| D202 | HP1564 | Outer membrane protein [Serie] |
| D314 | HP1582 | Pyridoxal phosphate biosynthetic protein J (Pdxy) |
| D262 | HP 1098 | Coserved hypothetical secreted protein |
| D313 | HP1562 | Iron (III) ABC transporter, periplasmatic iron-binding protein (ceu) [Serie] |
| D313 | HP1562 | Iron (III) ABC transporter, periplasmatic iron-binding protein (ceu) [Serie] |
| D200 | HP1186 | Carbonic anhydrase [Serie] |
| EF68 | HP0514 | 50s ribosomal protein L9 |
| B499 | HP0027 | Isocitrate dehydrogenase (Serie) |
| B499 | HP0027 | Isocitrate dehydrogenase (Serie) |

**Table 10: Classification of H. pylori (Hp) infected and noninfected patients**

| | Hp positive | | Hp negative | Gastric Tumors |
|---|---|---|---|---|
| | Gastritis | Ulcer | | |
| Eradication therapy | 5 | 1 | 0 | 0 |
| Previous Hp | 1 | 2 | 0 | 2 |
| No History of Hp known | 9 | 6 | 12 | 4 |
| Disease group | 15 | 9 | 12 | 6 |
| Total (n=42) | 24 | | 12 | 6 |

**Table 11. H. pylori antigens recognized by sera from infected individuals with a signal frequency > 10 or with significant difference of recognition compared to H. pylori negative sera**

| Protein class | | Spot | ORF | Identity | Short name | | Hp positive (n=24) | | Hp negative (n=12) | |
|---|---|---|---|---|---|---|---|---|---|---|
| | | | | | | | frequency^{d} | occurrence | frequency | occurrence |
| B 2. | 2'-deoxyribonucleotide metabolism | B48B | HP 0829 | lnosine-5'-monophosphatase dehydrogenase | GuaB | | 11 | 6 | 2 | 2 |
| B 4. | Central intermediary metabolism | D322 | HP 0073 | Urease alpha-subunit (Urea Amidohydrolase) | UreA | ^{a} | 33 | 19 | 27 | 7 |
| | | A343 | HP 0072 | Urease beta-subunit (Urea Amidohydrolase) | UreB | ^{a} | 28 | 14 | 15 | 8 |
| D 7. | Pyridoxine | D314 | HP 1582 | Pyridoxal phosphate biosynthetic protein J | PdxJ | | 11 | 7 | 7 | 3 |
| F 3. | Anaerobic of energy metabolism | 817 | HP 0192 | Fumarate reductase flavoprotein subunit | FrdA | ^{a} | 17 | 8 | 1 | |
| F 4. | ATP-proton motive force Interconversion | A209 | HP 1132 | ATP synthase beta chain | atpB | | 16 | 8 | 1 | 1 |
| | | A396 | HP 1134 | ATP synthase alpha chain | atpA | | 15* | 11 * | 1 | 1 |
| F11. | TCA cycle | 8497 | HP 0027 | Isocitrate dehydrogenase | Icd | ^{a} | 7 * | 8* | 0 | 0 |
| | | B66+B138 | | Protein associated with isocitrate | ... | ^{a,b} | 8 | 10 | 11 | 6 |
| | | | | dehydrogenase, n.i. ^{c} | | | | | | |
| 12. | DNA-dependent RNA- polymerase | A461 | HP 1293 | DNA-directed RNA polymerase alpha chain | RpoA | | 17 | 9 | 5 | 5 |
| J 3. | Ribosomal proteins: synthesis and modification | D165 | HP1307 | 50S ribosomal protein L5 | Rpl5 | | 13 | 5 | 2 | 2 |
| | | D299 | HP1201 | 50s ribosomal protein L1 | Rpl1 | | 17 | 10 | 6 | 3 |
| | | E35 | HP1199 | 50S ribosomal Protein L7/L12 | Rpl7/l12 | ^{a} | 61 | 21 | 27 | 10 |
| | | E35 | HP1199 | 50S ribosomal Protein L7/L12 | Rpl7/l12 | ^{a} | 6* | 11* | 0 | 0 |
| | | F82 | HP1302 | 30s ribosomal protein S5 | Rps5 | | 14 | 6 | 1 | 1 |
| J 5. | Translation factors | A477 | HP 1205 | Elongation Factor (EF-TU) | TufB | ^{a} | 18 | 9 | 3 | 3 |
| K2. | Protein folding and stabilization | A359 | HP 0109 | DnaK protein (Heat shock protein 70) | DnaK | ^{a} | 11 | 6 | 1 | 1 |
| | | A390 | HP 0010 | GroEL | GroEL | ^{a} | 37* | 20 | 14 | 8 |
| K 3. | Protein and peptide secretion | B320 | HP 1152 | Signal recognition particle protein (54 homolog) | Ffh | | 24 | 12 | 4 | 4 |
| K 4. | Degradation of proteins, peptides and glycopeptides | A308 | HP0264 | ClpB protein | ClpB | | 14 | 7 | 0 | 0 |
| | | B303 | HP 1350 | Protease | ... | | 30 | 14 | 8 | 7 |
| | | B429 | HP 1019 | Serine protease | HtrA | ^{a} | 10* | 9* | 0 | 0 |
| L 1. | Other regulatory functions | D265 | HP 0400 | Penicillin tolerance protein | LytB | | 13 | 7 | 1 | 1 |
| M 2. | Surface structures | D132 | HP 0410 | Putative neuraminyt-factose-binding | HpaA | ^{a} | 12 | 9 | 3 | 2 |
| | | | | hemagglutinin homolog | | | | | | |
| M 5. | Cell envelope: others | D202 | HP 1564 | Outer membrane protein | ... | | 18 | 14 | 4 | 4 |
| N 3. | Detoxification | B439 | HP 0875 | Catalase | ... | ^{a} | 48 | 20 | 26 | 8 |
| N 6. | Cellular processes: pathogenesis | B126 | HP 0547 | Cag 26 | Cag26 | | 37* | 8 | 1 | 1 |
| | | B466 | HP0537 | Cag 16 | Cag16 | | 11 | 14 | 4 | 3 |
| | | B443 | HP 0522 | Cag3 | Cag3 | | 14 | 7 | 0 | 0 |
| N 8. | Cellular processes: others | A424 | HP 0599 | Hemolysin secretion protein precursor | HylB | ^{a} | 27* | 12 | 2 | 2 |
| P 1. | Unknown function | A411 | HP0795 | Trigger factor | ... | | 19 | 6 | 0 | 0 |
| Q1. | Q1. Conserved hypothetical | D318 | HP 0318 | Conserved hypothetical protein | ... | | 13 | 9 | 7 | 3 |
| | | D262 | HP 1098 | Conserved hypothetical secreted protein ... | | | 14 | 8 | 24 | 6 |
| Q 2. | Hypothetical proteins | D226 | HP 0231 | H.pylori predicted coding region HP0231 | ... | ^{a} | 23* | 11* | 0 | 0 |
| | | D276 | HP 0305 | Hypothetical protein | ... | | 20 | 15 | 10 | 4 |

| | | | | | | | | | | |
|---|---|---|---|---|---|---|---|---|---|---|
| Nomenclature according to the Tigr database, ^{a} spot occurs in a series, ^{b} determined by localisation on the gel, ^{c} n.i. not yet identified, ^{d} signal frequency calculated as the sum of intensity grades (0.5, 1, 2, 3, 4) of each serum divided by the optimal reachable value (n x 4) x 100, where n = number of sera, * significant difference compared to negative group, p<0,05 | | | | | | | | | | |

**Table 12: Sera from gastritis, ulcer and cancer patients react with H. pylori proteins (significant differences)**

| Protein class | | Spot | ORF | Identity | Occurrence (%) | | | | | Mean intensity | | | Frequency (%)^{e} | | |
|---|---|---|---|---|---|---|---|---|---|---|---|---|---|---|---|
| | | | | | Short name | | G | U | C | G | U | C | G | U | C |
| | | | | | | | n=15 | n=9 | n=6 | n=15 | n=9 | n=6 | n=15 | n=9 | n=6 |
| A 6. | Amino acid biosynthesis | B377 | HP0695 | Hydantoin utilization protein A | HyuA | ^{a} | 33 | 22 | 50,0 | 0.5 | 0.2 | 1,8 ° | 13 | 5 | 45 |
| B 4. | Central intermediary metabolism | D321 | HP0073 | Urease alpha-subunit | UreA | | 20 | 55 | 50 | 0.3 | 1.3 | 1,1 | 8 | 33 | 28 |
| F 3. | Anaerobic energy metabolism | B17 | HP0192 | Fumarate reductase flavoprotein subunit | FrdA | | 20 | 55 | 67 * | 0.5 | 0.9 | 1,8 | 13 | 23 | 45 |
| F11. | TCA cycle | B492 | HP0027 | lsocitrate-dehydrogenase | Icd | ^{a,b} | 20 | 77 * | 67 * | 0.4 | 0.3 | 1,2 | 10 | 8 | 30 |
| | | B496 | HP0027 | lsocitrate-dehydrogenase | Icd | ^{a,b} | 20 | 55 | 50 | 0.3 | 0.3 | 1.1 | 8 | 8 | 28 |
| | | B497 | HP0027 | lsocitrate-dehydrogenase | Icd | ^{a,b} | 20 | 55 | 50 | 0.3 | 0.3 | 1.1 | 8 | 8 | 27 |
| | | B499 | HP0027 | Isocitrate-dehydrogenase | lcd | ^{a,b} | 20 | 55 | 67 * | 0.3 | 0.3 | 1.8 | 8 | 8 | 45 |
| | | B66+B138 | | Protein associated with Isocitrate dehydrogenase In the 2D gel, ^{d} n.l. | lcd | ^{a,c} | 26 | 66 | 83 * | 0.3 | 0.3 | 1.8 * | 8 | 8 | 45 |
| 12. | DNA-dependent RNA polymerase | A29 | HP1293 | DNA-directed RNA polymerase A alpha chain (Transciptase alpha chain) | RpoA | | 0 | 0 | 33 *,° | 0 | 0 | 0.8 * | 0 | 0 | 20 |
| J 3. | Ribosomal proteins | E35 | HP1199 | 50s ribosomal protein L7/L12 | Rpl7/I12 ^{a,b} | | 33 | 66 | 16 | 0.2 | 0.3 | 0.1 | 5 8 | | 3 |
| | | E44 | HP1199 | 50s ribosomal protein L7/L12 | Rpl7/l12^{a,b} | | 40 | 77 *# | 0 | 0.8 | 1.8 | # 0 | 20 | 45 | 0 |
| | | F68 | HP0514 | 50s ribosomal protein L9 | Rpl9 | | 0 | 33 * | 0 | 0 | 0.6 | * 0 | 0 | 15 | 0 |
| | | F82 | HP1302 | 30s ribosomal protein S5 | Rps5 | | 7 | 55 * | 33 | 0.3 | 1.1 | * 0.2 | 8 | 28 | 5 |
| J 5. | Translation factors | A477 | HP1205 | Elongation factor (EF-TU) | TufB | ^{a} | 20 | 66 * | 67 * | 0.4 | 1.3 * | 0.9 | 9 | 33 | 23 |
| K 3. | Protein and peptide secretion and trafficking | B320 | HP1152 | Signal recognition particle protein (fifty-four homolog) | Ffh | | 33 | 77 * | 50 | 0.5 | 1.8 | 0.8 | 13 | 45 | 20 |
| K 4. | Degradation of proteins, peptides, and glycopeptides | C84 | HP0794 | ATP-dependent Clp protease proteolytic subunit (Endopeptidase CLP) | ClpP | | 0 | 11 | 33 * | 0 | 0.1 | 0.2 | 0 | 1 | 5 |
| M 2. | Surface structures | D121 | HP0410 | Putative neuraminyl-lactose-binding hemagglutinin homolog | hpaA | ^{a} 6 | | 0 | 33 ° | 0.1 | 0 | 0.2 | 1 | 0 | 6 |
| N 6. | Cellular processes: pathogenesis | B466 | HP0537 | Cag 16 | Cag 16 | | 40 | 88 *# | 17 | 0.2 | 0.8 | *# 0.3 | 6 | 21 | 8 |
| P 1. | Unknown | B35 | HP1104 | Cinnamyl-alcohol dehydrogenase ELI 3-2 | Cad | | 6 | 22 | 67 * | 0.2 | 0.1 | 1.6 *° | 5 | 3 | 40 |
| Q 1. | Conserved hypothetical | D249 | HP0175 | Hypothetical protein HP0175 precursor | ... | | 13 | 66 * | 50 * | 0.2 | 0.7 | * 0.3 | 5 | 18 | 8 |
| | | D327 | HP1285 | Conserved hypothetical protein | ... | | 40 | 89 * | 50 | 0.6 | 0.4 | 0.8 | 15 | 11 | 20 |
| Q 2. | Hypothetical | D276 | HP0305 | Hypothetical protein | ... | | 46 | 88 * | 50 | 0 | 1.3 * | 1.7 * | 1 | 32 | 42 |
| | | B465 | | n.l.... | | | 7 | 27 * | 17 | 0.1 | 0.3 | 0.5 * | 2 | 8 | 13 |

| | | | | | | | | | | | | | | | |
|---|---|---|---|---|---|---|---|---|---|---|---|---|---|---|---|
| Nomenclature according to the Tigr database, ^{a}spot occurs In a series, ^{d} series of spots with different recognition rate, determined by localisation on the gel, " n.l. not yet identified, ^{e} signal frequency calculated as the sum of intensity grades (0.5, 1, 2, 3, 4) of each serum divided by the optimal reachable value (n x 4) x 100, where n = number of sera, ° significant difference compared to ulcer group, p< 0.05, * significant difference compared to gastritis group, p<0.05, # significant difference compared to cancer group, p< 0.05 | | | | | | | | | | | | | | | |

**Table 13. H. pylori proteins preferentially recognized by sera of cancer patients with a frequency > 10 or with high intensity ^{e}**

| Protein class | | Spot | ORF | Identity | Short name | | | Frequency^{d} | | |
|---|---|---|---|---|---|---|---|---|---|---|
| | | | | | | | | Gastritis | Ulcer | Cancer |
| A 6. | Amino acid biosynthesis | B377 | HP0695 | Hydantoin utilization protein A | ^{a} | HyuA | | 13 | 5 | 45 |
| D 7. | Biosynthesis of cofactors: pyridoxine | D314 | HP1582 | Pyridoxal phosphate biosynthetic protein J | | pdxJ | | 10 | 14 | 42 |
| E 4. | Central intermediary metabolism | D200 | HP1186 | Carbonic anhydrase | | ... | | 1 | 0 | 17 |
| | Amino acids and amines | B511 | HP0294 | Aliphatic amidase | | aimE | | 4 | 3 | 15 |
| | Electron transport | E29 | HP0824 | Thioredoxin | | TrxA | | 0 | 0 | 2 |
| F 3. | Anaerobic energy metabolism | B17 | HP0192 | Fumarate reductase flavoprotein subunit | | FrdA | # | 13 | 23 | 45 |
| F11. | TCA cycle | B2 | HP0779 | Aconitate hydratase 2 (Citrate hydro-Lysase-2) | ^{a} | AcnB | | 8 | 6 | 23 |
| | | B492 | HP0027 | isocitrate dehydrogenase | ^{a} | lcd | | 8 | 10 | 30 |
| | | B66+B138 | | Protein associated with isocitrate dehydrogenase, n.i.^{c} | ^{a,b} | lcd | | 8 | 8 | 45 |
| G 4. | Transport and binding proteins: cations | D313 | HP1562 | Iron (III) ABC transporter, periplasmatic iron-binding protein | | ceuE | | 4 | 4 | 21 |
| 12. | DNA-dependent RNA polymerase | A29 | HP1293 | DNA-directed RNA polymerase alpha chain | ^{a} | RpoA | | 0 | 0 | 20 |
| J 5. | Translation factors | A271 | HP1195 | Elongation factor G (EF-G) | | FusA | | 0 | 0 | 4 |
| K 2. | Protein folding and stabilization | A177 | HP0010 | GroEL | ^{a} | GroEL | | 4 | 6 | 19 |
| K 4. | Degradation of proteins, peptides and glycopeptides | C84 | HP0794 | ATP-dependent Clp protease proteolytic subunit (Endopeptidase CLP) | | | | 0 | 1 | 5 |
| M 2. | Surface structures | 0132 | HP0410 | Putative neuramlnyl-lactose- | ^{a} | hpaA | | 18 | 15 | 33 |
| | | D121 | HP0410 | binding hemagglutinin homolog | | hpaA | | 1 | 0 | 6 |
| M 5. | Cell envelope: others | D202 | HP1564 | Outer membrane protein | ^{a} | ... | | 12 | 29 | 50 |
| N 3. | Detoxification | C197 | HP0389 | Superoxidase dismutase | | sodB | | 0 | 0 | 4 |
| P 1. | Unknown | B35 | HP1104 | Cinnamyl-alcohol dehydrogenase ELI 3-2 | | Cad | | 5 | 3 | 40 |
| Q 1. | Conserved hypothetical | D262 | HP1098 | Conserved hypothetical secreted protein | | ... | | 12 | 14 | 42 |
| Q 2. | Hypothetical | F40 | HP0404 | Hypothetical protein | | ... | | 0 | 0 | 2 |

| | | | | | | | | | | |
|---|---|---|---|---|---|---|---|---|---|---|
| nomenclature according to the Tigr database, ^{a}spot occurs In a series, ^{b}determined by localisation on the gel, ^{c}n.i., not yet identified, ^{d} signal frequency calculated as the sum of intensity grades (0.5,1,2,3,4) of each serum divided by the optimal reachable value (n x 4) x 100, where n = number of sera, e antigens recognized by one or two cancer sera with intensity> 0.05 | | | | | | | | | | |

**Table 14: H. pylori specific antigens**

| | Protein class | | Spot | ORF | Identity | |
|---|---|---|---|---|---|---|
| new antigens | | | | | | |
| | F3. | Anaerobic energy metabolism | B17 | HP0192 | Fumarate reductase flavoprotein subunit | FrdA |
| | J3. | Ribosomal proteins: synthesis and modification | F82 | HP1302 | 30s ribosomal protein S5 | Rps5 |
| | K4. | Degradation of proteins, peptides and glycopeptides | B429 | HP 1019 | Serine protease (htrA) | HtrA |
| | N6. | Cellular processes: pathogenesis | B443 | HP0522 | Cag3 | Cag3 |
| | Q2. | Hypothetical | D226 | HP0231 | H. pylori predicted coding region | |

| proteins known from other studies | | | | | | |
|---|---|---|---|---|---|---|
| | | F4.ATP-proton motive force interconversion | A209 A396 | HP 1132 HP1134 | ATP synthase beta chain ATP synthase alpha chain | atpB atpA |
| | K2. | Protein folding and stabilization | A359 | HP 0109 | DNAK protein (Heat shock protein 70) | DnaK |
| | K4. | Degradation of proteins, peptides and glycopeptides | A308 | HP0264 | CLPB protein | ClpB |
| | L1. | Other regulatory functions | D265 | HP 0400 | Penicillin tolerance protein (lytB) | LytB |
| | N6. | Cellular processes: pathogenesis | B126 | HP 0547 | Cag 26 | Cag26 |
| | P1. | Unknown function | A411 | HP0795 | trigger factor | |

| minor spots from spot series in the gel | | | | | | |
|---|---|---|---|---|---|---|
| | F11. | TCA cycle | B497 | HP0027 | Isocitrate-dehydrogenase | lcd |
| | J3. | Ribosomal proteins: synthesis and modification | E44 | HP1199 | 50s ribosomal protein L7/L12 | Rpl7/12 |

**Table 15: H. pylori antigens associated with ulcer**

| **Protein class** | | | **Spot** | **ORF** | **Identity** | **Significant association** |
|---|---|---|---|---|---|---|
| new antigens | | | | | | |
| | F3. | Anaerobic energy metabolism | B17 | HP0192 | Fumarate reductase flavoprotein subunit | FrdA |
| | J3. | Ribosomal proteins: synthesis and modification | F68 | HP0514 | 50s ribosomal protein L9 | Rpl9 |
| | | | F82 | HP1302 | 30s ribosomal protein S5 | Rps5 · |
| | Q1. | Conserved hypothetical | D249 | HP0175 | Hypothetical protein HP0175 precursor | · |
| | Q1. | Conserved hypothetical | D327 | HP1285 | Conserved hypothetical protein | · |
| | Q2. | Hypothetical | D276 | HP0305 | hypothetical protein | · |
| | | | B465 | | n.i. | · |

| proteins known from other studies | | | | | | |
|---|---|---|---|---|---|---|
| | B4. | Central intermediary metabolism | D321 | HP0073 | Urease Alpha-Subunit | UreA * |
| | F11. | TCA cycle | B492 | HP0027 | Isocitrate-dehydrogenase | lcd * |
| | | | B499 | HP0027 | | * |
| | J3. | Ribosomal proteins: synthesis and modification | E44 | HP1199 | 50s ribosomal protein L7/L12 | Rpl7/12 * |
| | J5. | Translation factors | A477 | HP1205 | Elongation factor (EF-TU) | TufB * |
| | K3. | Protein and peptide secretion and trafficking | B320 | HP1152 | Signal recognition particle protein (fifty-four homolog) | Ffh * |
| | N6. | Cellular processes: pathogenesis | B466 | HP0537- | Cag16 | Cag16 * |

**Table 16: H. pylori antigens associated with cancer**

| Protein class | | | Spot | ORF | Identity | Significant association |
|---|---|---|---|---|---|---|
| relatively | A6. | Amino acid biosynthesis | B377 | HP0695 | Hydantoin utilization protein A | * |
| restricted | F2. | Amino acids and amines | B511 | HP0294 | Aliphatic amidase | |
| | F11. | TCA cycle | B2 B66+B138 | HP0779 | Aconitate hydratase protein associated with isocitrate dehydrogenase, localisation in the gel | |
| | 12. | DNA-dependent RNA polymerase | A29 | HP1293 | DNA-directed RNA polymerase A alpha chain | * |
| | K4. | Degradation of proteins, peptides, and glycopeptides | C84 | HP0794 | ATP-Dependent Clp Protease Proteolytic Subunit | * |
| | M2. | Surface structures | D121 D132 | HP0410 | Putative neuraminyl-lactose-binding hemagglutinin homolog | |
| also in ulcer sera | P1. | Unknown | B35 | HP1104 | Cinnamyl-alcohol dehydrogenase ELI 3-2 | * |
| | F3. | Anaerobic energy metabolism | B17 | HP0192 | Fumarate reductase flavoprotein subunit | * |
| | F11. | TCA cycle | B499 | HP0027 | Isocitrate-dehydrogenase | |
| | Q2. | Hypothetical | D276 | HP0305 | Hypothetical protein | * |

| also in negative sera | | | | | | |
|---|---|---|---|---|---|---|
| | D7. | Pyridoxines | D314 | HP1582 | Pyridoxal phosphate biosynthetic protein | |
| | M5. | Cell envelope: others | D202 | HP1564 | Outer membrane protein | |
| | Q1. | Conserved hypothetical | D262 | HP1098 | Conserved hypothetical secreted protein | |

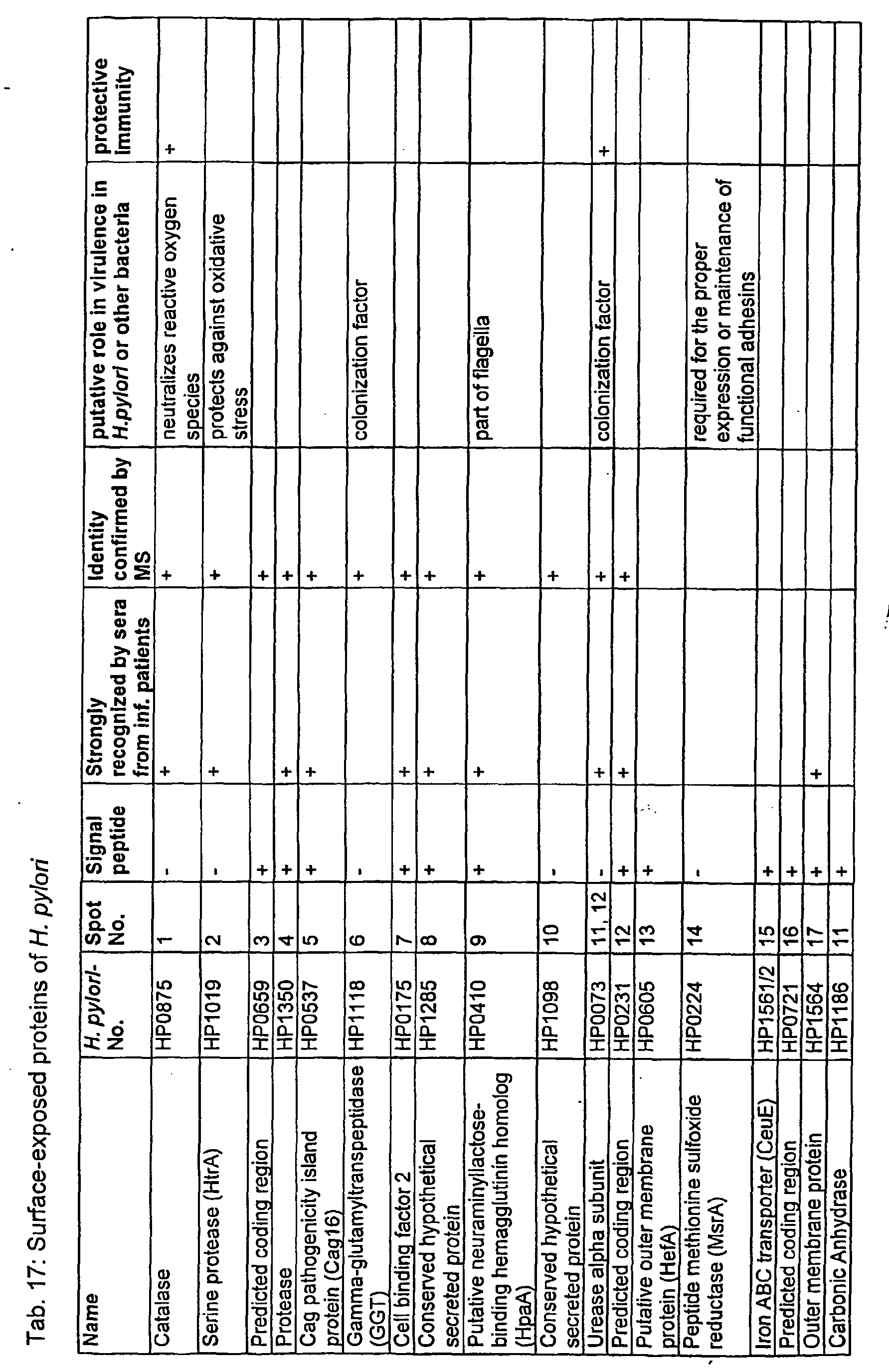

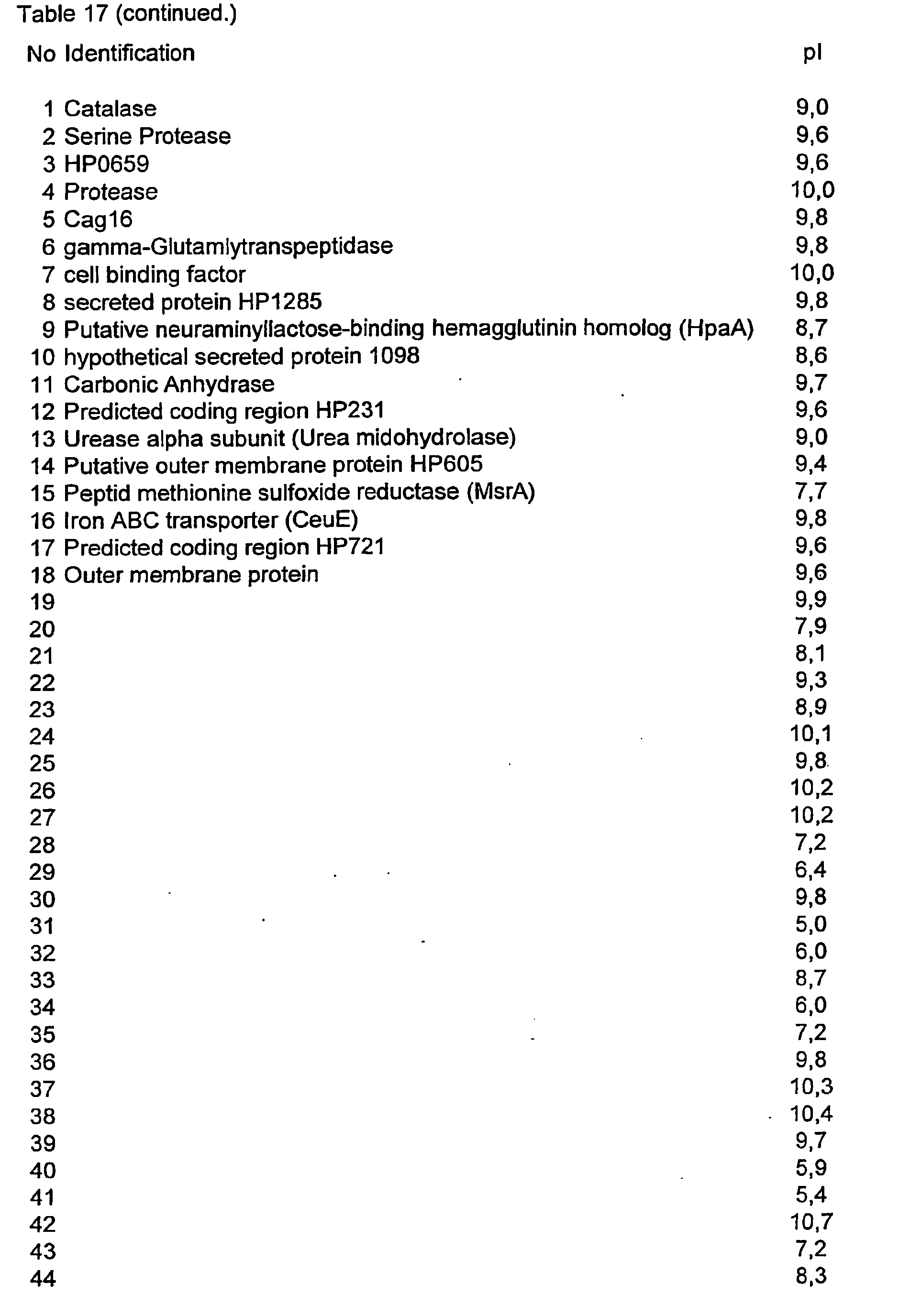

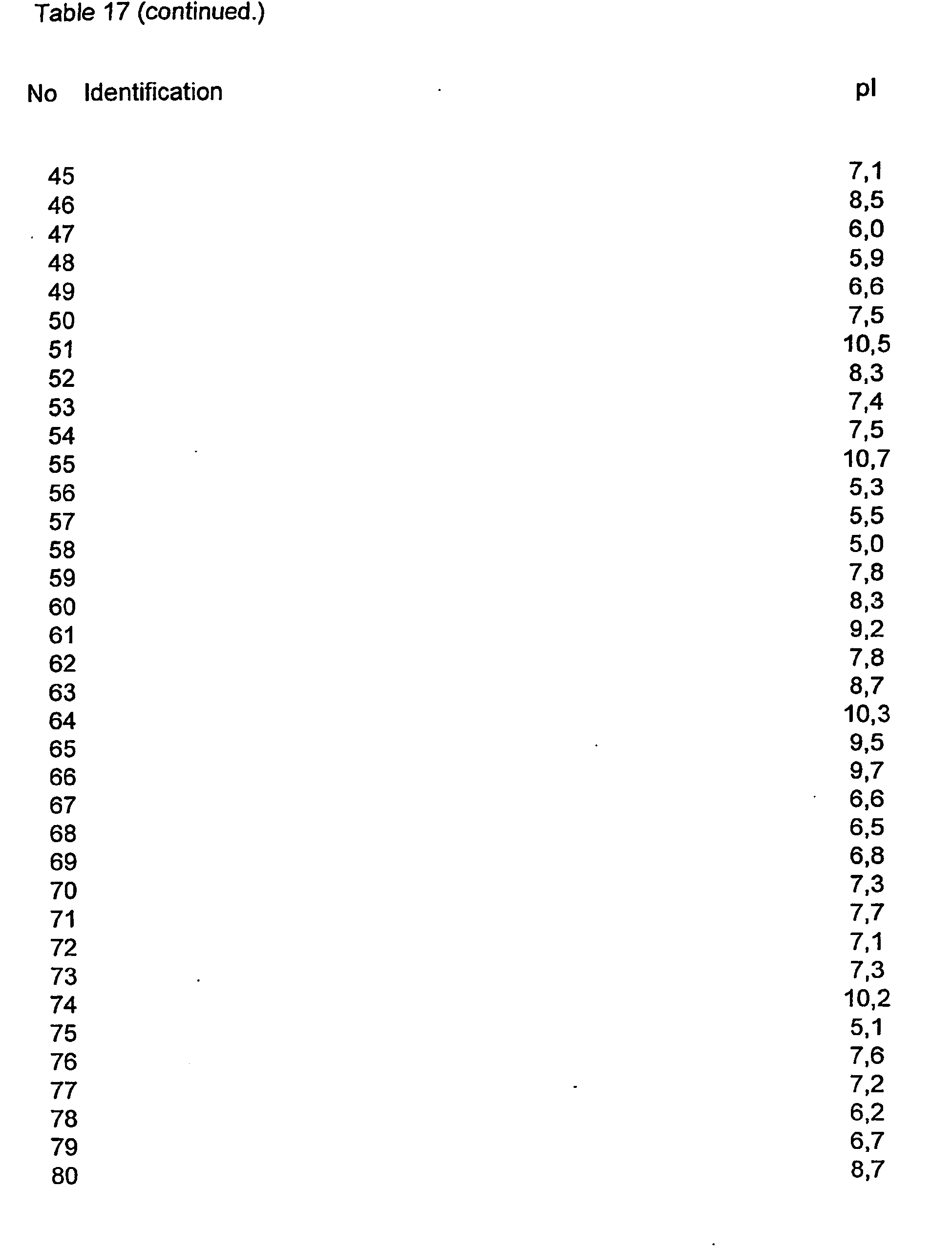

**Table 18**

| **Spot No** | **Seq. Cov** | **NCBI Accession No** | **Name** |
|---|---|---|---|
| 1 | 26% | 2499106 | Vacuolating cytotoxin precursor |
| 2 | 39% | 2506418 | Flagellar hook protein (FlgE) |
| 3 | 57% | 6015162 | Flavodoxin |
| 4 | 46% | 7464021 | conserved hypothetical secreted protein HP1286 |
| 5 | 11 % | 7433809 | gamma-glutamyltranspeptidase |
| 6 | | | |
| 7 | 45% | 7429911 | serine proteinase |
| 8 | 28% | 137076 | Urease beta subunit |
| 9 | 33% | 2492813 | Fructose-biphosphate aldolase |
| 10 | | | |
| 11 | 43% | 7464110 | hook assembly protein, flagella |
| 12 | 5% | 2499106 | Vacuolating cytotoxin precursor |
| 13 | 33% | 7464196 | hypothetical protein HP0231 |
| 14 | 30% | 2499779 | Hypothetical protein HP0175 precursor |
| 15 | 16% | 7433809 | gamma-glutamyltranspeptidase |
| 16 | 34% | 7464490 | hypothetical protein HP1173 |
| 17 | 52% | 3024719 | thioredoxin |
| 18 | 67% | 7430834 | thioredoxin |
| 19 | 38% | 7451814 | flagellar hook-basal body complex protein |
| 20 | 31% | 7465387 | thiol-disulfide interchange protein HP0377 |

## Claims

1. Use of the Helicobacter polypeptide HP0231 for the manufacture of a vaccine for the treatment of Helicobacter infections and/or a specific Helicobacter-mediated disease.

2. Use of a nucleic acid encoding for the Helicobacter polypeptide as claimed in claim 1 for the manufacture of a vaccine for the treatment of Helicobacter infections and/or a specific Helicobacter-mediated disease.

3. The use of claim 1 or 2, wherein the vaccine is selected from recombinant subunit vaccines, recombinant live vaccines and nucleic acid vaccines.

4. The use of any of the claims 1-3, wherein the specific Helicobacter-mediated disease is selected from gastritis, cancer or ulcer.

## Patentansprüche

1. Verwendung des Helicobacter-Polypeptides HP0231 zur Herstellung einer Vakzine zur Behandlung von Helicobacter-Infektionen und/oder einer spezifischen Helicobacter-vermittelten Krankheit.

2. Verwendung einer Nukleinsäure kodierend für das Helicobacter-Polypeptid wie in Anspruch 1 beansprucht zur Herstellung einer Vakzine zur Behandlung von Helicobacter-Infektionen und/oder einer spezifischen Helicobacter-vermittelten Krankheit.

3. Verwendung nach Anspruch 1 oder 2, wobei die Vakzine aus rekombinanten Untereinheits-Vakzinen, rekombinanten Lebendvakzinen und Nukleinsäurevakzinen ausgewählt wird.

4. Verwendung nach einem der Ansprüche 1 bis 3, wobei die spezifische Helicobacter-vermittelte Krankheit aus Gastritis, Krebs oder Ulcus ausgewählt wird.

## Revendications

1. Utilisation du polypeptide Hélicobacter HP0231 pour la fabrication d'un vaccin destiné au traitement des infections à Hélicobacter et/ou d'une maladie médiée par un Hélicobacter spécifique.

2. Utilisation d'un acide nucléique codant le polypeptide Hélicobacter selon la revendication 1 pour la fabrication d'un vaccin destiné au traitement des infections à Hélicobacter et/ou d'une maladie médiée par un Hélicobacter spécifique.

3. Utilisation de la revendication 1 ou 2 dans laquelle le vaccin est choisi parmi les vaccins à virus fractionnés recombinants, les vaccins vivants recombinants et les vaccins à base d'acides nucléiques.

4. Utilisation de l'une quelconque des revendications 1 à 3, dans laquelle la maladie médiée par un Hélicobacter spécifique est choisie parmi la gastrite, le cancer ou l'ulcère.


### SEQUENCE LISTING

<110> Max-Planck-Gesellschaft zur Foerderung der Wissens
<120> Methods for identifying Helicobacter antigens
<130> 22378PEP-WO/WWDI
<140> EP 01 931 657.9
   <141> 2001-04-26
<150> EP 00 108 968.9
   <151> 2000-04-27
<150> EP 01 101 439.6
   <151> 2001-01-23
<160> 4
<170> PatentIn Ver. 2.1
<210> 1
   <211> 795
   <212> DNA
   <213> Helicobacter pylori
<220>
   <221> CDS
   <222> (1)..(795)
<220>
   <223> HP0231
<400> 1
   atg ata tta aga gcg agt gtg ttg agc gcg tta ctt ctt gta ggc tta     48
   Met Ile Leu Arg Ala Ser Val Leu Ser Ala Leu Leu Leu Val Gly Leu
     1               5                  10                  15
   ggg gca gcc cct aaa cat tca gtt tca gct aat gac aaa cgg atg cag     96
   Gly Ala Ala Pro Lys His Ser Val Ser Ala Asn Asp Lys Arg Met Gln
                20                  25                  30
   gat aat tta gtg agc gtg att gaa aaa cag acc aat aaa aag gtg cgt    144
   Asp Asn Leu Val Ser Val Ile Glu Lys Gln Thr Asn Lys Lys Val Arg
            35                  40                  45
   att tta gaa atc aaa cct tta aaa tct agc cag gat tta aaa atg gtc    192
   Ile Leu Glu Ile Lys Pro Leu Lys Ser Ser Gln Asp Leu Lys Met Val
    50                  55                  60
   gtt att gaa gat ccg gac act aaa tac aat atc ccg ctt gtg gtg agt    240
   Val Ile Glu Asp Pro Asp Thr Lys Tyr Asn Ile Pro Leu Val Val Ser
   65                  70                  75                  80
   aag gat ggt aat tta atc ata ggg ctt agc aac ata ttc ttt agc aat    288
   Lys Asp Gly Asn Leu Ile Ile Gly Leu Ser Asn Ile Phe Phe Ser Asn
                   85                  90                  95
   aaa agc gat gat gtg caa tta gtt gca gaa acc aat caa aaa gtt caa    336
   Lys Ser Asp Asp Val Gln Leu Val Ala Glu Thr Asn Gln Lys Val Gln
               100                 105                 110
   gct ctt aac gcc acc caa caa aat agc gcg aaa ttg aac gct att ttt    384
   Ala Leu Asn Ala Thr Gln Gln Asn Ser Ala Lys Leu Asn Ala Ile Phe
           115                 120                 125
   aat gaa ata ccg gct gat tat gcg ata gag ttg ccc tct act aac gct    432
   Asn Glu Ile Pro Ala Asp Tyr Ala Ile Glu Leu Pro Ser Thr Asn Ala
       130                 135                 140
   gca aat aag gat aaa atc ctt tat att gtc tct gat ccc atg tgc cca    480
   Ala Asn Lys Asp Lys Ile Leu Tyr Ile Val Ser Asp Pro Met Cys Pro
   145                 150                 155                 160
   cat tgc caa aaa gag ctc act aaa ctt agg gat cat tta aaa gaa aac    528
   His Cys Gln Lys Glu Leu Thr Lys Leu Arg Asp His Leu Lys Glu Asn
                   165                 170                 175
   acc gtg aga atg gtc gtg gtg ggg tgg ctt ggg gtc aat tca gct aaa    576
   Thr Val Arg Met Val Val Val Gly Trp Leu Gly Val Asn Ser Ala Lys
               180                 185                 190
   aaa gcg gct tta atc caa gaa gaa atg gcg aaa gct agg gct agg gga    624
   Lys Ala Ala Leu Ile Gln Glu Glu Met Ala Lys Ala Arg Ala Arg Gly
           195                 200                 205
   gcg agc gtg gaa gat aag atc tct att ctt gaa aag att tat tcc acc    672
   Ala Ser Val Glu Asp Lys Ile Ser Ile Leu Glu Lys Ile Tyr Ser Thr
       210                 215                 220
   caa tac gat att aac gct caa aaa gag cct gaa gat tta cgc act aaa    720
   Gln Tyr Asp Ile Asn Ala Gln Lys Glu Pro Glu Asp Leu Arg Thr Lys
   225                 230                 235                 240
   gtg gaa aat acc act aaa aag att ttt gaa tct ggc gtg att aag ggt    768
   Val Glu Asn Thr Thr Lys Lys Ile Phe Glu Ser Gly Val Ile Lys Gly
   245 250 255
   gtg cct ttc tta tac cat tat aag gca 795
   Val Pro Phe Leu Tyr His Tyr Lys Ala
   260 265

<210> 2
   <211> 265
   <212> PRT
   <213> Helicobacter pylori
   <223> HP0231
<400> 2
   Met Ile Leu Arg Ala Ser Val Leu Ser Ala Leu Leu Leu Val Gly Leu
   1 5 10 15
   Gly Ala Ala Pro Lys His Ser Val Ser Ala Asn Asp Lys Arg Met Gln
   20 25 30
   Asp Asn Leu Val Ser Val Ile Glu Lys Gln Thr Asn Lys Lys Val Arg
   35 40 45
   Ile Leu Glu Ile Lys Pro Leu Lys Ser Ser Gln Asp Leu Lys Met Val
   50 55 60
   Val Ile Glu Asp Pro Asp Thr Lys Tyr Asn Ile Pro Leu Val Val Ser
   65 70 75 80
   Lys Asp Gly Asn Leu Ile Ile Gly Leu Ser Asn Ile Phe Phe Ser Asn
   85 90 95
   Lys Ser Asp Asp Val Gln Leu Val Ala Glu Thr Asn Gln Lys Val Gln
   100 105 110
   Ala Leu Asn Ala Thr Gln Gln Asn Ser Ala Lys Leu Asn Ala Ile Phe
   115 120 125
   Asn Glu Ile Pro Ala Asp Tyr Ala Ile Glu Leu Pro Ser Thr Asn Ala
   130 135 140
   Ala Asn Lys Asp Lys Ile Leu Tyr Ile Val Ser Asp Pro Met Cys Pro
   145 150 155 160
   His Cys Gln Lys Glu Leu Thr Lys Leu Arg Asp His Leu Lys Glu Asn
   165 170 175
   Thr Val Arg Met Val Val Val Gly Trp Leu Gly Val Asn Ser Ala Lys
                   180                 185                 190
   Lys Ala Ala Leu Ile Gln Glu Glu Met Ala Lys Ala Arg Ala Arg Gly
           195                 200                 205
   Ala Ser Val Glu Asp Lys Ile Ser Ile Leu Glu Lys Ile Tyr Ser Thr
       210                 215                 220
   Gln Tyr Asp Ile Asn Ala Gln Lys Glu Pro Glu Asp Leu Arg Thr Lys
   225                 230                 235                 240
   Val Glu Asn Thr Thr Lys Lys Ile Phe Glu Ser Gly Val Ile Lys Gly
                   245                 250                 255
   Val Pro Phe Leu Tyr His Tyr Lys Ala
               260                 265

<210> 3
   <211> 747
   <212> DNA
   <213> Helicobacter pylori
<220>
   <221> CDS
   <222> (1)..(747)
<220>
   <223> HP0410
<400> 3
   atg aaa aaa ggt agt ttg gca atc gtt tta gga tcg cta tta gcg agt      48
   Met Lys Lys Gly Ser Leu Ala Ile Val Leu Gly Ser Leu Leu Ala Ser
   1               5                   10                  15
   ggg gcg ttt tat acg gct cta gct gat gga atg cct gca aaa cag cag      96
   Gly Ala Phe Tyr Thr Ala Leu Ala Asp Gly Met Pro Ala Lys Gln Gln
               20                  25                  30
   cac aat aat acg ggc gag tca gtg gag ttg cat ttc cac tat cct att     144
   His Asn Asn Thr Gly Glu Ser Val Glu Leu His Phe His Tyr Pro Ile
           35                  40                  45
   aaa ggc aag caa gag cct aaa aac agc cat tta gtc gtt ttg atc gaa     192
   Lys Gly Lys Gln Glu Pro Lys Asn Ser His Leu Val Val Leu Ile Glu
       50                  55                  60
   cct aaa ata gag atc aat aaa gtt atc cct gaa agt tat caa aaa gag 240
   Pro Lys Ile Glu Ile Asn Lys Val Ile Pro Glu Ser Tyr Gln Lys Glu
   65 70 75 80
   ttt gag aag tct ttg ttt ctc cag ttg agt agt ttt tta gag aga aaa 288
   Phe Glu Lys Ser Leu Phe Leu Gln Leu Ser Ser Phe Leu Glu Arg Lys
   85 90 95
   ggc tat agc gtt tcg caa ttt aaa gat gct agc gaa atc cct caa gac 336
   Gly Tyr Ser Val Ser Gln Phe Lys Asp Ala Ser Glu Ile Pro Gln Asp
   100 105 110
   atc aaa gaa aaa gcg ttg ctc gtt tta cgc atg gat ggg aat gtg gct 384
   Ile Lys Glu Lys Ala Leu Leu Val Leu Arg Met Asp Gly Asn Val Ala
   115 120 125
   atc ttg gaa gat att gta gaa gag agc gat gcg ctt agc gaa gaa aaa 432
   Ile Leu Glu Asp Ile Val Glu Glu Ser Asp Ala Leu Ser Glu Glu Lys
   130 135 140
   gtg ata gac atg tct tca ggg tat ttg aac ttg aat ttt gtt gag cca 480
   Val Ile Asp Met Ser Ser Gly Tyr Leu Asn Leu Asn Phe Val Glu Pro
   145 150 155 160
   aaa agt gaa gat att atc cat agt ttt ggt att gat gtt tca aag att 528
   Lys Ser Glu Asp Ile Ile His Ser Phe Gly Ile Asp Val Ser Lys Ile
   165 170 175
   aag gct gtg att gaa aga gtg gaa ttg cgg cgc acc aat tct gga ggt 576
   Lys Ala Val Ile Glu Arg Val Glu Leu Arg Arg Thr Asn Ser Gly Gly
   180 185 190
   ttt gtc ccc aaa act ttt gtg cat agg att aag gaa acc gat cat gat 624
   Phe Val Pro Lys Thr Phe Val His Arg Ile Lys Glu Thr Asp His Asp
   195 200 205
   caa gcc att aga aaa atc atg aat caa gcc tat cac aaa gtg atg gtg 672
   Gln Ala Ile Arg Lys Ile Met Asn Gln Ala Tyr His Lys Val Met Val
   210 215 220
   cat att acc aaa gag tta agc aaa aaa cac atg gaa cat tat gaa aaa 720
   His Ile Thr Lys Glu Leu Ser Lys Lys His Met Glu His Tyr Glu Lys
   225 230 235 240
   gtt tct agt gaa atg aaa aaa cga aag 747
   Val Ser Ser Glu Met Lys Lys Arg Lys
   245

<210> 4
   <211> 249
   <212> PRT
   <213> Helicobacter pylori
   <223> HP0410
<400> 4
   Met Lys Lys Gly Ser Leu Ala Ile Val Leu Gly Ser Leu Leu Ala Ser
   1               5                   10                  15
   Gly Ala Phe Tyr Thr Ala Leu Ala Asp Gly Met Pro Ala Lys Gln Gln
               20                  25                  30
   His Asn Asn Thr Gly Glu Ser Val Glu Leu His Phe His Tyr Pro Ile
           35                  40                  45
   Lys Gly Lys Gln Glu Pro Lys Asn Ser His Leu Val Val Leu Ile Glu
       50                  55                  60
   Pro Lys Ile Glu Ile Asn Lys Val Ile Pro Glu Ser Tyr Gln Lys Glu
   65                  70                  75                  80
   Phe Glu Lys Ser Leu Phe Leu Gln Leu Ser Ser Phe Leu Glu Arg Lys
                   85                  90                  95
   Gly Tyr Ser Val Ser Gln Phe Lys Asp Ala Ser Glu Ile Pro Gln Asp
               100                 105                 110
   Ile Lys Glu Lys Ala Leu Leu Val Leu Arg Met Asp Gly Asn Val Ala
           115                 120                 125
   Ile Leu Glu Asp Ile Val Glu Glu Ser Asp Ala Leu Ser Glu Glu Lys
       130                 135                 140
   Val Ile Asp Met Ser Ser Gly Tyr Leu Asn Leu Asn Phe Val Glu Pro
   145                 150                 155                 160
   Lys Ser Glu Asp Ile Ile His Ser Phe Gly Ile Asp Val Ser Lys Ile
                   165                 170                 175
   Lys Ala Val Ile Glu Arg Val Glu Leu Arg Arg Thr Asn Ser Gly Gly
               180                 185                 190
   Phe Val Pro Lys Thr Phe Val His Arg Ile Lys Glu Thr Asp His Asp
           195                 200                 205
   Gln Ala Ile Arg Lys Ile Met Asn Gln Ala Tyr His Lys Val Met Val
       210                 215                 220
   His Ile Thr Lys Glu Leu Ser Lys Lys His Met Glu His Tyr Glu Lys
   225                 230                 235                 240
   Val Ser Ser Glu Met Lys Lys Arg Lys
                   245